# EUROPEAN PATENT SPECIFICATION

(11) **EP 2 981 544 B1**
(45) Date of publication and mention of the grant of the patent: **04.09.2019**
(21) Application number: 14779463.0
(22) Date of filing: 31.03.2014
(51) Int. Cl.: C07K 7/08, A61K 38/10, A61P 1/00

(54) **NOVEL ALPHA4BETA7 PEPTIDE DIMER ANTAGONISTS**
NEUARTIGE 4-7-PEPTIDDIMERANTAGONISTEN
NOUVEAUX ANTAGONISTES DIMÈRES PEPTIDIQUES DE L' ALPHA4BETA7

(30) Priority: 02.04.2013 US 201361807714 P; 28.03.2014 US 201414229784
(43) Date of publication of application: 10.02.2016
(73) Proprietor: Protagonist Therapeutics Inc., Newark, CA 94560 (US)
(72) Inventor: BHANDARI, Ashok, Pleasanton, California 94588 (US); PATEL, Dinesh V., Fremont, California 94539 (US); MATTHEAKIS, Larry C., Cupertino, California 95104 (US)
(74) Representative: Schiweck Weinzierl Koch Patentanwälte Partnerschaft mbB
(86) International application number: PCT/US2014/032391
(87) International publication number: WO 2014/165448

(56) References cited:
- WO-A1-99/26615
- WO-A2-97/25351
- WO-A2-2008/140602
- US-A1- 2010 196 441
- US-B1- 6 235 711
- US-B1- 6 818 617
- NAHAN J P ET AL: "Selective alpha4beta7 integrin antagonists and their potential as antiinflammatory agents", JOURNAL OF MEDICINAL CHEMISTRY, AMERICAN CHEMICAL SOCIETY, US, vol. 45, 1 August 2002 (2002-08-01), pages 3451-3457, XP009087310, ISSN: 0022-2623, DOI: 10.1021/JM020033K
- JACKSON DAVID Y: "Alpha 4 integrin antagonists", CURRENT PHARMACEUTICAL DESIGN, BENTHAM SCIENCE PUBLISHERS, NL, vol. 8, no. 14, 1 January 2002 (2002-01-01), pages 1229-1253, XP009185317, ISSN: 1381-6128

## Description

### FIELD OF THE INVENTION

The present invention relates to novel compounds having activity useful for treating conditions which arise or are exacerbated by integrin binding, pharmaceutical compositions comprising the compounds, methods of treatment using the compounds, and methods of blocking or disrupting integrin binding.

### BACKGROUND OF THE INVENTION

Integrins are noncovalently associated α/β heterodimeric cell surface receptors involved in numerous cellular processes ranging from cell adhesion and migration to gene regulation (Dubree, et al., Selective α4β7 Integrin Antagonist and Their Potential as Anti-inflammatory Agents, J. Med. Chem. 2002, 45, 3451-3457). Differential expression of integrins can regulate a cell's adhesive properties, allowing different leukocyte populations to be recruited to specific organs in response to different inflammatory signals. If left unchecked, integrins-mediated adhesion process can lead to chronic inflammation and autoimmune disease.

The α4 integrins, α4β1 and α4β7, play essential roles in lymphocyte migration throughout the gastrointestinal tract. They are expressed on most leukocytes, including B and T lymphocytes, where they mediate cell adhesion via binding to their respective primary ligands, vascular cell adhesion molecule (VCAM), and mucosal addressin cell adhesion molecule (MAdCAM), respectively. The proteins differ in binding specificity in that VCAM binds both α4β1 and to a lesser extent α4β7, while MAdCAM is highly specific for α4β7. In addition to pairing with the α4 subunit, the β7 subunit also forms a heterodimeric complex with αE subunit to form αEβ7, which is primarily expressed on intraepithelial lymphocytes (IEL) in the intestine, lung and genitourinary tract. αEβ7 is also expressed on dendritic cells in the gut. The αEβ7 heterodimer binds to E-cadherin on the epithelial cells. The IEL cells are thought to provide a mechanism for immune surveillance within the epithelial compartment. Therefore, blocking αEβ7 and α4β7 together may be a useful method for treating inflammatory conditions of the intestine

Inhibitors of specific integrins-ligand interactions have been shown effective as anti-inflammatory agents for the treatment of various autoimmune diseases. For example, monoclonal antibodies displaying high binding affinity for α4β7 have displayed therapeutic benefits for gastrointestinal auto-inflammatory/autoimmune diseases, such as Crohn's disease, and ulcerative colitis. *Id.* However, these therapies interfered with α4β1 integrin-ligand interactions thereby resulting in dangerous side effects to the patient. Therapies utilizing small molecule antagonists have shown similar side effects in animal models, thereby preventing further development of these techniques.

Jackson. D.Y, Current Pharmaceutical Design 2002, vol. 8, no. 14, 1229-1253 is a review article which deals with alpha 4 integrin antagonists. Diseases which may be treated with alpha integrin antagonists as well as examples of alpha 4 integrin antagonists are disclosed.

Accordingly, there is a need in the art for an integrin antagonist molecule having high affinity for the α4β7 integrin and high selectivity against the α4β1 integrin, as a therapy for various gastrointestinal autoimmune diseases.

Such an integrin antagonist molecule is disclosed herein.

### SUMMARY OF THE INVENTION

The present invention has been developed in response to the present state of the art, and in particular, in response to the problems and needs in the art that have not yet been fully solved by currently available integrin antagonists that are selective for α4β7. Thus, the present invention provides α4β7 antagonist dimer peptides for use as anti-inflammatory and/or immunosuppressive agents. Further, the present invention provides α4β7 antagonist dimer peptide for use in treating a condition that is associated with a biological function of α4β7 to tissues expressing MAdCAM.

The invention relates to a novel class of peptidic compounds exhibiting integrin antagonist activity. The present invention further relates to a novel class of peptidic compounds exhibiting high specificity for α4β7 integrin. Compounds of the present invention comprise two paired subunits that are linked together by their C- or N-terminus via a linking moiety. Each subunit of the present invention further comprises two natural or unnatural amino acids that are capable of bridging to form a cyclized structure. Thus, the compounds of the present invention comprise dimerized peptides, each subunit of the dimer forming a cyclized structure through at least one of a disulfide salt bridge, an amide bond, or an equivalent connection. This feature provides increased stability to the compound when administered orally as a therapeutic agent. This feature further provides for increased specificity and potency as compared to non-cyclized analogs.

In one aspect, the present invention provides a dimer compound comprising two linked subunits of Formula (I):
Xaa¹-Xaa²-Xaa³-Xaa⁴-Xaa⁵-Xaa⁶-Xaa⁷-Xaa⁸-Xaa⁹-Xaa¹⁰-Xaa¹¹-Xaa¹²-Xaa¹³-Xaa¹⁴ (SEQ ID NO:1), or a pharmaceutically acceptable salt thereof, wherein each subunit comprises a disulfide or lactam bond between Xaa⁴ and Xaa¹⁰, and further wherein Formula (I) represents a monomer subunit of a dimer molecule, wherein the monomer subunits are linked to form a dimer molecule in accordance with the present invention, and wherein Xaa¹ is absent, or Xaa¹ is selected from the group consisting of, Gln, Asp, Pro, Gly, His, Ala, Ile, Phe, Lys, Arg, Asn, Glu, Leu, Val, Tyr, Ser, Trp, Met, Thr, suitable isostere, and corresponding D-amino acids. Xaa² is absent, or Xaa² is selected from the group consisting of Gln, Asp, Pro, Gly, His, Ala, Ile, Phe, Lys, Arg, Asn, Glu, Leu, Val, Tyr, Trp, Met, Thr, a suitable isostere and corresponding D-amino acids. Xaa³ is absent, or Xaa³ is selected from the group consisting of an Gln, Asp, Pro, Gly, His, Ala, Ile, Phe, Lys, Arg, Asn, Glu, Leu, Val, Tyr, Trp, Met, Ser and Thr, a suitable isostere and corresponding D-amino acids.

Xaa⁴ is selected from the group consisting of Cys, Pen,; Xaa⁵ is selected from the group consisting of N(alpha)Me-Arg,. Xaa⁶ is selected from the group consisting of Ser,. Xaa⁷ is selected from the group consisting of Asp,. Xaa⁸ is selected from the group consisting of Thr, Xaa⁹ is selected from the group consisting of, Leu,. Xaa¹⁰ is selected from the group consisting of Cys, , Pen,; Xaa¹¹ is selected from the group consisting of Gly, Gln, Asn, Asp, Ala, Ile, Leu, Val, Met, Thr, Lys, Trp, Tyr, His, Glu, Ser, Arg, Pro, Phe, Sar, 1-Nal, 2-Nal, HPhe, Phe(4-F), O-Me-Tyr, dihydro-Trp, Dap, Dab,, Orn, D-Orn, N-Me-Orn, N-Me-Dap, D-Dap, D-Dab, Bip, Ala(3,3diphenyl), Biphenyl-Ala, aromatic ring substituted Phe, aromatic ring substituted Trp, aromatic ring substituted His, hetero aromatic amino acids, N-Me-Lys, N-Me-Lys(Ac), 4-Me-Phe, and corresponding D-amino acids and suitable isostere replacements.

In some embodiments, Xaa¹² is selected from the group consisting of Glu, , β-HGlu, 2-Nal, 1-Nal, D-Asp, Bip, β-HPhe, β-Glu, , suitable isosteres, and corresponding D-amino acids. Xaa¹³ may be absent, or Xaa¹³ is selected from the group consisting of Gln, Pro, Gly, His, Ala, Ile, Phe, Lys, Arg, Leu, Val, Tyr, Trp, Met, Glu, Ser, Asn, Gla, Dap, Dab, Orn, D-Orn, D-Lys, N-Me-Orn, N-Me-Dap, N-Me-Dab, N-Me-Lys, D-Dap, D-Dab, , suitable isosteres, and corresponding D-amino acids. Further, in some embodiments Xaa¹⁴ is absent, or Xaa¹⁴ is selected from the group consisting of natural amino acids, suitable isostere replacements, corresponding D-amino acids.

It is further disclosed herein that, in some embodiments Xaa¹-Xaa⁵, Xaa⁷-Xaa⁹, and Xaa¹¹-Xaa¹² are N(alpha)Methylated. It is further disclosed herein that Xaa⁵ may further be Arg-Me-sym or Arg-Me-asym, and it is further disclosed herein that Xaa¹¹ may be O-Me-Tyr, N-Me-Lys(Ac). Xaa¹¹ may be 4-Me-Phe. It is further disclosed herein that in some instances, Xaa¹-Xaa⁴, and Xaa¹¹-Xaa¹⁴ are acylated. For example, in some instances one or more residues at positions Xaa¹-Xaa⁴, and Xaa¹¹-Xaa¹⁴ are acylated with an acylating organic compound selected from the group consisting of 2-me-Trifluorobutyl, Trifluoropentyl, Acetyl, Octonyl, Butyl, Pentyl, Hexyl, Palmityl, Trifluoromethyl butyric, cyclopentane carboxylic, cyclopropylacetic, 4-fluorobenzoic, 4-fluorophenyl acetic, 3-Phenylpropionic, tetrahedro-2H-pyran-4carboxylic, succinic acid, and glutaric acid.

In some embodiments Xaa¹, Xaa², Xaa³, Xaa¹², Xaa¹³ or Xaa¹⁴ are modified with a suitable linker moiety to form a homo- or hetero-dimer molecule, wherein Formula (I) comprises a dimer formed from two subunits joined by a suitable C- or N-terminal linker selected from the group consisting of DIG, DIG-OH, PEG13, PEG25, PEG1K, PEG2K, PEG3.4K, PEG4K, PEG5K, IDA, IDA-Palm, IDA-Boc, IDA-Ac, IDA-Isovaleric acid, Triazine, Triazine-Boc, Isophthalic acid, 1,3-phenylenediacetic acid, 1,4-phenylenediacetic acid, cyclopropylacetic acid, 4-fluoorobenzoic acid, 4-fluorophenylacetic acid, 3-phenylpropionic acid, succinic acid, biotin, glutaric acid, Azelaic acid, Pimelic acid, Dodecanedioic acid, suitable aliphatics, suitable aromatics, heteroaromatics, and polyethylene glycols having a molecular weight from approximately 400Da to approximately 40,000Da.

One having skill in the art will appreciate that the C- and N-terminal linker moieties disclosed herein are non-limiting examples of suitable, and that the present invention may include any suitable linker moiety. Thus, some embodiments of the present invention comprises a homo- or heterodimer molecule comprised of two monomer subunits selected from the peptide molecules represented by SEQ ID NOs: 1-146, wherein the C- or N-termini of the respective monomers are linked by any suitable linker moiety to provide a dimer molecule having integrin antagonist activity.

In another aspect, it is disclosed hereina composition for treating a patient in need of integrin-antagonist therapy comprising a compound of Formula (I) in combination with a pharmaceutically acceptable carrier.

Yet another aspect of the present invention provides a composition for use in treating a patient in need of α4β7-specific antagonist therapy comprising a compound of Formula (I) having high selectivity for α4β7 integrin in combination with a pharmaceutically acceptable carrier.

Yet another aspect of the present invention provides a composition for use in treating a patient in need of α4β7 -specific antagonist therapy comprising a compound of Formula (I) having high selectivity for α4β7 against α4β1 integrins in combination with a pharmaceutically acceptable carrier.

Yet another aspect of the present invention provides a composition for use in treating a patient in need of α4β7 -specific antagonist therapy comprising a compound of Formula (I) having high selectivity for α4β7 against αEβ7 integrins in combination with a pharmaceutically acceptable carrier.

Yet another aspect of the present invention provides a composition for use in treating a patient in need of α4β7 -specific antagonist therapy comprising a compound of Formula (I) having low selectivity for α4β7 against αEβ7 integrins in combination with a pharmaceutically acceptable carrier.

It is disclosed hereina method for treating a patient in need of integrin-antagonist therapy comprising administering to the patient a therapeutically effective amount of a compound of Formula (I).

Still, yet another aspect of the present invention provides a composition for use the treatment of a disease from ulcerative colitis, Crohn's disease, Celiac disease (nontropical Sprue), enteropathy associated with seronegative arthropathies, microscopic or collagenous colitis, eosinophilic gastroenteritis, colitis associated with radio- or chemo-therapy, and various forms of gastrointestinal cancer. In another embodiment, the condition is pancreatitis, insulin-dependent diabetes mellitus, cholecystitis, cholangitis, pericholangitis, chronic bronchitis, chronic sinusitis, asthma or graft versus host disease. In addition, these compounds may be useful in the prevention or reversal of these diseases when used in combination with currently available therapies, medical procedures, and therapeutic agents.

In yet another aspect, it is disclosed hereina diagnostic method for visualizing and diagnosing a disease comprising administering an orally stable compound of Formula (I) that is further labeled with at least one of a chelating group and a detectable label for use as an *in vivo* imaging agent for non-invasive diagnostic procedures.

### BRIEF DESCRIPTION OF THE DRAWINGS

In order that the manner in which the above-recited and other features and advantages of the invention are obtained will be readily understood, a more particular description of the invention briefly described above will be rendered by reference to specific embodiments thereof which are illustrated in the appended drawings. Understanding that these drawings depict only typical embodiments of the invention and are not therefore to be considered to be limiting of its scope, the invention will be described and explained with additional specificity and detail through the use of the accompanying drawings in which:
Figure 1 is a schematic showing C and N-terminal dimerizations.
Figure 2 is a schematic showing a pair of integrin antagonist monomer subunits according to SEQ ID NO: 47, wherein the subunits are aligned and linked at their respective C-termini by a DIG linker in accordance with a representative embodiment of the present invention.
Figure 3 is a chart demonstrating stability data for integrin antagonist homodimer molecules represented by SEQ ID NOs: 46, 55, 74 and 93, wherein the amino acid sequences 55, 74 and 93 are in accordance with various representative embodiment of the present invention.
Figure 4 is a chart demonstrating potency and selectivity for integrin antagonist monomer and homodimer molecules represented by SEQ ID NOs: 51, 43. 48, 47, 50, and 94, wherein the amino acid sequences 47 and 94 are in accordance with a representative selection of various embodiments of the present invention.

### SEQUENCE LISTING

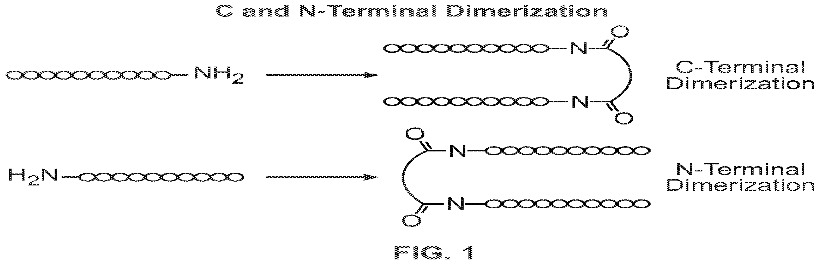

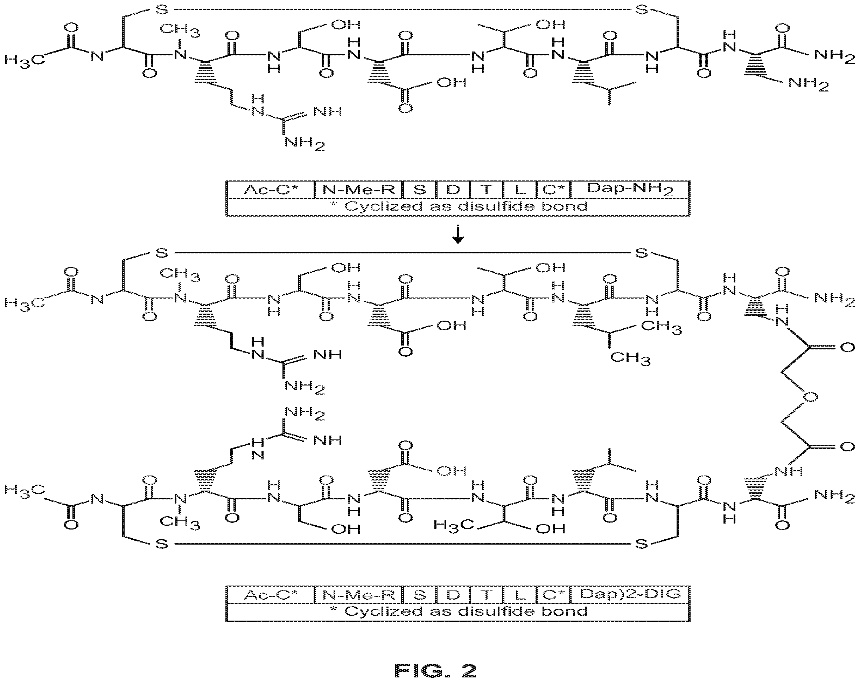

The amino acid sequences listed in the accompanying sequence listing are shown using three letter code for amino acids, as defined in 37 C.F.R. 1.822. Only the monomer subunit sequences are shown, however it is understood that the monomer subunits are dimerized to form peptide dimer molecules, in accordance with the present teaching and as shown generally in Figures 1 and 2. The monomer subunits are dimerized by a suitable linker moiety, as defined herein. Some of the monomer subunits are shown having C- and N-termini that both comprise free amine. Thus, a user must modify the monomer subunit to eliminate either the C- or N-terminal free amine, thereby permitting dimerization at the remaining free amine. Further, in some instances a terminal end of one or more monomer subunits is acylated with an acylating organic compound selected from the group consisting of 2-me-Trifluorobutyl, Trifluoropentyl, Acetyl, Octonyl, Butyl, Pentyl, Hexyl, Palmityl, Trifluoromethyl butyric, cyclopentane carboxylic, cyclopropylacetic, 4-fluorobenzoic, 4-fluorophenyl acetic, 3-Phenylpropionic, tetrahedro-2H-pyran-4carboxylic, succinic acid, and glutaric acid. In some instances, monomer subunits comprise both a free carboxy terminal and a free amino terminal, whereby a user may selectively modify the subunit to achieve dimerization at a desired terminus. One having skill in the art will therefore appreciate that the monomer subunits of the instant invention may be selectively modified to achieve a single, specific amine for a desired dimerization.

It is further understood that the C-terminal residues of the monomer subunits disclosed herein are amides, unless otherwise indicated. Further, it is understood that dimerization at the C-terminal is facilitated by using a suitable amino acid with a side chain having amine functionality, as is generally understood in the art. Regarding the N-terminal residues, it is generally understood that dimerization may be achieved through the free amine of the terminal residue, or may be achieved by using a suitable amino acid side chain having a free amine, as is generally understood in the art.

In the accompanying sequence listing:
SEQ ID NO: 1 shows a monomer subunit of a dimer compound of Formula (I).
SEQ ID NO: 2 shows a monomer subunit of a dimer compound of Formula (II).
SEQ ID NOs: 1-38, 46-52, 54-135, and 137-146 show amino acid sequences of monomer subunits that are dimerized to form various dimer compounds, wherein the amino acid sequences 47, 54, 55, 57, 58, 73, 74, 77, 78, 79, 80-82, 88, 90-97, 101, 104, 106, 107, 110, 120, 127, 133, 135, 137 and 140 arein accordance with the present invention, wherein these sequences have been substituted with an N(alpha)methylated arginine.
SEQ ID NO: 136 shows an amino acid sequence of a monomer subunit that is dimerized to form a dimer compound, wherein this sequence has been substituted with an N(alpha)methylated lysine.
SEQ ID NOs: 1-38 are general sequences that may be dimerized at their respective C- or N-termini to form various dimer compounds..
SEQ ID NOs: 39-45, 47, 48, 51-58, 61, 63, 65-86, 88-97, and 102-146 show amino acid sequences of monomer subunits, wherein the amino acid sequences 47, 54, 55, 57, 58, 73, 74, 77, 78, 79, 80-82, 88, 90-97, 104, 106, 107, 110, 120, 127, 133, 135, 137, 140 may be dimerized at their respective C-termini to form various dimer compounds in accordance with the present invention. Generally, these amino acid sequences are acylated at their N-termini prior to dimerization using one of the acylating organic compounds and methods disclosed herein, including but not limited to cyclopropylacetic acid, 4-Fluorobenzoic acid, 4-fluorophenylacetic acid, 3-Phenylpropionic acid, Succinic acid, Glutaric acid, Cyclopentane carboxylic acid, 3,3,3-trifluoropropeonic acid, 3-Fluoromethylbutyric acid, Tetrahedro-2H-Pyran-4-carboxylic acid.
SEQ ID NOs: 46, 49, 50, 59, 60, 62, 64, 87, and 98-101 102-103, 113 -119 show amino acid sequences of monomer subunits that may be dimerized at their respective N-termini to form various dimer compounds, wherein amino acid sequence 101 may be dimerized at the respective N-terminus to form a dimer compound in accordance with the present invention..

### DETAILED DESCRIPTION OF THE PREFERRED EMBODIMENTS

As used herein, the singular forms "a," "and" and "the" include plural references unless the context clearly dictates otherwise.

As used in the present specification the following terms have the meanings indicated:

The term "peptide," as used herein, refers broadly to a sequence of two or more amino acids joined together by peptide bonds. It should be understood that this term does not connote a specific length of a polymer of amino acids, nor is it intended to imply or distinguish whether the polypeptide is produced using recombinant techniques, chemical or enzymatic synthesis, or is naturally occurring.

The term "DRP," as used herein, refers to disulfide rich peptides.

The term "dimer," as used herein, refers broadly to a peptide comprising two or more subunits, wherein the subunits are DRPs that are linked at their C- or N-termini. Dimers of the present invention may include homodimers and heterodimers and function as integrin antagonists.

The term "L-amino acid," as used herein, refers to the "L" isomeric form of a peptide, and conversely the term "D-amino acid" refers to the "D" isomeric form of a peptide. The amino acid residues described herein are preferred to be in the "L" isomeric form, however, residues in the "D" isomeric form can be substituted for any L-amino acid residue, as long as the desired functional is retained by the peptide.

The term "NH2," as used herein, refers to the free amino group present at the amino terminus of a polypeptide. The term "OH," as used herein, refers to the free carboxy group present at the carboxy terminus of a peptide. Further, the term "Ac," as used herein, refers to Acetyl protection through acylation of the C- or N-terminus of a polypeptide.

The term "carboxy," as used herein, refers to -CO₂H.

The term "isostere replacement," as used herein, refers to any amino acid or other analog moiety having chemical and/or structural properties similar to a specified amino acid.

The term "cyclized," as used herein, refers to a reaction in which one part of a polypeptide molecule becomes linked to another part of the polypeptide molecule to form a closed ring, such as by forming a disulfide bridge or other similar bond.

The term "subunit," as used herein, refers to one of a pair of polypeptides monomers that are joined at the C- or N- terminus to form a dimer peptide composition.

The term "dimer," as used herein, refers to a chemical entity consisting of two structurally similar monomers joined by terminus bonds and/or a terminus linker.

The term "linker," as used herein, refers broadly to a chemical structure that is capable of linking together a plurality of peptide monomer subunits to form a dimer.

The term "receptor," as used herein, refers to chemical groups of molecules on the cell surface or in the cell interior that have an affinity for a specific chemical group or molecule. Binding between dimer peptides and targeted integrins can provide useful diagnostic tools.

The term "integrin-related diseases," as used herein, refer to indications that manifest as a result of integrin binding, and which may be treated through the administration of an integrin antagonist.

The term "pharmaceutically acceptable salt," as used herein, represents salts or zwitterionic forms of the compounds of the present invention which are water or oil-soluble or dispersible, which are suitable for treatment of diseases without undue toxicity, irritation, and allergic response; which are commensurate with a reasonable benefit/risk ratio, and which are effective for their intended use. The salts can be prepared during the final isolation and purification of the compounds or separately by reacting an amino group with a suitable acid. Representative acid addition salts include acetate, adipate, alginate, citrate, aspartate, benzoate, benzenesulfonate, bisulfate, butyrate, camphorate, camphorsulfonate, digluconate, glycerophosphate, hemisulfate, heptanoate, hexanoate, formate, fumarate, hydrochloride, hydrobromide, hydroiodide, 2-hydroxyethansulfonate (isethionate), lactate, maleate, mesitylenesulfonate, methanesulfonate, naphthylenesulfonate, nicotinate, 2-naphthalenesulfonate, oxalate, pamoate, pectinate, persulfate, 3-phenylproprionate, picrate, pivalate, propionate, succinate, tartrate, trichloroacetate, trifluoroacetate, phosphate, glutamate, bicarbonate, para-toluenesulfonate, and undecanoate. Also, amino groups in the compounds of the present invention can be quaternized with methyl, ethyl, propyl, and butyl chlorides, bromides, and iodides; dimethyl, diethyl, dibutyl, and diamyl sulfates; decyl, lauryl, myristyl, and steryl chlorides, bromides, and iodides; and benzyl and phenethyl bromides. Examples of acids which can be employed to form therapeutically acceptable addition salts include inorganic acids such as hydrochloric, hydrobromic, sulfuric, and phosphoric, and organic acids such as oxalic, maleic, succinic, and citric.

The term "N(alpha)Methylation", as used herein, describes the methylation of the alpha amine of an amino acid, also generally termed as an N-methylation.

The term "sym methylation" or "Arg-Me-sym", as used herein, describes the symmetrical methylation of the two nitrogens of the guanidine group of arginine. Further, the term "asym methylation" or "Arg-Me-asym" describes the methylation of a single nitrogen of the guanidine group of arginine.

The term "acylating organic compounds", as used herein refers to various compounds with carboxylic acid functionality that are used to acylate the N-terminus of an amino acid subunit prior to forming a C-terminal dimer. Non-limiting examples of acylating organic compounds include cyclopropylacetic acid, 4-Fluorobenzoic acid, 4-fluorophenylacetic acid, 3-Phenylpropionic acid, Succinic acid, Glutaric acid, Cyclopentane carboxylic acid, 3,3,3-trifluoropropeonic acid, 3-Fluoromethylbutyric acid, Tetrahedro-2H-Pyran-4-carboxylic acid.

All peptide sequences are written according to the generally accepted convention whereby the α-N-terminal amino acid residue is on the left and the α-C-terminal is on the right. As used herein, the term "α-N-terminal" refers to the free α-amino group of an amino acid in a peptide, and the term "α-C-terminal" refers to the free α-carboxylic acid terminus of an amino acid in a peptide.

For the most part, the names of naturally occurring and non-naturally occurring aminoacyl residues used herein follow the naming conventions suggested by the IUPAC Commission on the Nomenclature of Organic Chemistry and the IUPAC-IUB Commission on Biochemical Nomenclature as set out in "Nomenclature of α-Amino Acids (Recommendations, 1974)" Biochemistry, 14(2), (1975). To the extent that the names and abbreviations of amino acids and aminoacyl residues employed in this specification and appended claims differ from those suggestions, they will be made clear to the reader. Some abbreviations useful in describing the invention are defined below in the following Table 1.

**TABLE 1**

| Abbreviation | Definition |
|---|---|
| DIG | DIGlycolic acid (Linker) |
| Dap | Diaminopropionic acid |
| Dab | Diaminobutyric acid |
| Pen | Penicillamine |
| Sar | Sarcosine |
| Cit | Citroline |
| Cav | Cavanine |
| 4-Guan | 4-Guanidine-Phenylalanine |
| N-Me-Arg; N(alpha)Methylation | N-Methyl-Arginine |
| Ac- | Acetyl |
| 2-Nal | 2-Napthylalanine |
| 1-Nal | 1-Napthylalanine |
| Bip | Biphenylalanine |
| O-Me-Tyr | Tyrosine (O-Methyl) |
| N-Me-Lys | N-Methyl-Lysine |
| N-Me-Lys (Ac) | N-e-Acetyl-D-lysine |
| Ala (3,3 diphenyle) | 3,3 diphenyl alanine |
| NH2 | Free Amine |
| CONH2 | Amide |
| COOH | Acid |
| Phe (4-F) | 4-Fluoro-Phenylanine |
| PEG13 | Bifunctional PEG linker with 13 PolyEthylene Glycol units |
| PEG25 | Bifunctional PEG linker with 25 PolyEthylene Glycol units |
| PEG1K | Bifunctional PEG linker with PolyEthylene Glycol Mol wt of 1000Da |
| PEG2K | Bifunctional PEG linker with PolyEthylene Glycol Mol wt of 2000Da |
| PEG3.4K | Bifunctional PEG linker with PolyEthylene Glycol Mol wt of 3400Da |
| PEG5K | Bifunctional PEG linker with PolyEthylene Glycol Mol wt of 5000Da |
| IDA | β-Ala-Iminodiacetic acid (Linker) |
| IDA-Palm | β-Ala (Palmityl)-Iminodiacetic acid |
| HPhe | Homo Phenylalanine |
| Ahx | Aminohexanoic acid |
| DIG-OH | Glycolic monoacid |
| Triazine | Amino propyl Triazine di-acid |
| Boc-Triazine | Boc-Triazine di-acid |
| Trifluorobutyric acid | Acylated with 4,4,4-Trifluorobutyric acid |
| 2-Methly-trifluorobutyric acid | acylated with 2-methy-4,4,4-Butyric acid |
| Trifluorpentanoic acid | Acylated with 5,5,5-Trifluoropentnoic acid |
| *1,4-*Phenylenediacetic acid | *para-*Phenylenediacetic acid (Linker) |
| 1,3- Phenylenediacetic acid | *meta-*Phenylenediacetic acid (Linker) |
| DTT | Dithiothreotol |
| Nle | Norleucine |
| β-HTrp | β-homoTrypophane |
| β-HPhe | β-homophenylalanine |
| Phe(4-CF3) | 4-Trifluoromethyl Phenylalanine |
| β-Glu | β-Glutamic acid |
| β-HGlu | β-homoglutamic acid |
| 2-2-Indane | 2-Aminoindane-2-carboxylic acid |
| 1-1-Indane | 1-Aminoindane-1-carboxylic acid |
| HCha | homocyclohexyl Alanine |
| Cyclobutyl | Cyclobutylalanine |
| β-HPhe | β-homophenylalanine |
| HLeu | Homoleucine |
| Gla | Gama-Carboxy-Glutamic acid |

The present invention relates generally to peptides that have been shown to have integrin antagonist activity. In particular, the present invention relates to various peptide dimers comprising hetero- or homo-monomer subunits that each form cyclized structures through disulfide bonds. The monomer subunits are linked at either their C- or N-termini, as shown in Figure 1. The cyclized structure of each subunit has been shown to increase potency and selectivity of the dimer molecules, as discussed below. A non-limiting, representative illustration of the cyclized structure is shown in Figure 2.

The linker moieties of the present invention may include any structure, length, and/or size that is compatible with the teachings herein. In at least one embodiment, a linker moiety is selected from the non-limiting group consisting of DIG, PEG4, PEG4-biotin, PEG13, PEG25, PEG1K, PEG2K, PEG3.4K, PEG4K, PEG5K, IDA, ADA, Boc-IDA, Glutaric acid, Isophthalic acid, 1,3-phenylenediacetic acid, 1,4-phenylenediacetic acid, 1,2-phenylenediacetic acid, Triazine, Boc-Triazine, IDA-biotin, PEG4-Biotin, AADA, suitable aliphatics, aromatics, heteroaromatics, and polyethylene glycol based linkers having a molecular weight from approximately 400Da to approximately 40,000Da. Non-limiting examples of suitable linker moieties are provided in Table 2.

**TABLE 2**

| Abbrivation | Discription | Structure |
|---|---|---|
| DIG | DIGlycolic acid, | 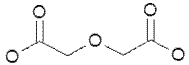 |
| PEG4 | Bifunctional PEG linker with 4 PolyEthylene Glycol units | 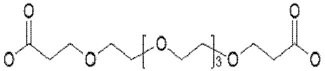 |
| PEG13 | Bifunctional PEG linker with 13 PolyEthylene Glycol units | 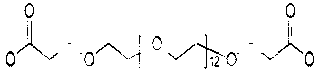 |
| PEG25 | Bifunctional PEG linker with 25 PolyEthylene Glycol units | 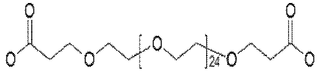 |
| PEG1K | Bifunctional PEG linker with PolyEthylene Glycol Mol wt of 1000Da | |
| PEG2K | Bifunctional PEG linker with PolyEthylene Glycol Mol wt of 2000Da | |
| PEG3.4K | Bifunctional PEG linker with PolyEthylene Glycol Mol wt of 3400Da | |
| PEG5K | Bifunctional PEG linker with PolyEthylene Glycol Mol wt of 5000Da | |
| DIG | DIGlycolic acid, | 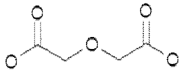 |
| IDA | β-Ala-Iminodiacetic acid | 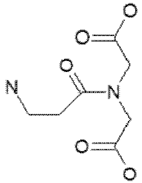 |
| Boc-I DA | Boc-β-Ala-Iminodiacetic acid | 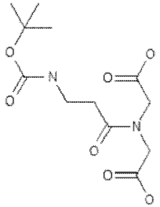 |
| Ac-IDA | Ac-β-Ala-Iminodiacetic acid | 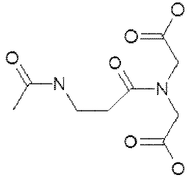 |
| IDA-Palm | Palmityl-β-Ala-Iminodiacetic acid | 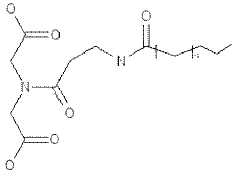 |
| GTA | Glutaric acid | 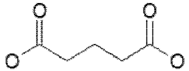 |
| PMA | Pemilic acid | 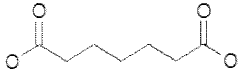 |
| AZA | Azelaic acid | 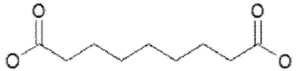 |
| DDA | Dodecanedioic acid | 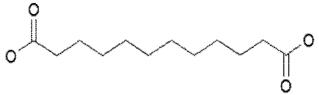 |
| IPA | Isopthalic aicd | 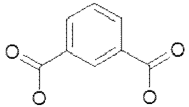 |
| 1,3-PDA | 1,3-Phenylenediacetic acid | 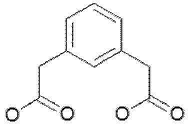 |
| 1,4-PDA | *1,4-*Phenylenediacetic acid | 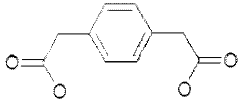 |
| 1,2-PDA | 1,2-Phenylenediacetic acid | 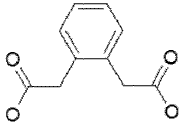 |
| Triazine | Amino propyl Triazine di-acid | 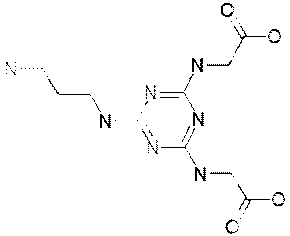 |
| Boc-Triazine | Boc-Triazine di-acid | 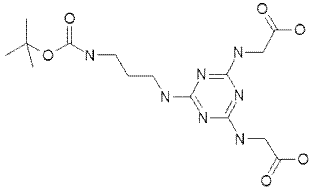 |
| ADA | Amino diacetic acid | 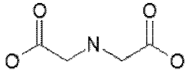 |
| AADA | n-Acetyl amino acetic acid | 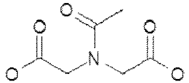 |
| PEG4-Biotin | PEG4-Biotin (Product number 10199, QuantaBioDesign) | 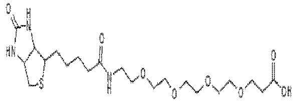 |
| IDA-Biotin | N-Biotin-β-Ala-Iminodiacetic acid | 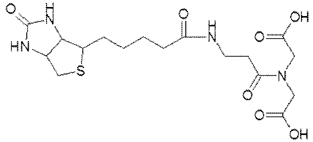 |

The present invention further includes various peptides that have been substituted with various modified amino acids. For example, some peptides include Dab, Dap, Pen, , Cav, HLeu, 2-Nal, d-1-Nal, d-2-Nal, Bip, O-Me-Tyr, β-HTrp, β-HPhe, Phe (4-CF3), , 1, β-HPhe, , , HPhe, 1-Nal, , homo amino acids, D-amino acids, 3-3-diPhe, , , β-HPhe, β-Glu, , and various N-methylated amino acids. One having skill in the art will appreciate that additional substitutions may be made to achieve similar desired results, and that such substitutions are within the teaching and spirit of the present invention.

In one aspect, the present invention relates to dimer compounds, each subunit of the dimer compound comprising the structure of Formula (I) Xaa¹-Xaa²-Xaa³-Xaa⁴-Xaa⁵-Xaa⁶-Xaa⁷-Xaa⁸-Xaa⁹-Xaa¹⁰-Xaa¹¹-Xaa¹²-Xaa¹³-Xaa¹⁴ SEQ ID NO: 1), wherein Xaa¹-Xaa²-Xaa³-Xaa⁴-Xaa⁵-Xaa⁶-Xaa⁷-Xaa⁸-Xaa⁹-Xaa¹⁰ (SEQ ID NO: 2), or a pharmaceutically acceptable salt thereof, further represent a subunit of a homo- or heterodimer molecule, wherein each subunit of the dimer molecule comprises 10 amino acids, and wherein Xaa¹-Xaa¹⁰ of Formula (II) corresponds to Xaa⁴-Xaa¹³ of Formula (I). Further, each subunit of Formula (I) and Formula (II) comprises a disulfide bond between Xaa⁴ and Xaa¹⁰, and Xaa1 and Xaa⁷, respectively.

Some sequences of the present invention are derived from the general sequences provided in Formula (I) and Formula (II). For example, the N-terminus of a decapeptide represented by Xaa⁴-Xaa¹³ of Formula (I) can be modified by one to three suitable groups, as represented by Xaa¹, Xaa², and Xaa³ of Formula (I). The N-terminus may further be acylated. In some instances, the N-terminus further comprises a suitable linker moiety to facilitate linking together two monomer subunits to form an N-terminal dimer molecule.

Similarly, the C-terminus of the decapeptide represented by Formula (I) can be modified by a suitable group. The C-terminus may further be acylated. In some instances, the C-terminus further comprises a suitable linker moiety to facilitate linking together two monomer subunits to form a C-terminal dimer molecule.

In some embodiments, Xaa¹, Xaa², and Xaa³ of Formula (I) are absent. In other embodiments, Xaa¹ is absent, and Xaa² and Xaa³ represent suitable groups for modifying the N-terminus of the decapeptide, wherein the decapeptide is represented by residues Xaa⁴-Xaa¹³ of Formula (I), and residues Xaa¹-Xaa¹⁰ of Formula (II). Further, in some embodiments Xaa¹ and Xaa² are absent, and Xaa³ represents a single suitable group for modifying the N-terminus of the decapeptide subunit.

With continued reference to the general formula of Formula (I), Xaa¹ is an amino acyl residue selected from the group consisting of Gln, Asp, Pro, Gly, His, Ala, Ile, Phe, Lys, Arg, Asn, Glu, Leu, Val, Tyr, Trp, Met, Thr, suitable isosteres, and corresponding D-amino acids. In some embodiments, Xaa¹ is acylated or free NH₂. In other embodiments, Xaa¹ is absent.

Xaa² is an amino acyl residue selected from the group consisting of Gln, Asp, Pro, Gly, His, Ala, Ile, Phe, Lys, Arg, Asn, Glu, Leu, Val, Ser, Tyr, Trp, Met, Thr, suitable isosteres, and corresponding D-amino acids. When Xaa¹ is absent, Xaa² is the N-terminus.

Xaa³ is an amino acyl residue selected from the group consisting of Gln, Asp, Pro, Gly, His, Ala, Ile, Phe, Lys, Arg, Asn, Glu, Leu, Val, Tyr, Trp, Met, Thr, Ser, and corresponding D-amino acids. When Xaa¹ and Xaa² are absent, Xaa³ is the N-terminus. In other embodiments, Xaa¹-Xaa³ are absent, wherein Xaa⁴ is the N-terminus.

In some embodiments, the N-terminal residue of Formula (I) further comprises a linker moiety selected from the group consisting of DIG, DIG-OH, PEG13, PEG25, PEG1K, PEG2K, PEG3.4K, PEG4K, PEG5K, IDA, IDA-Palm, IDA-Boc, IDA-Isovaleric acid, Triazine, Triazine-Boc, Isophthalic acid, 1,3-phenylenediacetic acid, 1,4-phenylenediacetic acid, cyclopropylacetic acid, 4-fluoorobenzoic acid, 4-fluorophenylacetic acid, 3-phenylpropionic acid, succinic acid, biotin, glutaric acid, Azelaic acid, Pimelic acid, Dodecanedioic acid, suitable aliphatics, suitable aromatics, heteroaromatics, and polyethylene glycols having a molecular weight from approximately 400Da to approximately 40,000Da. Further, in some embodiments Xaa¹-Xaa⁴ are acylated.

In some embodiments, Xaa⁴ is an amino acyl residue or analog selected from the group consisting of Cys, Pen.

When Xaa⁴ and Xaa¹⁰ are either Cys or Pen, each subunit of the dimer is cyclized though a disulfide bond between Xaa⁴ and Xaa¹⁰. Preferably, in one embodiment Xaa⁴ is Cys. In another embodiment, preferably Xaa⁴ is Pen.

Xaa⁵ is an amino acyl residue or analog selected from the group consisting of, N-Me-Arg,. In some embodiments, Xaa⁵ is N(alpha)Methylated. Preferably, Xaa⁵ is N-Me-Arg..

Xaa⁶ is an amino acyl residue or analog selected from the group consisting of Ser,. Preferably, Xaa⁶ is Ser.

Xaa⁷ is an amino acyl residue or analog selected from the group consisting of Asp, Preferably, Xaa⁷ is Asp.

Xaa⁸ is an amino acyl residue or analog selected from the group consisting of Thr,. Preferably, Xaa⁸ is Thr.

Xaa⁹ is an amino acyl residue or analog selected from the group consisting of Leu,. Preferably, Xaa⁹ is Leu.

Xaa¹⁰ is an amino acyl residue selected from the group consisting of Cys, Pen. In at least one embodiment, Xaa¹⁰ is Pen. When Xaa¹⁰ and Xaa⁴ are both either Cys or Pen, each subunit of the dimer is cyclized through a disulfide bond between Xaa⁴ and Xaa¹⁰. When Xaa¹¹ is absent, Xaa¹⁰ is the C-terminus of the subunit. Preferably, in one embodiment Xaa¹⁰ is Pen. In another embodiment, Xaa¹⁰ is preferably Cys.

Xaa¹¹ is an amino acyl residue selected from the group consisting of Gly, Gln, Asn, Asp, Ala, Ile, Leu, Val, Met, Thr, Lys, Trp, Tyr, His, Glu, Ser, Arg, Pro, Phe, Sar, 1-Nal, 2-Nal, , HPhe, Phe(4-F), O-Me-Tyr, dihydro-Trp, Dap, Dab, Orn, D-Orn, , N-Me Lys, D-Dap, D-Dab, D-Lys, , Bip, Ala(3,3diphenyl), Biphenyl-Ala, D-Phe, D-Trp, D-Tyr, D-Glu, D-His, D-Lys, 3,3-diPhe, β-HTrp, F(4CF3), 4-Me-Phe, aromatic ring substituted Phe, aromatic ring substituted Trp, aromatic ring substituted His, hetero aromatic amino acids, N-Me-Lys, N-Me-Lys(Ac), 4-Me-Phe, and corresponding D-amino acids and suitable isostere replacements. It is disclosed herein at least one embodiment , wherein Xaa¹¹ and Xaa¹² are absent. When Xaa¹² and Xaa¹³ are absent, Xaa¹¹ is the C-terminus of the subunit. It is disclosed herein, that when Xaa¹¹ is the C-terminus of the subunit, Xaa¹¹ may be modified to include a linker moiety in accordance with the present invention. Preferably, Xaa¹¹ is Trp. In other embodiments Xaa¹¹ is N(alpha)Methylated. Further, in some embodiments Xaa¹¹ is acylated.

Xaa¹² is an amino acyl residue selected from the group consisting of Glu, , , 2-Nal. 1-Nal, Bip, β-Glu, , D-Asp suitable isosters, and corresponding D-amino acids. When Xaa¹³ and Xaa¹⁴ are absent, Xaa¹² is the C-terminus of the subunit.Some embodiments are disclosed herein, wherein Xaa¹² is absent. When Xaa¹² is the C-terminus of the subunit, Xaa¹² may be modified to include a linker moiety in accordance with the present invention. Further, it is disclosed herein that in some embodiments Xaa¹² is N(alpha)Methylated. Further , it it is disclosed herein that in some embodiments Xaa¹² selected from the group consisting of Lys, D-Lys, and N-Me-Lys. Preferably, Xaa¹² is Glu, and β-HGlu,.

Xaa¹³ is an amino acyl residue selected from the group consisting of Gln, Pro, Gly, His, Ala, Ile, Phe, Lys, Arg, Leu, Val, Tyr, Trp, Met, Glu, Ser, Asn, Gla, Dap, Dab, Orn, D-Orn, N-Me-Orn, N-Me-Dap, N-Me-Dab, N-Me Lys, D-Dap, D-Dab, D-Lys, N-Me-D-Lys, suitable isosteres, and corresponding D-amino acids. In some embodiments, when Xaa¹⁴ is absent, Xaa¹³ is the C-terminus. When Xaa¹³ is the C-terminus of the subunit, Xaa¹³ may be modified to include a linker moiety in accordance with the present invention. In at least one embodiment, Xaa¹³ is Lys. In other embodiments, Xaa¹³ is absent. Further, in some embodiments Xaa¹³ is N(alpha)Methylated. Further, it is disclosed herein that in some embodiments Xaa¹³ is acylated. Further still, in some embodiments Xaa¹³ is D-Lys.

Xaa¹⁴ is an amino acyl residue selected from the group consisting of natural amino acids, , suitable isostere replacements, corresponding D-amino acids, and corresponding N-Methyl amino acids. In at least one embodiment, Xaa¹⁴ is absent. In at least one embodiment, Xaa¹⁴ is the C-terminus. When Xaa¹⁴ is the C-terminus of the subunit, Xaa14 may be modified to include a linker moiety in accordance with the present invention. Further, in some embodiments Xaa¹⁴ is N(alpha)Methylated.

In some embodiments, the C-terminal residue of Formula (I) further comprises a linker moiety selected from the group consisting of DIG, PEG13, PEG25, PEG1K, PEG2K, PEG3.4K, PEG4K, PEG5K, IDA, IDA-Palm, IDA-Boc, IDA-Isovaleric acid, Triazine, Triazine-Boc, Isophthalic acid, 1,3-phenylenediacetic acid, 1,4-phenylenediacetic acid, glutaric acid, Azelaic acid, Pimelic acid, Dodecanedioic acid, suitable aliphatics, aromatics, heteroaromatics, and polyethylene glycol based linkers having a molecular weight from approximately 400Da to approximately 40,000Da.

Some embodiments of the present invention further include a peptide homodimer or heterodimer molecule, wherein each subunit of the dimer molecule comprises an amino acid sequence represented by at least one of SEQ ID NOs: 47, 54, 55, 57, 58, 73, 74, 77, 78, 79, 80-82, 88, 90-97, 101, 104, 106, 107, 110, 120, 127, 133, 135, 137, 140. Other embodiments comprise a peptide homodimer or heterodimer molecule, wherein each subunit of the dimer molecule comprises an amino acid sequence comprising an N(alpha)methylated arginine residue, as represented by at least one of SEQ ID NOs: 47, 54, 55, 57, 58, 73, 74, 77, 78, 79, 80-82, 88, 90-97, 101, 104, 106, 107, 110, 120, 127, 133, 135, 137, 140. Further, it is disclosed herein that at least one embodiment comprises a peptide homodimer or heterodimer molecule, wherein at least one subunit of the dimer molecule comprises an amino acid sequence comprising an N(alpha)Methylated lysine residue, as represented by SEQ ID NO: 136.

Further, some embodiments of the present invention comprise a peptide homodimer or hetereodimer molecule, wherein each subunit of the dimer molecule is cyclized through a disulfide bond, as represented by at least one of SEQ ID NOs: 47, 54, 55, 57, 58, 73, 74, 77, 78, 79, 80-82, 88, 90-97, 101, 104, 106, 107, 110, 120, 127, 133, 135, 137, 140. Further, other embodiments are disclosed herein, wherein a peptide homo- or heterodimer molecule is provided, wherein each subunit of the dimer molecule is cyclized through a lactam bond, as represented by at least one of SEQ ID NOs: 1 and 2, wherein Xaa4 and XaalO are selected from the group consisting of Lys, HLys, Orn, Dap, Dab, Asp, HAsp, Glu and HGlu.

### Dimer Structure and Biological Activity

The present invention provides various novel antagonist disulfide peptide dimers. These compounds have been tested to more clearly characterize the increased affinity for α4β7 binding, increased selectivity against α4β1, and increased stability in simulated intestinal fluid (SIF). These novel antagonist molecules demonstrate high binding affinity with α4β7, thereby preventing binding between α4β7 and the MAdCAM ligand. Accordingly, these antagonist peptides have shown to be effective in eliminating and/or reducing the inflammation process in various experiments.

The present invention thus provides various dimer peptide compounds which bind or associate with the α4β7 integrin, in serum and SIF, to disrupt or block binding between α4β7 and the MAdCAM ligand. The various peptide compounds of the invention may be constructed solely of natural amino acids. Alternatively, the peptide compounds may include non-natural amino acids including, but not limited to, modified amino acids. Modified amino acids include natural amino acids which have been chemically modified to include a group, groups, or chemical moiety not naturally present on the amino acid. The peptide compounds of the invention may additionally include D-amino acids. Still further, the peptide compounds of the invention may include amino acid analogs.

Some antagonist disulfide dimers have been shown to be gastrointestinal stable and provide high levels of specificity and affinity for the α4β7 integrin. Some implementations of the present invention provide a disulfide dimer comprising a half-life of greater than 60 minutes when exposed to simulated intestinal fluids (SIF). Some implementations further provide a DRP comprising a half-life from approximately 1 minute to approximately 60 minutes.

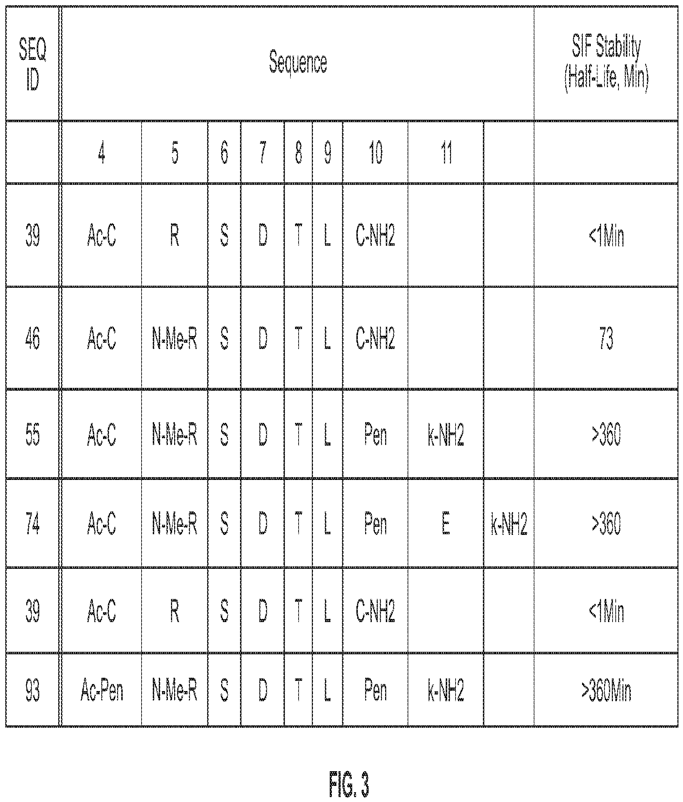

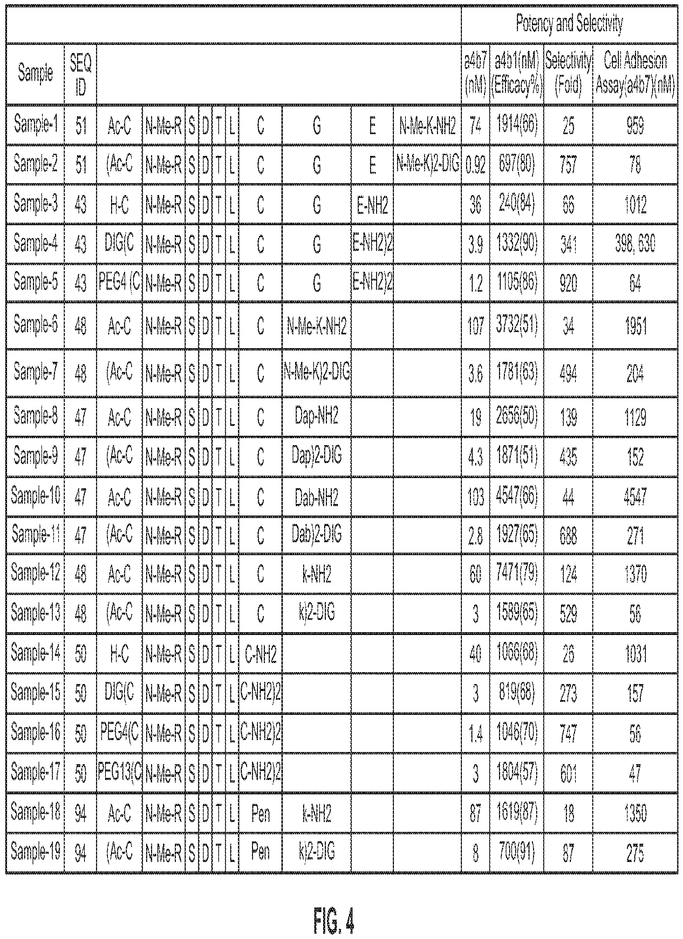

The compounds of the present invention are homo- or heterodimers formed by linking two subunit monomers at their C- or N-termini. Dimerization of the monomer subunits represented by SEQ ID NOs: 47, 54, 55, 57, 58, 73, 74, 77, 78, 79, 80-82, 88, 90-97, 101, 104, 106, 107, 110, 120, 127, 133, 135, 137, 140demonstrate increased potency over their non-dimerized, monomer analogs. Some dimer compounds of the present invention demonstrated further increased potency as a result of substituting various natural amino acyl residues with N-methylated analog residues. For example, SEQ ID NOs.: 47, 54, 55, 57, 58, 73, 74, 77, 78, 79, 80-82, 88, 90-97, 101, 104, 106, 107, 110, 120, 127, 133, 135, 137, 140 represent subunit monomers sequences that were substituted with N(alpha)methylated arginine. Further still, some dimer compounds of the present invention comprise monomer subunits that undergo independent cyclization, whereby the cyclized structures demonstrate increased stability over their non-cyclized monomer and dimer analogs. Specific examples and data illustrating these improvements are provided in Figures 3 and 4.

Referring now to Figure 3, a chart is provided which includes various data illustrating increased stability for various non-limiting sample homodimer molecules in accordance with the instant invention. Simulated Intestinal Fluid (SIF) Stability assays were performed for the majority of the instant monomer peptides, and their respective homodimer molecules. A selective sampling of these results is provided in Figure 3.

According to the protocols discussed herein, applicant successfully synthesized, purified and dimerized the majority of the integrin antagonist dimer molecules represented by SEQ ID NOs: 39-146 to form homodimers.

Dimerization of the monomer disulfide peptide subunits generally demonstrated increase stability, as compared to the monomer disulfide subunit peptides. Further, substitutions at arginine with N-Me-Arg increased half-life substantially in SIF, as demonstrated by the N(alpha)Methylated and non-methylated variations of SEQ ID NO: 46. In some embodiments, substitution of Cys with Penicillamine (Pen) increased stability significantly in simulated intestinal fluids (SIF), as demonstrated by SEQ ID NOs: 55, 74 and 93 when compared to SEQ ID NO: 46 with Cys. The substitution of Cys with Pen also increased stability under reduced conditions (DTT) indicating improved gastric stability.

Referring now to Figure 4, a chart is provided which includes various data illustrating increased potency and selectivity for various non-limiting sample homodimer molecules in accordance with the instant invention. Potency assays were performed for all of the monomer peptides, and their respective homodimer molecules, represented by SEQ ID NOs: 39-146. Selectivity assays were performed for majority of the monomer peptides, and their respective homodimer molecules, represented by SEQ ID NOs: 39-146. A selective sampling of these results is provided in Figure 4 wherein the homodimer peptides are represented by Samples 2, 4, 5, 7, 9, 11, 13, 15, 16, 17 and 19, and the respective monomer subunits molecules are represented by Samples 1, 3, 6, 8, 10, 12, 14, and 18. Through dimerization, significant improvement in potency was achieved for α4β7 in ELISA as well as cell adhesion assays. In addition, dimerization lead to significant improvement achieved in selectivity against α4β1 through improved potency for α4β7. The peptides also demonstrate low efficacy for α4β1 when compared to α4β7, thereby indicating selectivity against α4β7.

According to the protocols discussed herein, applicant successfully synthesized, purified and dimerized majority of the integrin antagonist dimer molecules represented by SEQ ID NOs: 39-146 to form homodimers. Each of these molecules was subjected to an α4β7-MAdCAM Competition ELISA assay, an α4β1-VCAM Competition ELISA assay, an α4β7-MadCAM cell adhesion assay. For many sequences, these assays were also performed on both the monomer subunit and dimer molecules. A small sampling of these results is provided in Figure 4.

Dimerization of the monomer disulfide peptides subunits generally demonstrated increased affinity for a4b7 and/or decreased affinity for a4b1 leading to increased selectivity against a4b1, as compared to the monomer disulfide subunit peptides.

Upon C- and N-terminal dimerization, a significant improvement in potency for α4β7 was also observed. In addition dimerization also lead to either decrees of potency for α4β1 or no significant change in potency leading to increased selectivity for α4β7 in ELISA and cell adhesion assays. When Arg is replaced with N-Me-Arg, a significant improvement in potency for α4β7 was shown in both ELISA and cell adhesion assays. N(alpha)methylation further demonstrated increased molecular stability. One having skill in the art will appreciate that methylated isosteres of arginine may further demonstrate similar increases in potency and/or stability.

### Compositions

As discussed above, integrins are heterodimers that function as cell adhesion molecules. The α4 integrins, α4β1 and α4β7, play essential roles in lymphocyte migration throughout the gastrointestinal tract. They are expressed on most leukocytes, including B and T lymphocytes, monocytes, and dendritic cells, where they mediate cell adhesion via binding to their respective primary ligands, namely vascular cell adhesion molecule (VCAM) and mucosal addressin cell adhesion molecule (MAdCAM). VCAM and MAdCAM differ in binding specificity, in that VCAM binds both α4β1 and α4β7, while MAdCAM is highly specific for α4β7.

Differences in the expression profiles of VCAM and MAdCAM provide the most convincing evidence of their role in inflammatory diseases. Both are constitutively expressed in the gut; however, VCAM expression extends into peripheral organs, while MAdCAM expression is confined to organs of the gastrointestinal tract. In addition, elevated MAdCAM expression in the gut has now been correlated with several gut-associated inflammatory diseases, including Crohn's disease, ulcerative colitis, and hepatitis C.

The compounds of the invention, including but not limited to those specified in the examples, possess integrin-antagonist activity. In one embodiment, the condition or medical indication comprises at least one of Inflammatory Bowel Disease (IBD), ulcerative colitis, Crohn's disease, Celiac disease (*nontropical Sprue*), enteropathy associated with seronegative arthropathies, microscopic or collagenous colitis, eosinophilic gastroenteritis, colitis associated with radio- or chemo-therapy, colitis associated with disorders of innate immunity as in leukocyte adhesion deficiency-1, chronic granulomatous disease, glycogen storage disease type 1b, Hermansky-Pudlak syndrome, Chediak-Higashi syndrome, and Wiskott-Aldrich Syndrome, or pouchitis resulting after proctocolectomy and ileoanal anastomosis and various forms of gastrointestinal cancer, osteoporosis, arthritis, multiple sclerosis, chronic pain, weight gain, and depression. In another embodiment, the condition is pancreatitis, insulin-dependent diabetes mellitus, mastitis, cholecystitis, cholangitis, pericholangitis, chronic bronchitis, chronic sinusitis, asthma or graft versus host disease. In addition, these compounds may be useful in the prevention or reversal of these diseases when used in combination with currently available therapies, medical procedures, and therapeutic agents.

The compounds of the invention may be used in combination with other compositions and procedures for the treatment of disease. Additionally, the compounds of the present invention may be combined with pharmaceutically acceptable excipients, and optionally sustained-release matrices, such as biodegradable polymers, to form therapeutic compositions.

### Methods οf Treatment

It is disclosed herein a method for treating an individual afflicted with a condition or indication characterized by integrin binding, wherein the method comprises administering to the individual an integrin antagonist dimer molecule according to Formulas (I) or (II). In one embodiment, a method is provided for use in treating an individual afflicted with a condition or indication characterized by inappropriate trafficking of cells expressing α4β7 to tissues comprising cells expressing MAdCAM, comprising administering to the individual an α4β7-antagonist dimer molecule according to at least one of Formula (I) in an amount sufficient to inhibit (partially or fully) the trafficking of cells expressing α4β7 to tissues comprising cells expressing MAdCAM.

In some embodiments, the present invention provides a method whereby a pharmaceutical composition comprising an integrin antagonist dimer molecule according to Formula (I) is administered to a patient as a first treatment. In another embodiment, the method further comprises administering to the subject a second treatment. In another embodiment, the second treatment is administered to the subject before and/or simultaneously with and/or after the pharmaceutical composition is administered to the subject. In other embodiment, the second treatment comprises an anti-inflammatory agent. In another embodiment, the second pharmaceutical composition comprises an agent selected from the group consisting of non-steroidal anti-inflammatory drugs, steroids, and immune modulating agents. In another embodiment, the method comprises administering to the subject a third treatment.

In one embodiment, a method is provided for use in treating an individual afflicted with a condition or indication characterized by α4β7 integrin binding, wherein the method comprises administering to the individual an effective amount of an α4β7 integrin antagonist dimer molecule containing subunits selected from SEQ ID NOs: 47, 54, 55, 57, 58, 73, 74, 77, 78, 79, 80-82, 88, 90-97, 101, 104, 106, 107, 110, 120, 127, 133, 135, 137, 140. In some instances, an α4β7 integrin antagonist dimer molecule having subunits selected from and corresponding to SEQ ID NOs: 47, 54, 55, 57, 58, 73, 74, 77, 78, 79, 80-82, 88, 90-97, 101, 104, 106, 107, 110, 120, 127, 133, 135, 137, 140, and having high specificity for α4β7 is administered to an individual as part of a therapeutic treatment for a condition or indication characterized by α4β7 integrin binding. Some embodiments of the present invention further provide a method for use in treating an individual with an α4β7 integrin antagonist dimer molecule that is suspended in a sustained-release matrix. A sustained-release matrix, as used herein, is a matrix made of materials, usually polymers, which are degradable by enzymatic or acid-base hydrolysis or by dissolution. Once inserted into the body, the matrix is acted upon by enzymes and body fluids. A sustained-release matrix desirably is chosen from biocompatible materials such as liposomes, polylactides (polylactic acid), polyglycolide (polymer of glycolic acid), polylactide co-glycolide (copolymers of lactic acid and glycolic acid) polyanhydrides, poly(ortho)esters, polypeptides, hyaluronic acid, collagen, chondroitin sulfate, carboxylic acids, fatty acids, phospholipids, polysaccharides, nucleic acids, polyamino acids, amino acids such as phenylalanine, tyrosine, isoleucine, polynucleotides, polyvinyl propylene, polyvinylpyrrolidone and silicone. A preferred biodegradable matrix is a matrix of one of either polylactide, polyglycolide, or polylactide co-glycolide (co-polymers of lactic acid and glycolic acid).

In some aspects, the invention provides a pharmaceutical composition for oral delivery. The various embodiments and dimer compositions of the instant invention may be prepared for oral administration according to any of the methods, techniques, and/or delivery vehicles described herein. Further, one having skill in the art will appreciate that the dimer compositions of the instant invention may be modified or integrated into a system or delivery vehicle that is not disclosed herein, yet is well known in the art and compatible for use in oral delivery of small dimer peptide molecules.

Oral dosage forms or unit doses compatible for use with the dimer peptides of the present invention may include a mixture of dimer peptide active drug components, and nondrug components or excipients, as well as other non-reusable materials that may be considered either as an ingredient or packaging. Oral compositions may include at least one of a liquid, a solid, and a semi-solid dosage forms. In some embodiments, an oral dosage form is provided comprising an effective amount of dimer peptide having subunits selected from and corresponding to SEQ ID NOs: 47, 54, 55, 57, 58, 73, 74, 77, 78, 79, 80-82, 88, 90-97, 101, 104, 106, 107, 110, 120, 127, 133, 135, 137, 140, wherein the dosage form comprises at least one of a pill, a tablet, a capsule, a gel, a paste, a drink, a syrup, ointment, and suppository. In some instances, an oral dosage form is provided that is designed and configured to achieve delayed release of the peptide dimer in the subjects small intestine and/or colon

In one embodiment, an oral pharmaceutical composition according to Formula (I) comprises an enteric coating that is designed to delay release of the peptide dimer in the small intestine. In at least some embodiments, a pharmaceutical composition is provided which comprises a peptide dimer compound having subunits selected from and corresponding to SEQ ID NOs: 47, 54, 55, 57, 58, 73, 74, 77, 78, 79, 80-82, 88, 90-97, 101, 104, 106, 107, 110, 120, 127, 133, 135, 137, 140, and a protease inhibitor, such as aprotinin, in a delayed release pharmaceutical formulation. In some instances it is preferred that a pharmaceutical composition of the instant invention comprise an enteric coat that is soluble in gastric juice at a pH of about 5.0 or higher. In at least one embodiment, a pharmaceutical composition is provided comprising an enteric coating comprising a polymer having dissociable carboxylic groups, such as derivatives of cellulose, including hydroxypropylmethyl cellulose phthalate, cellulose acetate phthalate and cellulose acetate trimellitate and similar derivatives of cellulose and other carbohydrate polymers.

In one embodiment, a pharmaceutical composition having subunits selected from and corresponding to SEQ ID NOs: 47, 54, 55, 57, 58, 73, 74, 77, 78, 79, 80-82, 88, 90-97, 101, 104, 106, 107, 110, 120, 127, 133, 135, 137, 140is provided in an enteric coating, the enteric coating being designed to protect and release the pharmaceutical composition in a controlled manner within the subjects lower gastrointestinal system, and to avoid systemic side effects. In addition to enteric coatings, the dimer peptides of the instant invention may be encapsulated, coated, engaged or otherwise associated within any compatible oral drug delivery system or component. For example, in some embodiments a dimer peptide of the present invention is provided in a lipid carrier system comprising at least one of polymeric hydrogels, nanoparticles, microspheres, micelles, and other lipid systems.

To overcome peptide degradation in the small intestine, some implementations of the present invention comprise a hydrogel polymer carrier system in which a peptide dimer in accordance with the present invention is contained, whereby the hydrogel polymer protect the peptide dimer from proteolysis in the small intestine and/or colon. The peptide dimers of the present invention may further be formulated for compatible use with a carrier system that is designed to increase the dissolution kinetics and enhance intestinal absorption of the dimer peptides. These methods include the use of liposomes, micelles and nanoparticles to increase GI tract permeation of peptides.

Various bioresponsive systems may also be combined with one or more peptide dimers of the present invention to provide a pharmaceutical agent for oral delivery. in some embodiments, a peptide dimer of the instant invention is used in combination with a bioresponsive system, such as hydrogels and mucoadhesive polymers with hydrogen bonding groups (e.g., PEG, poly(methacrylic) acid [PMAA], cellulose, Eudragit®, chitosan and alginate) to provide a therapeutic agent for oral administration. Other embodiments include a method for optimizing or prolonging drug residence time for a peptide dimer disclosed herein, wherein the surface of the peptide dimer surface is modified to comprise mucoadhesive properties through hydrogen bonds, polymers with linked mucins or/and hydrophobic interactions. These modified dimer molecules may demonstrate increase drug residence time within the subject, in accordance with a desired feature of the invention. Moreover, targeted mucoadhesive systems may specifically bind to receptors at the enterocytes and M-cell surfaces, thereby further increasing the uptake of particles containing the dimer peptide.

Other embodiments comprise a dimer peptide having subunits selected from and corresponding to SEQ ID NOs: 47, 54, 55, 57, 58, 73, 74, 77, 78, 79, 80-82, 88, 90-97, 101, 104, 106, 107, 110, 120, 127, 133, 135, 137, 140, for use in oral delivery wherein the dimer peptide is used in combination with permeation enhancers that promote the transport of the dimer peptides across the intestinal mucosa by increasing paracellular or transcellular permeation. For example, in one embodiment a permeation enhancer is combined with a dimer peptide having subunits selected from and corresponding to SEQ ID NOs: 47, 54, 55, 57, 58, 73, 74, 77, 78, 79, 80-82, 88, 90-97, 101, 104, 106, 107, 110, 120, 127, 133, 135, 137, 140, wherein the permeation enhancer comprises at least one of a long-chain fatty acid, a bile salt, an amphiphilic surfactant, and a chelating agent. In one embodiment, a permeation enhancer comprising sodium N-[hydroxybenzoyl)amino] caprylate is used to form a weak noncovalent association with the dimer peptide of the instant invention, wherein the permeation enhancer favors membrane transport and further dissociation once reaching the blood circulation. In another embodiment, a peptide dimer of the present invention is conjugated to oligoarginine, thereby increasing cellular penetration of the dimer peptides into various cell types. Further, in at least one embodiment a noncovalent bond is provided between a dimer peptide having subunits selected from and corresponding to SEQ ID NOs: 47, 54, 55, 57, 58, 73, 74, 77, 78, 79, 80-82, 88, 90-97, 101, 104, 106, 107, 110, 120, 127, 133, 135, 137, 140and a permeation enhancer selected from the group consisting of a cyclodextrin (CD) and a dendrimers, wherein the permeation enhancer reduces peptide aggregation and increasing stability and solubility for the peptide dimer molecule.

Other embodiments of the invention provide α4β7 integrin antagonist dimer molecule having an increased half-life for use in treating an individual.. In one aspect, the present invention provides an integrin antagonist dimer molecule having a half-life of at least several hours to one day *in vitro* or *in vivo* (e.g., when administered to a human subject) sufficient for daily (q.d.) or twice daily (b.i.d.) dosing of a therapeutically effective amount. In another embodiment, the dimer molecule has a half-life of three days or longer sufficient for weekly (q.w.) dosing of a therapeutically effective amount. Further, in another embodiment the dimer molecule has a half-life of eight days or longer sufficient for biweekly (b.i.w.) or monthly dosing of a therapeutically effective amount. In another embodiment, the dimer molecule is derivatized or modified such that is has a longer half-life as compared to the underivatized or unmodified dimer molecule. In another embodiment, the dimer molecule contains one or more chemical modifications to increase serum half-life.

When used in at least one of the treatments or delivery systems described herein, a therapeutically effective amount of one of the compounds of the present invention may be employed in pure form or, where such forms exist, in pharmaceutically acceptable salt form. As used herein, a "therapeutically effective amount" of the compound of the invention is meant to describe a sufficient amount of the peptide dimer compound to treat an integrin-related disease, (for example, to reduce inflammation associated with IBD) at a desired benefit/risk ratio applicable to any medical treatment. It will be understood, however, that the total daily usage of the compounds and compositions of the present invention will be decided by the attending physician within the scope of sound medical judgment. The specific therapeutically effective dose level for any particular patient will depend upon a variety of factors including: a) the disorder being treated and the severity of the disorder; b) activity of the specific compound employed; c) the specific composition employed, the age, body weight, general health, sex and diet of the patient; d) the time of administration, route of administration, and rate of excretion of the specific compound employed; e) the duration of the treatment; f) drugs used in combination or coincidental with the specific compound employed, and like factors well known in the medical arts. For example, it is well within the skill of the art to start doses of the compound at levels lower than those required to achieve the desired therapeutic effect and to gradually increase the dosage until the desired effect is achieved.

Alternatively, a compound of the present invention may be administered as pharmaceutical compositions containing the compound of interest in combination with one or more pharmaceutically acceptable excipients. A pharmaceutically acceptable carrier or excipient refers to a non-toxic solid, semi-solid or liquid filler, diluent, encapsulating material or formulation auxiliary of any type. The compositions may be administered parenterally, intracisternally, intravaginally, intraperitoneally, intrarectally, topically (as by powders, ointments, drops, suppository, or transdermal patch), or buccally. The term "parenteral" as used herein refers to modes of administration which include intravenous, intramuscular, intraperitoneal, intrasternal, subcutaneous, intradermal and intraarticular injection and infusion.

Pharmaceutical compositions for parenteral injection comprise pharmaceutically acceptable sterile aqueous or nonaqueous solutions, dispersions, suspensions or emulsions, as well as sterile powders for reconstitution into sterile injectable solutions or dispersions just prior to use. Examples of suitable aqueous and nonaqueous carriers, diluents, solvents or vehicles include water, ethanol, polyols (such as glycerol, propylene glycol, polyethylene glycol, and the like), carboxymethylcellulose and suitable mixtures thereof, vegetable oils (such as olive oil), and injectable organic esters such as ethyl oleate. Proper fluidity may be maintained, for example, by the use of coating materials such as lecithin, by the maintenance of the required particle size in the case of dispersions, and by the use of surfactants.

These compositions may also contain adjuvants such as preservative, wetting agents, emulsifying agents, and dispersing agents. Prevention of the action of microorganisms may be ensured by the inclusion of various antibacterial and antifungal agents, for example, paraben, chlorobutanol, phenol sorbic acid, and the like. It may also be desirable to include isotonic agents such as sugars, sodium chloride, and the like. Prolonged absorption of the injectable pharmaceutical form may be brought about by the inclusion of agents which delay absorption, such as aluminum monostearate and gelatin.

Injectable depot forms are made by forming microencapsule matrices of the drug in biodegradable polymers such as polylactide-polyglycolide, poly(orthoesters), poly(anhydrides), and (poly)glycols, such as PEG. Depending upon the ratio of drug to polymer and the nature of the particular polymer employed, the rate of drug release can be controlled. Depot injectable formulations are also prepared by entrapping the drug in liposomes or microemulsions which are compatible with body tissues.

The injectable formulations may be sterilized, for example, by filtration through a bacterial-retaining filter, or by incorporating sterilizing agents in the form of sterile solid compositions which can be dissolved or dispersed in sterile water or other sterile injectable medium just prior to use.

Topical administration includes administration to the skin or mucosa, including surfaces of the lung and eye. Compositions for topical lung administration, including those for inhalation and intranasal, may involve solutions and suspensions in aqueous and non-aqueous formulations and can be prepared as a dry powder which may be pressurized or non-pressurized. In non-pressurized powder compositions, the active ingredient in finely divided form may be used in admixture with a larger-sized pharmaceutically acceptable inert carrier comprising particles having a size, for example, of up to 100 micrometers in diameter. Suitable inert carriers include sugars such as lactose.

Alternatively, the composition may be pressurized and contain a compressed gas, such as nitrogen or a liquefied gas propellant. The liquefied propellant medium and indeed the total composition is preferably such that the active ingredient does not dissolve therein to any substantial extent. The pressurized composition may also contain a surface active agent, such as a liquid or solid non-ionic surface active agent or may be a solid anionic surface active agent. It is preferred to use the solid anionic surface active agent in the form of a sodium salt.

A further form of topical administration is to the eye. A compound of the invention is delivered in a pharmaceutically acceptable ophthalmic vehicle, such that the compound is maintained in contact with the ocular surface for a sufficient time period to allow the compound to penetrate the corneal and internal regions of the eye, as for example the anterior chamber, posterior chamber, vitreous body, aqueous humor, vitreous humor, cornea, iris/ciliary, lens, choroid/retina and sclera. The pharmaceutically acceptable ophthalmic vehicle may, for example, be an ointment, vegetable oil or an encapsulating material. Alternatively, the compounds of the invention may be injected directly into the vitreous and aqueous humour.

Compositions for rectal or vaginal administration are preferably suppositories which may be prepared by mixing the compounds of this invention with suitable nonirritating excipients or carriers such as cocoa butter, polyethylene glycol or a suppository wax which are solid at room temperature but liquid at body temperature and therefore melt in the rectum or vaginal cavity and release the active compound.

Compounds of the present invention may also be administered in the form of liposomes. As is known in the art, liposomes are generally derived from phospholipids or other lipid substances. Liposomes are formed by mono- or multi-lamellar hydrated liquid crystals that are dispersed in an aqueous medium. Any non-toxic, physiologically acceptable and metabolizable lipid capable of forming liposomes can be used. The present compositions in liposome form can contain, in addition to a compound of the present invention, stabilizers, preservatives, excipients, and the like. The preferred lipids are the phospholipids, including the phosphatidyl cholines (lecithins) and serines, both natural and synthetic. Methods to form liposomes are known in the art.

Total daily dose of the compositions of the invention to be administered to a human or other mammal host in single or divided doses may be in amounts, for example, from 0.0001 to 300 mg/kg body weight daily and more usually 1 to 300 mg/kg body weight.

### Non-invasive Detection of Intestinal Inflammation

The peptides of the invention may be used for detection, assessment and diagnosis of intestinal inflammation by microPET imaging using an orally stable compound having subunits selected from and corresponding to SEQ ID NOs: 47, 54, 55, 57, 58, 73, 74, 77, 78, 79, 80-82, 88, 90-97, 101, 104, 106, 107, 110, 120, 127, 133, 135, 137, 140, and that is further labeled with at least one of a chelating group and a detectable label as part of a non-invasive diagnostic procedure. In one embodiment, an integrin antagonist dimer molecule is conjugated with a bifunctional chelator to provide an orally stable dimer molecule. In another embodiment, an integrin antagonist dimer molecule is radiolabeled to provide an orally stable dimer molecule. The orally stable, chelated or radiolabeled dimer molecule is then administered to a subject orally or rectally. In one embodiment, the orally stable dimer molecule is included in drinking water. Following uptake of the dimer molecules, microPET imaging may be used to visualize inflammation throughout the subject's bowels and digestive track.

### Synthesis of Peptide Subunits

The monomer peptide subunits of the present invention may be synthesized by many techniques that are known to those skilled in the art. Novel and unique monomer subunits were synthesized, purified, and dimerized using the techniques provided herein.

The peptides of the present invention were synthesized using the Merrifield solid phase synthesis techniques on Protein Technology's Symphony multiple channel synthesizer. The peptides were assembled using HBTU (O-Benzotriazole-N,N,N',N'-tetramethyl-uronium-hexafluoro-phosphate), Diisopropylethylamine(DIEA) coupling conditions. For some amino acid couplings PyAOP(7-Azabenzotriazol-1-yloxy)tripyrrolidinophosponium hexafluorophosphate) and DIEA conditions were used. Rink Amide MBHA resin (100-200mesh, 0.57mmol/g) is used for peptide with C-terminal amides and pre-loaded Wang Resin with N-a-Fmoc protected amino acid is used for peptide with C-terminal acids. The coupling reagents (HBTU and DIEA premixed) were prepared at 100mmol concentration. Similarly amino acids solutions were prepared at 100mmol concentration. The peptides were assembled using standard Symphony protocols.

### Assembly

The peptide sequences were assembled as follows: Resin (250mg, 0.14mmol) in each reaction vial was washed twice with 4ml of DMF followed by treatment with 2.5ml of 20% 4-methyl piperidine (Fmoc de-protection) for 10min. The resin was then filtered and washed two times with DMF (4ml) and re-treated with N-methyl piperifine for additional 30 minute. The resin was again washed three times with DMF (4ml) followed by addition 2.5ml of amino acid and 2.5ml of HBTU-DIEA mixture. After 45min of frequent agitations, the resin was filtered and washed three timed with DMF (4ml each). For a typical peptide of the present invention, double couplings were performed for first 25 amino acid, and triple couplings were performed for the remaining residues. After completing the coupling reaction, the resin was washed three times with DMF (4ml each) before proceeding to the next amino acid coupling.

### Cleavage

Following completion of the peptide assembly, the peptide was cleaved from the resin by treatment with cleavage reagent, such as reagent K (82.5% trigluoroacetic acid, 5% water, 5% thioanisole, 5% phenol, 2.5% 1,2-ethanedithiol). The cleavage reagent was able to successfully cleave the peptide from the resin, as well as all remaining side chain protecting groups.

The cleaved were precipitated in cold diethyl ether followed by two washings with ethyl ether. The filtrate was poured off and a second aliquot of cold ether was added, and the procedure repeated. The crude peptide was dissolved in a solution of acetonitrile:water (7:3 with 1% TFA) and filtered. The quality of linear peptide was then verified using electrospray ionization mass spectrometry (ESI-MS) (Micromass/Waters ZQ) before being purified.

### Disulfide Bond Formation via Oxidation

50mg of crude, cleaved peptide was dissolved in 20ml of water:acetonitrile. Saturated Iodine in acetic acid was then added drop wise with stirring until yellow color persisted. The solution was stirred for 15 minutes and the reaction was monitored with analytic HPLC and LCMS. When the reaction was completed, solid ascorbic acid was added until the solution became clear. The solvent mixture was then purified by first being diluted with water and then loaded onto a reverse phase HPLC machine (Luna C18 support, 10u, 100A, Mobile phase A: water containing 0.1% TFA, mobile phase B: Acetonitrile (ACN) containing 0.1% TFA, gradient began with 5% B, and changed to 50% B over 60 minutes at a flow rate of 15ml/min). Fractions containing pure product were then freeze-dried on a lyophilyzer.

### Lactam Bond Formation

100mg of crude, cleaved peptide (approx. 0.12mmol) was dissolved in 100ml of anhydrous dichloromethane. HOBt (1-Hydroxybenzotriazole hydrate) (0.24mmol, 2 equivalents) was added followed by DIEA (N,N-Diisopropylethylamine) (1.2mmol, 10equivalents) and TBTU (O-(Benzotriazol-1-yl)-N,N,N',N'-tetramethyluronium tetrafluoroborate)(0.24 mmol, 2 equivalents). The mixture was stirred overnight and followed the reaction by HPLC. When the reaction was completed, dichloromethane was evaporated and diluted with water and Acetonitrile and then loaded onto a reverse phase HPLC machine (Luna C18 support, 10u, 100A, Mobile phase A: water containing 0.1% TFA, mobile phase B: Acetonitrile (ACN) containing 0.1% TFA, gradient began with 5% B, and changed to 50% B over 60 minutes at a flow rate of 15ml/min). Fractions containing pure product were then freeze-dried on a lyophilyzer.

### Purification

Analytical reverse-phase, high performance liquid chromatography (HPLC) was performed on a Gemini C18 column (4.6 mm x 250 mm) (Phenomenex). Semi-Preparative reverse phase HPLC was performed on a Gemini 10 µm C18 column (22 mm x 250 mm) (Phenomenex) or Jupiter 10 µm, 300 A ° C18 column (21.2 mm x 250 mm) (Phenomenex). Separations were achieved using linear gradients of buffer B in A (Mobile phase A: water containing 0.15% TFA, mobile phase B: Acetonitrile (ACN) containing 0.1% TFA), at a flow rate of 1 mL/min (analytical) and 15 mL/min (preparative). Separations were achieved using linear gradients of buffer B in A (Mobile phase A: water containing 0.15% TFA, mobile phase B: Acetonitrile (ACN) containing 0.1% TFA), at a flow rate of 1 mL/min (analytical) and 15mL/min (preparative).

### Linker Activation and Dimerization

Small Scale DIG Linker Activation Procedure: 5mL of NMP was added to a glass vial containing IDA diacid (304.2 mg, 1 mmol), N-hydroxysuccinimide (NHS, 253.2 mg, 2.2 eq. 2.2mmol) and a stirring bar. The mixture was stirred at room temperature to completely dissolve the solid starting materials. N, N'-Dicyclohexylcarbodiimide (DCC, 453.9mg, 2.2 eq., 2.2 mmol) was then added to the mixture. Precipitation appeared within 10 min and the reaction mixture was further stirred at room temperature overnight. The reaction mixture was then filtered to remove the precipitated dicyclohexylurea (DCU). The activated linker was kept in a closed vial prior to use for dimerization. The nominal concentration of the activated linker was approximately 0.20 M.

For dimerization using PEG linkers, there is no pre-activation step involved. Commercially available pre-activated bi-functional PEG linkers were used.

Dimerization Procedure: 2mL of anhydrous DMF was added to a vial containing peptide monomer (0.1 mmol). The pH of the peptide was the adjusted to 8∼9 with DIEA. Activated linker (IDA or PEG13, PEG 25) (0.48eq relative to monomer, 0.048 mmol) was then added to the monomer solution. The reaction mixture was stirred at room temperature for one hour. Completion of the dimerization reaction was monitored using analytical HPLC. The time for completion of dimerization reaction varied depending upon the linker. After completion of reaction, the peptide was precipitated in cold ether and centrifuged. The supernatant ether layer was discarded. The precipitation step was repeated twice. The crude dimer was then purified using reverse phase HPLC (Luna C18 support, 10u, 100A, Mobile phase A: water containing 0.1% TFA, mobile phase B: Acetonitrile (ACN) containing 0.1% TFA, gradient of 15%B and change to 45%B over 60min, flow rate 15ml/min). Fractions containing pure product were then freeze-dried on a lyophilyzer.

### Simulated Intestinal Fluid (SIF) Stability Assay

Studies were carried out in simulated intestinal fluid (SIF) to evaluate gastric stability of the dimer molecules of the instant invention. SIF was prepared by adding 6.8 g of monobasic potassium phosphate and 10.0 g of pancreatin to 1.0 L of water. After dissolution, the pH was adjusted to 6.8 using NaOH. DMSO stocks (2 mM) were first prepared for the test compounds. Aliquots of the DMSO solutions were dosed into 6 individual tubes, each containing 0.5 mL of SIF, which had been pre-warmed to 37°C.

The final test compound concentration was 20 µM. The vials were kept in a benchtop Thermomixer® for the duration of the experiment. At each timepoint (0, 5, 10, 20, 40, and 60 minutes), 1.0 mL of acetonitrile containing 1% formic acid was added to one vial to terminate the reaction. Samples were stored at 4°C until the end of the experiment. After the final timepoint was sampled, the tubes were mixed and then centrifuged at 3,000 rpm for 10 minutes. Aliquots of the supernatant were removed, diluted 1:1 into distilled water containing internal standard, and analyzed by LCMS/MS. Percent remaining at each timepoint was calculated based on the peak area response ratio of test to compound to internal standard. Time 0 was set to 100%, and all later timepoints were calculated relative to time 0. Half-lives were calculated by fitting to a first-order exponential decay equation using Graphpad. A small sampling of the results of these studies is provided and discussed in connection Figure 3, above.

### EXAMPLES

### α4β7-MAdCAM Competition ELISA

A nickel coated plate (Pierce # 15442) was coated with recombinant human integrin α4β7 (R&D Systems #5397-A30) at 800ng/well and incubated at room temperature with shaking for 1hr. The solution was then remove by shaking and blocked with assay buffer (50mM Tris-HCl pH7.6, 150mM NaCl, 1mM MnCl₂, 0.05% Tween-20 and 0.5% BSA) at 250ul/well. The plate was then incubated at room temperature for 1hr. Each well was washed 3 times with wash buffer (50mM Tris-HCl pH7.6, 100mM NaCl, 1mM MnCl₂, 0.05% Tween-20). To each well was added 25ul of a serial dilution (3-fold dilutions in assay buffer) of peptides starting at 20µM or lower concentration. 25 ul of recombinant human MAdCAM-Fc chimera (R&D Systems #6056-MC) was then added to each well at a fixed concentration 20nM. The final starting peptide concentration was 10µM, and the final MAdCAM-1 concentration was 10nM. The plates were then incubated at room temperature for 1hr to reach binding equilibrium. The wells were then washed three times with wash buffer. 50ul of mouse anti-human IgG1-HRP (Invitrogen # A10648) diluted in 1:2000 in assay buffer was then added to each well. The wells were incubated at room temperature for 45 min with shaking. The wells were then washed 3 times with wash buffer. 100ul of TMB were then added to each well and closely observe during development time. The reaction was stopped with 2N H₂SO₄ and absorbance was read at 450nm.

### α4β1-VCAM Competition ELISA

A Nunc MaxiSorp plate was coated with rh VCAM-1/CD106 Fc chimera (R&D #862-VC) at 400ng/well in 50ul per well in 1XPBS and incubated overnight at 4° C. The solution was removed by shaking and then blocked with 250ul of 1% BSA in 1XPBS per well. The wells were then incubated at room temperature for 1hr with shaking. Each well was then washed once with wash buffer (50mM Tris-HCl pH7.6, 100mM NaCL, 1mM MnCl₂, 0.05% Tween-20). 25ul of serial dilutions of peptides starting at 200µM or lower concentration in assay buffer (Assay buffer: 50mM Tris-HCl pH7.6, 100mM NaCl, 1mM MnCl₂, 0.05% Tween-20) was added to each well. Additionally, 25 ul of α4β1 (R&D Systems #5668-A4) was added to each well at a fixed concentration of 20nM. The final peptide and α4β1 concentrations were 100µM and 10nM, respectively. The plates were then incubated at 37°C for 2hr. The solution was then removed by shaking and each well was washed three times with wash buffer. 50ul of 9F10 antibody at 4ug/ml (purified mouse anti-human CD49d, BD Bioscience Cat# 555502) was then added to each well, and the plate was incubated at room temperature for 1hr with shaking. The solution was again removed by shaking, and each well was washed three times with wash buffer. 50ul of peroxidase-conjugated AffiniPure Goat anti-mouse IgG (Jackson immune research cat #115-035-003) diluted in 1:5000 in assay buffer was added to each well. The plate was incubated at room temperature for 30 min with shaking. Each well was then washed 3 times with wash buffer. 100ul of TMB was then added to each well and closely observe during developing time. The reaction was stepped with 2N H₂SO₄ and absorbance was read at 450nm.

### Example 3: α4β7-MAdCAM Cell Adhesion Assay

RPMI 8866 cells (Sigma #95041316) are cultured in RPMI 1640 HEPES medium (Invitrogen #22400-089) supplemented with 10% serum (Fetal Bovine Serum, Invitrogen # 16140-071), 1 mM sodium pyruvate (Invitrogen #11360-070), 2mM L-glutamine (Invitrogen # 25030-081) and Penicillin-Streptomycin (Invitrogen # 15140-122) at 100 units of penicillin and 100 µg of streptomycin per ml. The cells are washed two times in DMEM medium (ATCC #30-2002) supplemented with 0.1% BSA, 10 mM HEPES pH 7 and 1 mM MnCl₂. The cells are re-suspended in supplemented DMEM medium at a density of 4 X 10⁶ cells/ml.

A Nunc MaxiSorp plate was coated with rh MAdCAM-1/ Fc Chimera (R&D 6056-MC) at 200 ng per well in 50 ul per well in 1XPBS and incubated at 4°C overnight. The solution was then removed by shaking, blocked with 250 ul per well PBS containing 1% BSA, and incubated at 37°C for 1 hr. The solution was removed by shaking. Peptides are diluted by serial dilution in a final volume of 50 ul per well (2X concentration). To each well, 50 ul of cells (200,000 cells) are added and the plate is incubated at 37°C, 5% CO₂ for 30-45 min to allow cell adhesion. The wells are washed manually three times (100 ul per wash) with supplemented DMEM. After the final wash, 100ul/well of supplemented DMEM and 10ul/well of MTT reagent (ATTC cat# 30-1010K) are added. The plate is incubated at 37°C, 5% CO2 for 2-3hrs until a purple precipitate is visible. 100ul of Detergent Reagent (ATTC cat# 30-1010K) is added to each well. The plate is covered from the light, wrapped in Parafilm to prevent evaporation, and left overnight at room temperature in the dark. The plate is shaken for 5 min and the absorbance at 570 nm is measured. To calculate the dose response, the absorbance value of control wells not containing cells is subtracted from each test well.

### Example 4: α4β1-VCAM Cell Adhesion Assay

Jurkat E6.1 cells (Sigma #88042803) are cultured in RPMI 1640 HEPES medium (Invitrogen #22400-089) supplemented with 10% serum (Fetal Bovine Serum, Invitrogen # 16140-071), 1 mM sodium pyruvate (Invitrogen #11360-070), 2mM L-glutamine (Invitrogen # 25030-081) and Penicillin-Streptomycin (Invitrogen # 15140-122) at 100 units of penicillin and 100 µg of streptomycin per ml. The cells are washed two times in DMEM medium (ATCC #30-2002) supplemented with 0.1% BSA, 10 mM HEPES pH 7 and 1 mM MnCl₂. The cells are re-suspended in supplemented DMEM medium at a density of 4 X 10⁶ cells/ml.

A Nunc MaxiSorp plate was coated with rh VCAM-1/CD106 Fc chimera (R&D #862-VC) at 400 ng per well in 50 ul per well in 1XPBS and incubated at 4°C overnight. The solution was then removed by shaking, blocked with 250 ul per well PBS containing 1% BSA, and incubated at 37°C for 1 hr. The solution was removed by shaking. peptides are diluted by serial dilution in a final volume of 50 ul per well (2X concentration). To each well, 50 ul of cells (200,000 cells) are added and the plate is incubated at 37°C, 5% CO₂ for 30-45 min to allow cell adhesion. The wells are washed manually three times (100 ul per wash) with supplemented DMEM. After the final wash, 100ul/well of supplemented DMEM and 10ul/well of MTT reagent (ATTC cat# 30-1010K) are added. The plate is incubated at 37°C, 5% CO2 for 2-3hrs until a purple precipitate is visible. 100ul of Detergent Reagent (ATTC cat# 30-1010K) is added to each well. The plate is covered from the light, wrapped in Parafilm to prevent evaporation, and left overnight at room temperature in the dark. The plate is shaken for 5 min and the absorbance at 570 nm is measured. To calculate the dose response, the absorbance value of control wells not containing cells is subtracted from each test well.

The present invention may be embodied in other specific forms without departing from its structures, methods, or other essential characteristics as broadly described herein and claimed hereinafter. The described embodiments are to be considered in all respects only as illustrative, and not restrictive. The scope of the invention is, therefore, indicated by the appended claims, rather than by the foregoing description. All changes that come within the meaning and range of equivalency of the claims are to be embraced within their scope.

## Claims

1. A peptide dimer compound comprising two peptide monomer subunits of Formula (I) Xaa1-Xaa2-Xaa3-Xaa4-Xaa5-Xaa6-Xaa7-Xaa8-Xaa9-Xaa10-Xaa11-Xaa12-Xaa13-Xaa14 (Formula I), or a pharmaceutically acceptable salt thereof, wherein
Xaa1 is absent or selected from the group consisting of: Gln, Asp, Pro, Gly, His, Ala, Ile, Phe, Lys, Arg, Asn, Glu, Leu, Val, Tyr, Trp, Ser, Met, Thr, a suitable isostere, and a corresponding D-amino acid;
Xaa2 is absent or selected from the group consisting of: Gln, Asp, Pro, Gly, His, Ala, Ile, Phe, Lys, Arg, Asn, Glu, Leu, Val, Tyr, Trp, Ser, Met, Thr, a suitable isostere, and a corresponding D-amino acid;
Xaa3 is absent or selected from the group consisting of: Gln, Asp, Pro, Gly, His, Ala, Ile, Phe, Lys, Arg, Asn, Glu, Leu, Val, Tyr, Trp, Met, Thr, Ser, a suitable isostere, and a corresponding D-amino acid;
Xaa4 is Cys or Pen;
Xaa5 is N-Me-Arg;
Xaa6 is Ser;
Xaa7 is Asp;
Xaa8 is Thr;
Xaa9 is Leu;
Xaa10 is Cys or Pen;
Xaa11 is absent or selected from the group consisting of: Gly, Gln, Asn, Asp, Ala, Ile, Leu, Val, Met, Thr, Lys, Trp, Tyr, His, Glu, Ser, Arg, Pro, Phe, Sar, 1-Nal, 2-Nal, HPhe, Phe(4-F), dihydro-Trp, Dap, Dab, Orn, D-Orn, D-Dap, D-Dab, Bip, Ala(3,3 diphenyl), Biphenyl-Ala, D-Phe, D-Trp, D-Tyr, D-Glu, D-His, D-Lys, 3,3-diPhe, β-HTrp, F(4-CF3), O-Me-Tyr, 4-Me-Phe, an aromatic ring substituted Phe, an aromatic ring substituted Trp, an aromatic ring substituted His, a hetero aromatic amino acid, N-Me-Lys, N-Me-Lys(Ac), 4-Me-Phe, a corresponding D-amino acid; a suitable isostere; and a suitable linker moiety.
Xaa12 is selected from the group consisting of: Glu, β-HGlu, 2-Nal, 1-Nal, D-Asp, Bip, β-HPhe, βGlu, a suitable isostere, a suitable linker moiety, and a corresponding D-amino acid;
Xaa13 is absent or selected from the group consisting of: Gln, Pro, Gly, His, Ala, Ile, Phe, Lys, Arg, Leu, Val, Tyr, Trp, Met, Glu, Gla, Ser, Asn, Dap, Dab, Orn, D-Orn, N-Me-Orn, N-Me-Dap, N-Me-Dab, N-Me-Lys, D-Dap, D-Dab, D-Lys, N-Me-D-Lys, a suitable isostere, and a corresponding D-amino acid; and
Xaa14 is absent or selected from the group consisting of: a natural amino acid, a suitable isostere, and a corresponding D-amino acid,
wherein the peptide dimer compound comprises a disulfide bond between Xaa4 and Xaa10, and wherein the two peptide monomer subunits are joined by a suitable linker moiety, optionally wherein the N-terminus of each monomer subunit is joined by the suitable linker moiety to provide an N-terminus dimer compound, or optionally wherein the C-terminus of each monomer subunit is joined by the suitable linker moiety to provide a C-terminus dimer compound.

2. The peptide dimer compound or pharmaceutically acceptable salt thereof of claim 1, wherein the suitable linker moiety is selected from the group consisting of diglycolic acid (DIG), DIG-OH, PEG13, PEG25, PEG1K, PEG2K, PEG3.4K, PEG4K, PEG5K, β-Ala-Iminodiacetic acid (IDA), IDA-Palm, IDA-Boc, IDA-Isovaleric acid, Triazine, Triazine-Boc, Isophthalic acid, 1,3-phenylenediacetic acid, 1,4-phenylenediacetic acid, cyclopropylacetic acid, 4-fluoorobenzoic acid, 4-fluorophenylacetic acid, 3-phenylpropionic acid, succinic acid, biotin, glutaric acid, Azelaic acid, Pimelic acid, Dodecanedioic acid, suitable aliphatics, suitable aromatics, heteroaromatics, and polyethylene glycols having a molecular weight from approximately 400Da to approximately 40,000Da.

3. The peptide dimer compound or pharmaceutically acceptable salt thereof of claim 1 or claim 2, comprising:
acylation at one or more position selected from the group consisting of Xaa1-Xaa4 and Xaa11-Xaa14, optionally wherein the acylation is at the N-terminus of the peptide dimer compound.

4. The peptide dimer compound or pharmaceutically acceptable salt thereof of claim 1 or claim 2, comprising:
a C-terminal amide or OH.

5. The peptide dimer compound or pharmaceutically acceptable salt thereof of any of claims 1-4, wherein Xaa1-Xaa3 are absent.

6. The peptide dimer compound or pharmaceutically acceptable salt thereof of any one of claims 1-5, wherein Xaa¹⁰ is Pen.

7. The peptide dimer compound or pharmaceutically acceptable salt thereof of claim 6, wherein both Xaa⁴ and Xaa¹⁰ are Pen.

8. A peptide dimer compound or pharmaceutically acceptable salt thereof, wherein the peptide dimer compound comprises a peptide monomer subunit having an amino acid sequence set forth in any of SEQ ID NOs: 106, 107, 133, 120 and 137.

9. A pharmaceutical composition comprising the peptide dimer compound or pharmaceutically acceptable salt thereof of any one of claims 1-8 and a pharmaceutically acceptable excipient.

10. The pharmaceutical composition of claim 9, wherein the pharmaceutical composition comprises an enteric coating.

11. The peptide dimer compound of any one of claims 1-8 or the pharmaceutical composition of claim 9 or claim 10 for use in treating a subject afflicted with a condition associated with a biological function of α4β7, wherein the condition is selected from the group consisting of Inflammatory Bowel Disease (IBD), ulcerative colitis, Crohn's disease, Celiac disease (nontropical Sprue), enteropathy associated with seronegative arthropathies, microscopic colitis, collagenous colitis, eosinophilic gastroenteritis, radiotherapy, chemotherapy, pouchitis resulting after proctocolectomy and ileoanal anastomosis, gastrointestinal cancer, pancreatitis, insulin-dependent diabetes mellitus, mastitis, cholecystitis, cholangitis, pericholangitis, chronic bronchitis, chronic sinusitis, asthma, and graft versus host disease.

12. The peptide dimer compound or the pharmaceutical composition for use of claim 11, wherein the condition is ulcerative colitis or Crohn's disease.

13. The peptide dimer compound or the pharmaceutical composition for use of claim 11 or claim 12, wherein:
(ii) the peptide dimer compound or the pharmaceutical composition is administered by a form of administration selected from oral, intravenous, peritoneal, intradermal, subcutaneous, intramuscular, intrathecal, inhalation, vaporization, nebulization, sublingual, buccal, parenteral, rectal, vaginal, and topical.

14. The peptide dimer compound or the pharmaceutical composition for use of claim 13, wherein the peptide dimer compound or the pharmaceutical composition is administered orally, topically, intravenously, or subcutaneously.

15. The peptide dimer compound or the pharmaceutical composition for use of claims 11-14, wherein the subject is a human.

## Patentansprüche

1. Peptiddimer-Verbindung, umfassend zwei Peptidmonomer-Untereinheiten der Formel (I) Xaa1-Xaa2-Xaa3-Xaa4-Xaa5-Xaa6-Xaa7-Xaa8-Xaa9-Xaa10-Xaa11-Xaa12-Xaa13-Xaa14 (Formel I) oder ein pharmazeutisch verträgliches Salz davon, wobei
Xaa1 fehlt oder ausgewählt ist aus der Gruppe bestehend aus: Gln, Asp, Pro, Gly, His, Ala, Ile, Phe, Lys, Arg, Asn, Glu, Leu, Val, Tyr, Trp, Ser, Met, Thr, einem geeigneten Isoster und einer entsprechenden D-Aminosäure;
Xaa2 fehlt oder ausgewählt ist aus der Gruppe bestehend aus: Gln, Asp, Pro, Gly, His, Ala, Ile, Phe, Lys, Arg, Asn, Glu, Leu, Val, Tyr, Trp, Ser, Met, Thr, einem geeigneten Isoster und einer entsprechenden D-Aminosäure;
Xaa3 fehlt oder ausgewählt ist aus der Gruppe bestehend aus: Gln, Asp, Pro, Gly, His, Ala, Ile, Phe, Lys, Arg, Asn, Glu, Leu, Val, Tyr, Trp, Met, Thr, Ser, einem geeigneten Isoster und einer entsprechenden D-Aminosäure;
Xaa4 ist Cys oder Pen;
Xaa5 ist N-Me-Arg;
Xaa6 ist Ser;
Xaa7 ist Asp;
Xaa8 ist Thr;
Xaa9 ist Leu;
Xaa10 ist Cys oder Pen;
Xaa11 fehlt oder ausgewählt ist aus der Gruppe bestehend aus: Gly, Gln, Asn, Asp, Ala, Ile, Leu, Val, Met, Thr, Lys, Trp, Tyr, His, Glu, Ser, Arg, Pro, Phe, Sar, 1-Nal, 2-Nal, HPhe, Phe(4-F), dihydro-Trp, Dap, Dab, Orn, D-Orn, D-Dap, D-Dab, Bip, Ala(3,3 diphenyl), Biphenyl-Ala, D-Phe, D-Trp, D-Tyr, D-Glu, D-His, D-Lys, 3,3-diPhe, β-HTrp, F(4-CF3), O-Me-Tyr, 4-Me-Phe, einem am aromatischen Ring substituierten Phe, einem am aromatischen Ring substituierten Trp, einem am aromatischen Ring substituierten His, einer heteroaromatischen Aminosäure, N-Me-Lys, N-Me-Lys (Ac), 4-Me-Phe, einer entsprechenden D-Aminosäure; einem geeigneten Isoster; und einer geeigneten Linkereinheit;
Xaa12 ausgewählt ist aus der Gruppe bestehend aus: Glu, β-HGlu, 2-Nal, 1-Nal, D-Asp, Bip, β-HPhe, β-Glu, einem geeigneten Isoster, einer geeigneten Linkereinheit und einer entsprechenden D-Aminosäure;
Xaa13 fehlt oder ist ausgewählt aus der Gruppe bestehend aus: Gln, Pro, Gly, His, Ala, Ile, Phe, Lys, Arg, Leu, Val, Tyr, Trp, Met, Glu, Gla, Ser, Asn, Dap, Dab, Orn, D-Orn, N-Me-Orn, N-Me-Dap, N-Me-Dab, N-MeLys, D-Dap, D-Dab, D-Lys, N-Me-D-Lys, einem geeigneten Isoster und einer entsprechenden D-Aminosäure; und
Xaa14 fehlt oder ist ausgewählt aus der Gruppe bestehend aus: einer natürlichen Aminosäure, einem geeigneten Isoster, und einer entsprechenden D-Aminosäure,
wobei die Peptiddimer-Verbindung eine Disulfidbindung zwischen Xaa4 und Xaa10 umfasst, und wobei die zwei Peptidmonomer-Untereinheiten durch eine geeignete Linkereinheit verbunden sind, optional wobei der N-Terminus jeder Monomer-Untereinheit durch die geeignete Linkereinheit verbunden ist, um eine N-Terminus-Dimer-Verbindung bereitzustellen, oder optional wobei der C-Terminus jeder Monomer-Untereinheit durch die geeignete Linkereinheit verbunden ist, um eine C-Terminus-Dimer-Verbindung bereitzustellen.

2. Peptiddimer-Verbindung oder pharmazeutisch verträgliches Salz davon nach Anspruch 1, wobei die geeignete Linkereinheit ausgewählt ist aus der Gruppe bestehend aus Diglykolsäure (DIG), DIG-OH, PEG13, PEG25, PEG1K, PEG2K, PEG3.4K, PEG4K, PEG5K, β-Ala-Iminodiessigsäure (IDA), IDA-Palm, IDA-Boc, IDA-Isovaleriansäure, Triazin, Triazin-Boc, Isophthalsäure, 1,3-Phenylendiessigsäure, 1,4-Phenylendiessigsäure, Cyclopropylessigsäure, 4-Fluorbenzoesäure, 4-Fluorphenylessigsäure, 3-Phenylpropionsäure, Bernsteinsäure, Biotin, Glutarsäure, Azelainsäure, Pimelinsäure, Dodecandisäure, geeigneten Aliphaten, geeigneten Aromaten, Heteroaromaten und Polyethylenglykolen mit einem Molekulargewicht von ungefähr 400Da bis ungefähr 40.000Da.

3. Peptiddimer-Verbindung oder pharmazeutisch verträgliches Salz davon nach Anspruch 1 oder Anspruch 2, umfassend:
Acylierung an einer oder mehreren Positionen ausgewählt aus der Gruppe bestehend aus Xaa1-Xaa4 und Xaa11-Xaa14, optional wobei sich die Acylierung am N-Terminus der Peptiddimer-Verbindung befindet.

4. Peptiddimer-Verbindung oder pharmazeutisch verträgliches Salz davon nach Anspruch 1 oder Anspruch 2, umfassend:
ein C-terminales Amid oder OH.

5. Peptiddimer-Verbindung oder pharmazeutisch verträgliches Salz davon nach einem der Ansprüche 1 bis 4, wobei Xaa1-Xaa3 fehlt.

6. Peptiddimer-Verbindung oder pharmazeutisch verträgliches Salz davon nach einem der Ansprüche 1-5, worin Xaa¹⁰ Pen ist.

7. Peptiddimer-Verbindung oder pharmazeutisch verträgliches Salz davon nach Anspruch 6, wobei sowohl Xaa⁴ als auch Xaa¹⁰ Pen sind.

8. Peptiddimer-Verbindung oder pharmazeutisch verträgliches Salz davon, wobei die Peptiddimer-Verbindung eine Peptidmonomer-Untereinheit umfasst, die eine Aminosäuresequenz gemäß einer der SEQ ID NOs: 106, 107, 133, 120 und 137 aufweist.

9. Pharmazeutische Zusammensetzung, umfassend die Peptiddimer-Verbindung oder das pharmazeutisch akzeptables Salz davon nach einem der Ansprüche 1 bis 8 und einen pharmazeutisch akzeptablen Träger.

10. Pharmazeutische Zusammensetzung nach Anspruch 9, wobei die pharmazeutische Zusammensetzung eine magensaftresistente Beschichtung umfasst.

11. Peptiddimer-Verbindung nach einem der Ansprüche 1-8 oder pharmazeutische Zusammensetzung nach Anspruch 9 oder Anspruch 10 zur Verwendung bei der Behandlung eines Patienten, der an einem Zustand leidet, der mit einer biologischen Funktion von a4ß7 assoziiert ist, wobei der Zustand ausgewählt ist aus der Gruppe bestehend aus entzündlicher Darmkrankheit (IBD), Colitis Ulcerosa, Morbus Crohn, Zöliakie (nicht-tropische Sprue), Enteropathie assoziiert mit seronegativen Arthropathien, mikroskopischer Kolitis, kollagener Kolitis, eosinophiler Gastroenteritis, Strahlentherapie, Chemotherapie, Pouchitis nach Proktokolektomie und ileoanaler Anastomose, Magen-Darm-Krebs, Pankreatitis, insulinabhängigem Diabetes mellitus, Mastitis, Cholezystitis, Cholangitis, Pericholangitis, chronischer Bronchitis, chronischer Sinusitis, Asthma und Graft-versus-Host-Krankheit.

12. Peptiddimer-Verbindung oder pharmazeutische Zusammensetzung zur Verwendung nach Anspruch 11, wobei der Zustand Colitis Ulcerosa oder Morbus Crohn ist.

13. Peptiddimer-Verbindung oder pharmazeutische Zusammensetzung zur Verwendung nach Anspruch 11 oder Anspruch 12, wobei:
(ii) die Peptiddimer-Verbindung oder die pharmazeutische Zusammensetzung verabreicht wird durch eine Form von Verabreichung ausgewählt aus oral, intravenös, peritoneal, intradermal, subkutan, intramuskulär, intrathekal, Inhalation, Verdampfung, Vernebelung, sublingual, bukkal, parenteral, rektal, vaginal und topisch.

14. Peptiddimer-Verbindung oder pharmazeutische Zusammensetzung zur Verwendung nach Anspruch 13 wobei die Peptiddimer-Verbindung oder die pharmazeutische Zusammensetzung oral, topisch, intravenös oder subkutan verabreicht wird.

15. Peptiddimer-Verbindung oder die pharmazeutische Zusammensetzung zur Verwendung nach den Ansprüchen 11 bis 14, wobei der Patient ein Mensch ist.

## Revendications

1. Composé peptidique dimère comprenant deux sous-unités de monomère peptidique de formule (I) Xaa1-Xaaa2-Xaaa3-Xaaa4-Xaaa5-Xaaa6-Xaaa7-Xaaa8-Xaa9-Xaa10-Xaa11-Xaa 12-Xaa13-Xaa14 (formule I), ou un sel pharmaceutique-ment acceptable de celui-ci, où
Xaa1 est absent ou choisi dans le groupe constitué de: XaaI : Gin, Asp, Pro, Pro, Gly, His, Ala, lie, Phe, Lys, Arg, Asn, Glu, Leu, Val, Tyr, Trp, Ser, Met, Thr, un isostère approprié, et un acide D-aminé correspondant ; Xaa2 est absent ou choisi dans le groupe constitué de: Gin, Asp, Pro, Pro, Gly, His, Ala, lie, Phe, Lys, Arg, Asn, Glu, Leu, Val, Tyr, Trp, Ser, Met, Thr, un isostère approprié, et un Acide D-aminé correspondant ; Xaa3 est absent ou choisi dans le groupe constitué de: Gin, Asp, Pro, Gly, His, Ala, lie, Phe, Lys, Arg, Asn, Glu, Leu, Val, Tyr, Trp, Met, Thr, Ser, un isostère approprié, et un acide D-aminé correspondant; Xaa4 est Cys ou Pen; Xaa5 est N-Me-Arg; Xaa6 est Ser; Xaa7 est Asp; Xaa8 estThr; Xaaa 9 est Leu; Xaa10 est Cys ou Pen;
Xaa11 est absent ou choisi dans le groupe constitué de: Gly, Gin, Asn, Asp, Ala, lie, Leu, Val, Met, Thr, Lys, Trp, Tyr, His, Glu, Ser, Arg, Pro, Phe, Sar, 1-Nal, 2-Nal, HPhe, Phe(4-F), dihydro-Trp, Dap, D-Tab, Orn, D-Orn, D-Dap, D-Dap, D-Dab, Bip, Ala(3,3 diphényle), Biphényle-Ala, D-Phe, D-Trp, D-Tyr, D-Glu, D-His, D-Lys, 3,3-diPhe, (3-HTrp, F(4-CF3), O-Me-Tyr, 4-Me-Phe, Phe substitué par un cycle aromatique, Trp substitué par un cycle aromatique, His substitué par un cycle aromatique, N-Me-Lys, N-Me-Lys(Ac), 4-Me-Phe, un Acide D-aminé correspondant; un isostère approprié; et un fragment de liaison approprié.
Xaa12 est sélectionné parmi le groupe constitué de: Glu, (3-HGlu, 2-Nal, 1-Nal, D-Asp, Bip, p-HPhe, |3Glu, un isostère approprié, un fragment de liaison approprié, et un acide D-aminé correspondant;
Xaa13 est absent ou choisi dans le groupe constitué de: Gin, Pro, Gly, His, Ala, lie, Phe, Lys, Arg, Leu, Val, Tyr, Trp, Met, Glu, Gla, Ser, Ser, Asn, Dap, Dab, Orn, D-Orn, N-Me-Orn, N-Me-Dap, N-Me-Dap, N-Me-Dab, N-Me-Lys, D-Dap, D-Lys, N-Me-D-Lys, un isostère approprié et un acide D-aminé correspondant; et Xaa14 est absent ou choisi dans le groupe constitué de: un acide aminé naturel, un isostère approprié et un acide D-aminé correspondant,
dans lequel le composé dimère peptidique comprend une liaison disulfure entre Xaaa4 et Xaa10, et dans laquelle les deux sous-unités monomères peptidiques sont reliées par un fragment de liaison approprié, éventuellement dans lequel l'extrémité N-terminale de chaque sous-unité monomère est reliée par le fragment de liaison approprié pour fournir un composé dimère N-terminal, ou éventuellement dans lequel l'extrémité C-terminal de chaque sous-unité monomère est reliée par le fragment de liaison approprié pour fournir un composé dimère C-terminal.

2. Composé dimère peptidique ou sel pharmaceutiquement acceptable de celui-ci selon la revendication 1, dans lequel le fragment de liaison approprié est choisi dans le groupe constitué par l'acide diglycolique (DIG), DIG-OH, PEG13, PEG25, PEG1K, PEG2K et PEG3.4K, PEG4K, PEG5K, acide P-Ala-Iminodiacétique (IDA), IDA-Palm, IDA-Boc, acide IDA-Isovalérique, triazine, triazine-Boc, acide isophtalique, acide 1,3-phénylènediacétique, acide 1,4-phénylènedicétique, acide cyclopropylacétique, acide 4-fluoorobenzoïque et acide 4-fluorophénylacétique, acide 3-phénylpropionique, acide succinique, biotine, acide glutarique, acide azélaïque, acide pimélique, acide dodécanedioïque, acides aliphatiques appropriés, aromatiques appropriés, hétéroaromatiques et polyéthylèneglycols ayant un poids moléculaire d'environ 400Da à environ 40,000Da.

3. Composé peptidique dimère ou sel pharmaceutiquement acceptable de celui-ci selon les revendications 1 ou 2, comprenant:
acylation en une ou plusieurs positions choisies dans le groupe constitué de Xaa1-Xaaa4 et Xaa11-Xaa14, facultativement dans lequel l'acylation est à l'extrémité N-terminale du composé dimère peptide.

4. Composé peptidique dimère ou sel pharmaceutiquement acceptable de celui-ci selon la revendication 1 ou la revendication 2, comprenant:
un amide C-terminal ou OH.

5. Composé peptidique dimère ou sel pharmaceutiquement acceptable de celui-ci selon l'une quelconque des revendications 1-4, dans lequel Xaa1-Xaa3 sont absents.

6. Composé peptidique dimère ou sel pharmaceutiquement acceptable de celui-ci selon l'une quelconque des revendications 1-5, dans lequel Xaa¹⁰ est Pen.

7. Composé peptidique dimère ou sel pharmaceutiquement acceptable de celui-ci selon la revendication 6, dans lequel Xaa⁴ et Xaa¹⁰ sont tous deux Pen.

8. Composé dimère peptidique ou sel pharmaceutiquement acceptable de celui-ci, dans lequel le composé dimère peptidique comprend une sous-unité monomère peptidique ayant une séquence d'acides aminés présente dans l'une quelconque des SEQ ID NO: 106, 107, 133, 120 et 137.

9. Composition pharmaceutique comprenant le composé dimère peptidique ou un sel pharmaceutiquement acceptable de celui-ci de l'une quelconque des revendications 1-8 et un excipient pharmaceutiquement acceptable.

10. Composition pharmaceutique selon la revendication 9, dans laquelle la composition pharmaceutique comprend un enrobage entérique.

11. Composé peptidique dimère de l'une quelconque des revendications 1-8 ou la composition pharmaceutique de la revendication 9 ou de la revendication 10 pour une utilisation dans le traitement d'un sujet atteint d'une condition associée à une fonction biologique de ct4B7, dans laquelle l'affection est choisie dans le groupe constitué de la maladie intestinale inflammatoire (IBD), une colite ulcéreuse, la maladie de Crohn, la maladie coeliaque (propagation non tropicale), une entéropathie associée à des arthropathies séronégatives, une colite microscopique, une colite collagène, une gastro-entérite éosinophilique, une radiothérapie, une chimiothérapie, une pochite résultant d'une proctocolectomie et d'une anastomose iléoanale, un cancer gastro-intestinal, une pancréatite, un diabète sucré insulino-dépendant, une mastite, une cholécystite, une cholangite, d'une péricholangite, une bronchite chronique, une sinusite chronique, un asthme et une maladie du greffon contre l'hôte.

12. Composé peptidique dimère ou composition pharmaceutique pour l'utilisation de la revendication 11,
où l'affection est une colite ulcéreuse ou une maladie de Crohn.

13. Composé peptidique dimère ou composition pharmaceutique pour utilisation selon la revendication 11 ou 12, dans lequel:
(ii) le composé dimère peptidique ou bien la composition pharmaceutique sont administrés par voie orale, intraveineuse, péritonéale, intradermique, sous-cutanée, intramusculaire, intrathécale, inhalation, vaporisation, nébulisation, sublinguale, buccale, parentérale, rectale, vaginale et topique.

14. Composé peptidique dimère ou composition pharmaceutique pour l'utilisation selon la revendication 13,
dans lequel le composé peptidique dimère ou la composition pharmaceutique sont administrés par voie orale, topique, intraveineuse ou sous-cutanée.

15. Composé peptidique dimère ou composition pharmaceutique pour utilisation selon les revendications 11-14,
dans lequel le sujet est un humain.


### SEQUENCE LISTING

<110> Bhandari, Ashok
   Mattheakis, Larry C.
   Patel, Dinesh V.
<120> NOVEL a4b7 PEPTIDE DIMER ANTAGONISTS
<130> 18595.13
<150> 61/807,714
   <151> 2013-04-02
<150> 14/229,784
   <151> 2014-03-28
<160> 146
<170> PatentIn version 3.5
<210> 1
   <211> 14
   <212> PRT
   <213> Unknown
<220>
   <223> Synthetic Construct
<220>
   <221> VARIANT
   <222> (1)..(3)
   <223> absent, Gln, Asn, Asp, Pro, Gly, His, Ala, Ile, Phe, Lys, Arg, Glu, Leu, Val, Tyr, Trp, Ser, Met, Thr
<220>
   <221> MOD_RES
   <222> (1)..(4)
   <223> ACETYLATION
<220>
   <221> MOD_RES
   <222> (3)..(3)
   <223> D-Lys
<220>
   <221> MOD_RES
   <222> (3)..(3)
   <223> N(alpha)METHYLATION
<220>
   <221> LACTAM
   <222> (4)..(10)
<220>
   <221> VARIANT
   <222> (4)..(4)
   <223> Cys, Asp, Glu, Lys
<220>
   <221> MOD_RES
   <222> (4)..(4)
   <223> Pen, HGlu, HLys, Orn, Dap, Dab, Beta-Asp, Beta-Glu
<220>
   <221> DISULFID
   <222> (4)..(10)
   <223> Disulfide bond between Xaa at position 4 and Xaa at position 10
<220>
   <221> VARIANT
   <222> (5)..(5)
   <223> Gln, Asn, Asp, Pro, Gly, His, Ala, Ile, Phe, Lys, Arg, Glu, Leu, Val, Tyr, Trp, Met, Thr
<220>
   <221> MOD_RES
   <222> (5)..(5)
   <223> HArg, 4-Guan, Cit, Cav, Dap, Dab
<220>
   <221> MOD_RES
   <222> (5)..(5)
   <223> N(alpha)METHYLATION, Arg-Me-sym, Arg-Me-asym
<220>
   <221> VARIANT
   <222> (6)..(6)
   <223> Ser, Gln, Asn, Asp, Pro, Gly, His, Ala, Ile, Phe, Lys, Arg, Glu, Leu, Val, Thr, Trp, Tyr, Met
<220>
   <221> MOD_RES
   <222> (7)..(7)
   <223> N(alpha)METHYLATION
<220>
   <221> MOD_RES
   <222> (7)..(7)
   <223> D-Asp
<220>
   <221> VARIANT
   <222> (7)..(7)
   <223> Asp
<220>
   <221> VARIANT
   <222> (8)..(8)
   <223> Thr, Gln, Ser, Asn, Asp, Pro, Gly, His, Ala, Ile, Phe, Lys, Arg, Glu, Val, Tyr, Trp, Leu, Met
<220>
   <221> MOD_RES
   <222> (8)..(8)
   <223> N(alpha)METHYLATION
<220>
   <221> MOD_RES
   <222> (9)..(9)
   <223> HLeu, n-Butyl Ala, n-Pentyl Ala, n-Hexyl Ala, Nle, cyclobutyl-Ala, HCha
<220>
   <221> MOD_RES
   <222> (9)..(9)
   <223> N(alpha)METHYLATION
<220>
   <221> VARIANT
   <222> (9)..(9)
   <223> Gln, Asn, Asp, Pro, Gly, Ala, Phe, Leu, Glu, Ile, Val
<220>
   <221> MOD_RES
   <222> (10)..(10)
   <223> Pen, HAsp, HGlu, HLys, Orn, Dap, Dab
<220>
   <221> VARIANT
   <222> (10)..(10)
   <223> Cys, Asp, Lys, Glu
<220>
   <221> VARIANT
   <222> (11)..(11)
   <223> absent, Gly, Gln, Asn, Asp, Ala, Ile, Leu, Val, Met, Thr, Lys, Trp, Tyr, His, Glu, Ser, Arg, Pro, Phe
<220>
   <221> MOD_RES
   <222> (11)..(11)
   <223> Sar, 1-Nal, 2-Nal, HPhe, Phe(4-F), dihydro-Trp, Dap, Dab, Orn, D-Orn, D-Dap, D-Dab, Bip, Ala(3,3 diphenyl), Biphenyl-Ala, D-Phe, D-Trp, D-Tyr, D-Glu, D-His, D-Lys, 3,3-diPhe, Beta-HTrp, F(4-CF3), O-Me-Tyr, 4-Me-Phe, Phe(2,4-Cl2), Phe(3,4-Cl2),
<220>
   <221> MOD_RES
   <222> (11)..(11)
   <223> 1,1-Indane, 2,2-Indane, D-HPhe
<220>
   <221> MOD_RES
   <222> (11)..(11)
   <223> N(alpha)METHYLATION
<220>
   <221> MOD_RES
   <222> (11)..(11)
   <223> ACETYLATION
<220>
   <221> VARIANT
   <222> (12)..(12)
   <223> absent, Glu, Lys, Gln, Pro, Gly, His, Ala, Ile, Phe, Arg, Leu, Val, Tyr, Trp, Met, Ser, Asn, Asp
<220>
   <221> MOD_RES
   <222> (12)..(12)
   <223> ACETYLATION
<220>
   <221> MOD_RES
   <222> (12)..(12)
   <223> Dap, Dab, Orn, D-Orn, D-Dap, D-Dab, D-Lys, D-Glu, Beta-HGlu, 2-Nal, 1-Nal, D-Asp, Bip, Beta-HPhe, Beta-Glu, D-Tyr, Beta-HGlu, Gla
<220>
   <221> MOD_RES
   <222> (12)..(12)
   <223> N(alpha)METHYLATION
<220>
   <221> VARIANT
   <222> (13)..(13)
   <223> absent, Gln, Pro, Gly, His, Ala, Ile, Phe, Lys, Arg, Leu, Val, Tyr, Trp, Met, Glu, Ser, Asn
<220>
   <221> MOD_RES
   <222> (13)..(13)
   <223> Dap, Dab, Orn, D-Orn, D-Dap, D-Dab, D-Lys, Gla
<220>
   <221> MOD_RES
   <222> (13)..(13)
   <223> ACETYLATION
<220>
   <221> MOD_RES
   <222> (13)..(13)
   <223> N(alpha)METHYLATION
<220>
   <221> MOD_RES
   <222> (14)..(14)
   <223> ACETYLATION
<220>
   <221> VARIANT
   <222> (14)..(14)
   <223> absent, can be any naturally occuring amino acid
<220>
   <221> MOD_RES
   <222> (14)..(14)
   <223> N(alpha)METHYLATION
<400> 1
   Xaa Xaa Xaa Xaa Xaa Xaa Xaa Xaa Xaa Xaa Xaa Xaa Xaa Xaa
   1               5                   10
<210> 2
   <211> 10
   <212> PRT
   <213> Unknown
<220>
   <223> Synthetic Construct
<220>
   <221> VARIANT
   <222> (1)..(1)
   <223> Cys, Asp, Glu, Lys
<220>
   <221> MOD_RES
   <222> (1)..(1)
   <223> Pen, HGlu, HLys, Orn, Dap, Dab
<220>
   <221> MOD_RES
   <222> (1)..(1)
   <223> ACETYLATION
<220>
   <221> DISULFID
   <222> (1)..(7)
   <223> Disulfide bond between Xaa at position 1 and Xaa at position 7
<220>
   <221> LACTAM
   <222> (1)..(7)
<220>
   <221> VARIANT
   <222> (2)..(2)
   <223> Gln, Asn, Asp, Pro, Gly, His, Ala, Ile, Phe, Lys, Arg, Glu, Leu, Val, Tye, Trp, Met, Thr
<220>
   <221> MOD_RES
   <222> (2)..(2)
   <223> HArg, Dap, Dab, 4-Guan, Cit, Cav
<220>
   <221> MOD_RES
   <222> (2)..(2)
   <223> N(alpha)METHYLATION; N-Me-Arg; Arg-Me-sym; Arg-Me-asym
<220>
   <221> VARIANT
   <222> (3)..(3)
   <223> Ser, Thr, Gln, Asn, Asp, Pro, Gly, His, Ala, Ile, Phe, Lys, Arg, Glu, Leu, Val, Tyr, Trp, Met
<220>
   <221> MOD_RES
   <222> (4)..(4)
   <223> N(alpha)METHYLATION
<220>
   <221> VARIANT
   <222> (4)..(4)
   <223> Asp
<220>
   <221> MOD_RES
   <222> (5)..(5)
   <223> N(alpha)METHYLATION
<220>
   <221> VARIANT
   <222> (5)..(5)
   <223> Thr, Gln, Ser, Asn, Asp, Pro, Gly, His, Ala, Ile, Phe, Lys, Arg, Glu, Val, Tyr, Trp, Met
<220>
   <221> MOD_RES
   <222> (6)..(6)
   <223> HLeu, n-Butyl Ala, n-Pentyl Ala
<220>
   <221> MOD_RES
   <222> (6)..(6)
   <223> N(alpha)METHYLATION
<220>
   <221> VARIANT
   <222> (6)..(6)
   <223> Gln, Asn, Asp, Pro, Gly, Ala, Phe, Leu, Glu, Ile, Val
<220>
   <221> MOD_RES
   <222> (7)..(7)
   <223> Pen, HLys, Orn, HGlu, Dap, Dab
<220>
   <221> VARIANT
   <222> (7)..(7)
   <223> Cys, Asp, Lys, Glu
<220>
   <221> VARIANT
   <222> (8)..(8)
   <223> Gly, Gln, Asn, Asp, Ala, Ile, Leu, Val, Met, Thr, Lys, Trp, Tyr, His, Glu, Ser, Arg, Pro, Phe
<220>
   <221> MOD_RES
   <222> (8)..(8)
   <223> Sar, 1-Nal, 2-Nal, HPhe, Phe(4-F), dihydro-Trp, Dap, Dab, Orn, D-Orn, D-Dap, D-Dab, Bip, Ala(3,3 diphenyl), Biphenyl-Ala, D-Phe, D-Trp, D-Tyr, D-Glu, D-His, D-Lys, 3,3-diPhe, Beta-HTrp, F(4-CF3), O-Me-Tyr, 4-Me-Phe, Phe(2,4-Cl2), Phe(3,4-Cl2),
<220>
   <221> MOD_RES
   <222> (8)..(8)
   <223> 1,1-Indane, 2,2-Indane, D-HPhe
<220>
   <221> MOD_RES
   <222> (8)..(8)
   <223> N(alpha)METHYLATION
<220>
   <221> MOD_RES
   <222> (8)..(10)
   <223> ACETYLATION
<220>
   <221> MOD_RES
   <222> (9)..(9)
   <223> Dap, Dab, Orn, D-Orn, D-Dap, D-Dab, D-Lys, D-Glu, Beta-HGlu, 2-Nal, 1-Nal, D-Asp, Bip, Beta-HPhe, Beta-Glu, D-Tyr, Beta-HGlu
<220>
   <221> MOD_RES
   <222> (9)..(9)
   <223> N(alpha)METHYLATION
<220>
   <221> VARIANT
   <222> (9)..(9)
   <223> absent, Glu, Lys, Gln, Pro, Gly, His, Ala, Asp, Ile, Phe, Arg, Leu, Val, Tyr, Trp, Met, Ser, Asn
<220>
   <221> VARIANT
   <222> (10)..(10)
   <223> absent, Gln, Pro, Gly, His, Ala, Ile, Phe, Lys, Arg, Leu, Val, Tyr, Trp, Met, Glu, Ser, Asn
<220>
   <221> MOD_RES
   <222> (10)..(10)
   <223> Dap, Dab, Orn, D-Orn, D-Dap, D-Dab, D-Lys, Gla
<220>
   <221> MOD_RES
   <222> (10)..(10)
   <223> N(alpha)METHYLATION
<400> 2
   Xaa Xaa Xaa Xaa Xaa Xaa Xaa Xaa Xaa Xaa
   1               5                   10
<210> 3
   <211> 9
   <212> PRT
   <213> Unknown
<220>
   <223> Synthetic Construct
<220>
   <221> MOD_RES
   <222> (1)..(1)
   <223> ACETYLATION
<220>
   <221> DISULFID
   <222> (1)..(7)
   <223> Disulfide bond between Cys at position 1 and Cys at position 7
<220>
   <221> MOD_RES
   <222> (2)..(2)
   <223> N(alpha)METHYLATION
<220>
   <221> VARIANT
   <222> (3)..(3)
   <223> Xaa can be any naturally occurring amino acid
<220>
   <221> VARIANT
   <222> (5)..(6)
   <223> Xaa can be any naturally occurring amino acid
<220>
   <221> VARIANT
   <222> (8)..(9)
   <223> Xaa can be any naturally occurring amino acid
<220>
   <221> MOD_RES
   <222> (9)..(9)
   <223> ACETYLATION
<220>
   <221> MOD_RES
   <222> (9)..(9)
   <223> N(alpha)METHYLATION
<220>
   <221> MOD_RES
   <222> (9)..(9)
   <223> D-Lys, Dap, Dab, Orn, D-Orn, D-Dab, D-Dap
<400> 3
   Cys Arg Xaa Asp Xaa Xaa Cys Xaa Xaa
   1               5
<210> 4
   <211> 9
   <212> PRT
   <213> Unknown
<220>
   <223> Synthetic Construct
<220>
   <221> DISULFID
   <222> (1)..(7)
   <223> Disulfide bond between Cys at position 1 and Xaa at position 7
<220>
   <221> MOD_RES
   <222> (1)..(1)
   <223> ACETYLATION
<220>
   <221> MOD_RES
   <222> (2)..(2)
   <223> N(alpha)METHYLATION
<220>
   <221> VARIANT
   <222> (3)..(3)
   <223> Xaa can be any naturally occurring amino acid
<220>
   <221> VARIANT
   <222> (5)..(6)
   <223> Xaa can be any naturally occurring amino acid
<220>
   <221> MOD_RES
   <222> (7)..(7)
   <223> Pen
<220>
   <221> VARIANT
   <222> (8)..(9)
   <223> Can be any naturally occurring amino acid
<220>
   <221> MOD_RES
   <222> (9)..(9)
   <223> N(alpha)METHYLATION
<220>
   <221> MOD_RES
   <222> (9)..(9)
   <223> ACYLATION
<220>
   <221> MOD_RES
   <222> (9)..(9)
   <223> ACETYLATION
<220>
   <221> MOD_RES
   <222> (9)..(9)
   <223> D-Lys, Dap, Dab, Orn, D-Orn, D-Dab, D-Dap
<400> 4
   Cys Arg Xaa Asp Xaa Xaa Xaa Xaa Xaa
   1               5
<210> 5
   <211> 9
   <212> PRT
   <213> Unknown
<220>
   <223> Synthetic Construct
<220>
   <221> MOD_RES
   <222> (1)..(1)
   <223> Pen
<220>
   <221> MOD_RES
   <222> (1)..(1)
   <223> ACETYLATION
<220>
   <221> DISULFID
   <222> (1)..(7)
   <223> Disulfide bond between Xaa at position 1 and Cys at position 7
<220>
   <221> MOD_RES
   <222> (2)..(2)
   <223> N(alpha)METHYLATION
<220>
   <221> VARIANT
   <222> (3)..(3)
   <223> Xaa can be any naturally occurring amino acid
<220>
   <221> VARIANT
   <222> (5)..(6)
   <223> Xaa can be any naturally occurring amino acid
<220>
   <221> VARIANT
   <222> (8)..(9)
   <223> Xaa can be any naturally occurring amino acid
<220>
   <221> MOD_RES
   <222> (9)..(9)
   <223> N(alpha)METHYLATION
<220>
   <221> MOD_RES
   <222> (9)..(9)
   <223> ACETYLATION
<220>
   <221> MOD_RES
   <222> (9)..(9)
   <223> D-Lys, Dap, Dab, Orn, D-Orn, D-Dab, D-Dap
<400> 5
   Xaa Arg Xaa Asp Xaa Xaa Cys Xaa Xaa
   1               5
<210> 6
   <211> 9
   <212> PRT
   <213> Unknown
<220>
   <223> Synthetic Construct
<220>
   <221> MOD_RES
   <222> (1)..(1)
   <223> Pen
<220>
   <221> MOD_RES
   <222> (1)..(1)
   <223> ACETYLATION
<220>
   <221> DISULFID
   <222> (1)..(7)
   <223> Disulfide bond between Xaa at position 1 and Xaa at position 7
<220>
   <221> MOD_RES
   <222> (2)..(2)
   <223> N(alpha)METHYLATION
<220>
   <221> VARIANT
   <222> (3)..(3)
   <223> Xaa can be any naturally occurring amino acid
<220>
   <221> VARIANT
   <222> (5)..(6)
   <223> Xaa can be any naturally occurring amino acid
<220>
   <221> MOD_RES
   <222> (7)..(7)
   <223> Pen
<220>
   <221> VARIANT
   <222> (8)..(9)
   <223> Xaa can be any naturally occurring amino acid
<220>
   <221> MOD_RES
   <222> (9)..(9)
   <223> ACETYLATION
<220>
   <221> MOD_RES
   <222> (9)..(9)
   <223> N(alpha)METHYLATION
<220>
   <221> MOD_RES
   <222> (9)..(9)
   <223> D-Lys, Dap, Dab, Orn, D-Orn, D-Dab, D-Dap
<400> 6
   Xaa Arg Xaa Asp Xaa Xaa Xaa Xaa Xaa
   1               5
<210> 7
   <211> 10
   <212> PRT
   <213> Unknown
<220>
   <223> Synthetic Construct
<220>
   <221> VARIANT
   <222> (1)..(1)
   <223> Xaa can be any naturally occurring amino acid
<220>
   <221> MOD_RES
   <222> (1)..(1)
   <223> ACETYLATION
<220>
   <221> DISULFID
   <222> (2)..(8)
<220>
   <221> DISULFID
   <222> (2)..(8)
   <223> Disulfide bond between Cys at position 2 and Cys at position 8
<220>
   <221> MOD_RES
   <222> (3)..(3)
   <223> N(alpha)METHYLATION
<220>
   <221> VARIANT
   <222> (4)..(4)
   <223> Xaa can be any naturally occurring amino acid
<220>
   <221> VARIANT
   <222> (6)..(7)
   <223> Xaa can be any naturally occurring amino acid
<220>
   <221> VARIANT
   <222> (9)..(10)
   <223> Xaa can be any naturally occurring amino acid
<220>
   <221> MOD_RES
   <222> (10)..(10)
   <223> ACETYLATION
<220>
   <221> MOD_RES
   <222> (10)..(10)
   <223> N(alpha)METHYLATION
<220>
   <221> MOD_RES
   <222> (10)..(10)
   <223> D-Lys, Dap, Dab, Orn, D-Orn, D-Dab, D-Dap
<400> 7
   Xaa Cys Arg Xaa Asp Xaa Xaa Cys Xaa Xaa
   1               5                   10
<210> 8
   <211> 10
   <212> PRT
   <213> Unknown
<220>
   <223> Synthetic Construct
<220>
   <221> VARIANT
   <222> (1)..(1)
   <223> Xaa can be any naturally occurring amino acid
<220>
   <221> MOD_RES
   <222> (1)..(1)
   <223> ACETYLATION
<220>
   <221> DISULFID
   <222> (2)..(8)
   <223> Disulfide bond between Cys at position 2 and Xaa at position 8
<220>
   <221> MOD_RES
   <222> (3)..(3)
   <223> N(alpha)METHYLATION
<220>
   <221> VARIANT
   <222> (4)..(4)
   <223> Xaa can be any naturally occurring amino acid
<220>
   <221> VARIANT
   <222> (6)..(7)
   <223> Xaa can be any naturally occurring amino acid
<220>
   <221> MOD_RES
   <222> (8)..(8)
   <223> Pen
<220>
   <221> VARIANT
   <222> (9)..(10)
   <223> Xaa can be any naturally occurring amino acid
<220>
   <221> MOD_RES
   <222> (10)..(10)
   <223> ACETYLATION
<220>
   <221> MOD_RES
   <222> (10)..(10)
   <223> N(alpha)METHYLATION
<220>
   <221> MOD_RES
   <222> (10)..(10)
   <223> D-Lys, Dap, Dab, Orn, D-Orn, D-Dab, D-Dap
<400> 8
   Xaa Cys Arg Xaa Asp Xaa Xaa Xaa Xaa Xaa
   1               5                   10
<210> 9
   <211> 10
   <212> PRT
   <213> Unknown
<220>
   <223> Synthetic Construct
<220>
   <221> VARIANT
   <222> (1)..(1)
   <223> Xaa can be any naturally occurring amino acid
<220>
   <221> MOD_RES
   <222> (1)..(1)
   <223> ACETYLATION
<220>
   <221> MOD_RES
   <222> (2)..(2)
   <223> Pen
<220>
   <221> DISULFID
   <222> (2)..(8)
   <223> Disulfide bond between Xaa at position 2 and Cys at position 8
<220>
   <221> MOD_RES
   <222> (3)..(3)
   <223> N(alpha)METHYLATION
<220>
   <221> VARIANT
   <222> (4)..(4)
   <223> Xaa can be any naturally occurring amino acid
<220>
   <221> VARIANT
   <222> (6)..(7)
   <223> Xaa can be any naturally occurring amino acid
<220>
   <221> VARIANT
   <222> (9)..(10)
   <223> Xaa can be any naturally occurring amino acid
<220>
   <221> MOD_RES
   <222> (10)..(10)
   <223> ACETYLATION
<220>
   <221> MOD_RES
   <222> (10)..(10)
   <223> N(alpha)METHYLATION
<220>
   <221> MOD_RES
   <222> (10)..(10)
   <223> D-Lys, Dap, Dab, Orn, D-Orn, D-Dab, D-Dap
<400> 9
   Xaa Xaa Arg Xaa Asp Xaa Xaa Cys Xaa Xaa
   1               5                   10
<210> 10
   <211> 10
   <212> PRT
   <213> Unknown
<220>
   <223> Synthetic Construct
<220>
   <221> VARIANT
   <222> (1)..(1)
   <223> Xaa can be any naturally occurring amino acid
<220>
   <221> MOD_RES
   <222> (1)..(1)
   <223> ACETYLATION
<220>
   <221> MOD_RES
   <222> (2)..(2)
   <223> Pen
<220>
   <221> DISULFID
   <222> (2)..(8)
   <223> Disulfide bond between Xaa at position 2 and Xaa at position 8
<220>
   <221> MOD_RES
   <222> (3)..(3)
   <223> N(alpha)METHYLATION
<220>
   <221> VARIANT
   <222> (4)..(4)
   <223> Xaa can be any naturally occurring amino acid
<220>
   <221> VARIANT
   <222> (6)..(7)
   <223> Xaa can be any naturally occurring amino acid
<220>
   <221> MOD_RES
   <222> (8)..(8)
   <223> Pen
<220>
   <221> VARIANT
   <222> (9)..(10)
   <223> Xaa can be any naturally occurring amino acid
<220>
   <221> MOD_RES
   <222> (10)..(10)
   <223> ACETYLATION
<220>
   <221> MOD_RES
   <222> (10)..(10)
   <223> N(alpha)METHYLATION
<220>
   <221> MOD_RES
   <222> (10)..(10)
   <223> D-Lys, Dap, Dab, Orn, D-Orn, D-Dab, D-Dap
<400> 10
   Xaa Xaa Arg Xaa Asp Xaa Xaa Xaa Xaa Xaa
   1               5                   10
<210> 11
   <211> 10
   <212> PRT
   <213> Unknown
<220>
   <223> Synthetic Construct
<220>
   <221> MOD_RES
   <222> (1)..(1)
   <223> ACETYLATION
<220>
   <221> DISULFID
   <222> (1)..(7)
   <223> Disulfide bond between Cys at position 1 and Cys at position 7
<220>
   <221> MOD_RES
   <222> (2)..(2)
   <223> N(alpha)METHYLATION
<220>
   <221> VARIANT
   <222> (3)..(3)
   <223> Xaa can be any naturally occurring amino acid
<220>
   <221> VARIANT
   <222> (5)..(5)
   <223> Xaa can be any naturally occurring amino acid
<220>
   <221> VARIANT
   <222> (6)..(6)
   <223> Xaa can be any naturally occurring amino acid
<220>
   <221> VARIANT
   <222> (8)..(10)
   <223> Xaa can be any naturally occurring amino acid
<220>
   <221> MOD_RES
   <222> (10)..(10)
   <223> N(alpha)METHYLATION
<220>
   <221> MOD_RES
   <222> (10)..(10)
   <223> D-Lys, Dap, Dab, Orn, D-Orn, D-Dab, D-Dap
<220>
   <221> MOD_RES
   <222> (10)..(10)
   <223> ACETYLATION
<400> 11
   Cys Arg Xaa Asp Xaa Xaa Cys Xaa Xaa Xaa
   1               5                   10
<210> 12
   <211> 10
   <212> PRT
   <213> Unknown
<220>
   <223> Synthetic Construct
<220>
   <221> MOD_RES
   <222> (1)..(1)
   <223> ACETYLATION
<220>
   <221> DISULFID
   <222> (1)..(7)
   <223> Disulfide bond between Cys at position 1 and Xaa at position 7
<220>
   <221> MOD_RES
   <222> (2)..(2)
   <223> N(alpha)METHYLATION
<220>
   <221> VARIANT
   <222> (3)..(3)
   <223> Xaa can be any naturally occurring amino acid
<220>
   <221> VARIANT
   <222> (5)..(5)
   <223> Xaa can be any naturally occurring amino acid
<220>
   <221> VARIANT
   <222> (6)..(6)
   <223> Xaa can be any naturally occurring amino acid
<220>
   <221> MOD_RES
   <222> (7)..(7)
   <223> Pen
<220>
   <221> VARIANT
   <222> (8)..(10)
   <223> Xaa can be any naturally occurring amino acid
<220>
   <221> MOD_RES
   <222> (10)..(10)
   <223> ACETYLATION
<220>
   <221> MOD_RES
   <222> (10)..(10)
   <223> N(alpha)METHYLATION
<220>
   <221> MOD_RES
   <222> (10)..(10)
   <223> D-Lys, Dap, Dab, Orn, D-Orn, D-Dab, D-Dap
<400> 12
   Cys Arg Xaa Asp Xaa Xaa Xaa Xaa Xaa Xaa
   1               5                   10
<210> 13
   <211> 10
   <212> PRT
   <213> Unknown
<220>
   <223> Synthetic Construct
<220>
   <221> MOD_RES
   <222> (1)..(1)
   <223> Pen
<220>
   <221> MOD_RES
   <222> (1)..(1)
   <223> ACETYLATION
<220>
   <221> DISULFID
   <222> (1)..(7)
   <223> Disulfide bond between Xaa at position 1 and Cys at position 7
<220>
   <221> MOD_RES
   <222> (2)..(2)
   <223> N(alpha)METHYLATION
<220>
   <221> VARIANT
   <222> (3)..(3)
   <223> Xaa can be any naturally occurring amino acid
<220>
   <221> VARIANT
   <222> (5)..(6)
   <223> Xaa can be any naturally occurring amino acid
<220>
   <221> VARIANT
   <222> (8)..(10)
   <223> Xaa can be any naturally occurring amino acid
<220>
   <221> MOD_RES
   <222> (10)..(10)
   <223> ACETYLATION
<220>
   <221> MOD_RES
   <222> (10)..(10)
   <223> N(alpha)METHYLATION
<220>
   <221> MOD_RES
   <222> (10)..(10)
   <223> D-Lys, Dap, Dab, Orn, D-Orn, D-Dab, D-Dap
<400> 13
   Xaa Arg Xaa Asp Xaa Xaa Cys Xaa Xaa Xaa
   1               5                   10
<210> 14
   <211> 10
   <212> PRT
   <213> Unknown
<220>
   <223> Synthetic Construct
<220>
   <221> MOD_RES
   <222> (1)..(1)
   <223> Pen
<220>
   <221> MOD_RES
   <222> (1)..(1)
   <223> ACETYLATION
<220>
   <221> DISULFID
   <222> (1)..(7)
   <223> Disulfide bond between Xaa at position 1 and Xaa at position 7
<220>
   <221> MOD_RES
   <222> (2)..(2)
   <223> N(alpha)METHYLATION
<220>
   <221> VARIANT
   <222> (3)..(3)
   <223> Xaa can be any naturally occurring amino acid
<220>
   <221> VARIANT
   <222> (5)..(6)
   <223> Xaa can be any naturally occurring amino acid
<220>
   <221> MOD_RES
   <222> (7)..(7)
   <223> Pen
<220>
   <221> VARIANT
   <222> (8)..(10)
   <223> Xaa can be any naturally occurring amino acid
<220>
   <221> MOD_RES
   <222> (10)..(10)
   <223> ACETYLATION
<220>
   <221> MOD_RES
   <222> (10)..(10)
   <223> N(alpha)METHYLATION
<220>
   <221> MOD_RES
   <222> (10)..(10)
   <223> D-Lys, Dap, Dab, Orn, D-Orn, D-Dab, D-Dap
<400> 14
   Xaa Arg Xaa Asp Xaa Xaa Xaa Xaa Xaa Xaa
   1               5                   10
<210> 15
   <211> 11
   <212> PRT
   <213> Unknown
<220>
   <223> Synthetic Construct
<220>
   <221> VARIANT
   <222> (1)..(1)
   <223> Xaa can be any naturally occurring amino acid
<220>
   <221> MOD_RES
   <222> (1)..(1)
   <223> ACETYLATION
<220>
   <221> DISULFID
   <222> (2)..(8)
   <223> Disulfide bond between Cys at position 2 and Cys at position 8
<220>
   <221> MOD_RES
   <222> (3)..(3)
   <223> N(alpha)METHYLATION
<220>
   <221> VARIANT
   <222> (4)..(4)
   <223> Xaa can be any naturally occurring amino acid
<220>
   <221> VARIANT
   <222> (6)..(7)
   <223> Xaa can be any naturally occurring amino acid
<220>
   <221> VARIANT
   <222> (9)..(11)
   <223> Xaa can be any naturally occurring amino acid
<220>
   <221> MOD_RES
   <222> (11)..(11)
   <223> ACETYLATION
<220>
   <221> MOD_RES
   <222> (11)..(11)
   <223> N(alpha)METHYLATION
<220>
   <221> MOD_RES
   <222> (11)..(11)
   <223> D-Lys, Dap, Dab, Orn, D-Orn, D-Dab, D-Dap
<400> 15
   Xaa Cys Arg Xaa Asp Xaa Xaa Cys Xaa Xaa Xaa
   1               5                   10
<210> 16
   <211> 11
   <212> PRT
   <213> Unknown
<220>
   <223> Synthetic Construct
<220>
   <221> VARIANT
   <222> (1)..(1)
   <223> Xaa can be any naturally occurring amino acid
<220>
   <221> MOD_RES
   <222> (1)..(1)
   <223> ACETYLATION
<220>
   <221> DISULFID
   <222> (2)..(8)
   <223> Disulfide bond between Cys at position 2 and Xaa at position 8
<220>
   <221> MOD_RES
   <222> (3)..(3)
   <223> N(alpha)METHYLATION
<220>
   <221> VARIANT
   <222> (4)..(4)
   <223> Xaa can be any naturally occurring amino acid
<220>
   <221> VARIANT
   <222> (6)..(7)
   <223> Xaa can be any naturally occurring amino acid
<220>
   <221> MOD_RES
   <222> (8)..(8)
   <223> Pen
<220>
   <221> VARIANT
   <222> (9)..(11)
   <223> Xaa can be any naturally occurring amino acid
<220>
   <221> MOD_RES
   <222> (11)..(11)
   <223> ACETYLATION
<220>
   <221> MOD_RES
   <222> (11)..(11)
   <223> N(alpha)METHYLATION
<220>
   <221> MOD_RES
   <222> (11)..(11)
   <223> D-Lys, Dap, Dab, Orn, D-Orn, D-Dab, D-Dap
<400> 16
   Xaa Cys Arg Xaa Asp Xaa Xaa Xaa Xaa Xaa Xaa
   1               5                   10
<210> 17
   <211> 11
   <212> PRT
   <213> Unknown
<220>
   <223> Synthetic Construct
<220>
   <221> VARIANT
   <222> (1)..(1)
   <223> Xaa can be any naturally occurring amino acid
<220>
   <221> MOD_RES
   <222> (1)..(1)
   <223> ACETYLATION
<220>
   <221> DISULFID
   <222> (2)..(8)
   <223> Disulfide bond between Xaa at position 2 and Cys at position 8
<220>
   <221> MOD_RES
   <222> (2)..(2)
   <223> Pen
<220>
   <221> MOD_RES
   <222> (3)..(3)
   <223> N(alpha)METHYLATION
<220>
   <221> VARIANT
   <222> (4)..(4)
   <223> Xaa can be any naturally occurring amino acid
<220>
   <221> VARIANT
   <222> (6)..(7)
   <223> Xaa can be any naturally occurring amino acid
<220>
   <221> VARIANT
   <222> (9)..(11)
   <223> Xaa can be any naturally occurring amino acid
<220>
   <221> MOD_RES
   <222> (11)..(11)
   <223> ACETYLATION
<220>
   <221> MOD_RES
   <222> (11)..(11)
   <223> N(alpha)METHYLATION
<220>
   <221> MOD_RES
   <222> (11)..(11)
   <223> D-Lys, Dap, Dab, Orn, D-Orn, D-Dab, D-Dap
<400> 17
   Xaa Xaa Arg Xaa Asp Xaa Xaa Cys Xaa Xaa Xaa
   1               5                   10
<210> 18
   <211> 11
   <212> PRT
   <213> Unknown
<220>
   <223> Synthetic Construct
<220>
   <221> VARIANT
   <222> (1)..(1)
   <223> Xaa can be any naturally occurring amino acid
<220>
   <221> MOD_RES
   <222> (1)..(1)
   <223> ACETYLATION
<220>
   <221> DISULFID
   <222> (2)..(8)
   <223> Disulfide bond between Xaa at position 2 and Xaa at position 8
<220>
   <221> MOD_RES
   <222> (2)..(2)
   <223> Pen
<220>
   <221> MOD_RES
   <222> (3)..(3)
   <223> N(alpha)METHYLATION
<220>
   <221> VARIANT
   <222> (4)..(4)
   <223> Xaa can be any naturally occurring amino acid
<220>
   <221> VARIANT
   <222> (6)..(7)
   <223> Xaa can be any naturally occurring amino acid
<220>
   <221> MOD_RES
   <222> (8)..(8)
   <223> Pen
<220>
   <221> VARIANT
   <222> (9)..(11)
   <223> Xaa can be any naturally occurring amino acid
<220>
   <221> MOD_RES
   <222> (11)..(11)
   <223> ACETYLATION
<220>
   <221> MOD_RES
   <222> (11)..(11)
   <223> N(alpha)METHYLATION
<220>
   <221> MOD_RES
   <222> (11)..(11)
   <223> D-Lys, Dap, Dab, Orn, D-Orn, D-Dab, D-Dap
<400> 18
   Xaa Xaa Arg Xaa Asp Xaa Xaa Xaa Xaa Xaa Xaa
   1               5                   10
<210> 19
   <211> 12
   <212> PRT
   <213> Unknown
<220>
   <223> Synthetic Construct
<220>
   <221> VARIANT
   <222> (1)..(2)
   <223> Xaa can be any naturally occurring amino acid
<220>
   <221> MOD_RES
   <222> (1)..(1)
   <223> ACETYLATION
<220>
   <221> DISULFID
   <222> (3)..(9)
   <223> Disulfide bond between Cys at position 3 and Cys at position 9
<220>
   <221> MOD_RES
   <222> (4)..(4)
   <223> N(alpha)METHYLATION
<220>
   <221> VARIANT
   <222> (5)..(5)
   <223> Xaa can be any naturally occurring amino acid
<220>
   <221> VARIANT
   <222> (7)..(8)
   <223> Xaa can be any naturally occurring amino acid
<220>
   <221> VARIANT
   <222> (10)..(12)
   <223> Xaa can be any naturally occurring amino acid
<220>
   <221> MOD_RES
   <222> (12)..(12)
   <223> N(alpha)METHYLATION
<220>
   <221> MOD_RES
   <222> (12)..(12)
   <223> D-Lys, Dap, Dab, Orn, D-Orn, D-Dab, D-Dap
<220>
   <221> MOD_RES
   <222> (12)..(12)
   <223> ACETYLATION
<400> 19
   Xaa Xaa Cys Arg Xaa Asp Xaa Xaa Cys Xaa Xaa Xaa
   1               5                   10
<210> 20
   <211> 12
   <212> PRT
   <213> Unknown
<220>
   <223> Synthetic Construct
<220>
   <221> VARIANT
   <222> (1)..(2)
   <223> Xaa can be any naturally occurring amino acid
<220>
   <221> MOD_RES
   <222> (1)..(1)
   <223> ACETYLATION
<220>
   <221> DISULFID
   <222> (3)..(9)
   <223> Disulfide bond between Cys at position 3 and Xaa at position 9
<220>
   <221> MOD_RES
   <222> (4)..(4)
   <223> N(alpha)METHYLATION
<220>
   <221> VARIANT
   <222> (5)..(5)
   <223> Xaa can be any naturally occurring amino acid
<220>
   <221> VARIANT
   <222> (7)..(8)
   <223> Xaa can be any naturally occurring amino acid
<220>
   <221> MOD_RES
   <222> (9)..(9)
   <223> Pen
<220>
   <221> VARIANT
   <222> (10)..(12)
   <223> Xaa can be any naturally occurring amino acid
<220>
   <221> MOD_RES
   <222> (12)..(12)
   <223> N(alpha)METHYLATION
<220>
   <221> MOD_RES
   <222> (12)..(12)
   <223> D-Lys, Dap, Dab, Orn, D-Orn, D-Dab, D-Dap
<220>
   <221> MOD_RES
   <222> (12)..(12)
   <223> ACETYLATION
<400> 20
   Xaa Xaa Cys Arg Xaa Asp Xaa Xaa Xaa Xaa Xaa Xaa
   1               5                   10
<210> 21
   <211> 12
   <212> PRT
   <213> Unknown
<220>
   <223> Synthetic Construct
<220>
   <221> VARIANT
   <222> (1)..(2)
   <223> Xaa can be any naturally occurring amino acid
<220>
   <221> MOD_RES
   <222> (1)..(1)
   <223> ACETYLATION
<220>
   <221> MOD_RES
   <222> (3)..(3)
   <223> Pen
<220>
   <221> DISULFID
   <222> (3)..(9)
   <223> Disulfide bond between Xaa at position 3 and Cys at position 9
<220>
   <221> MOD_RES
   <222> (4)..(4)
   <223> N(alpha)METHYLATION
<220>
   <221> VARIANT
   <222> (5)..(5)
   <223> Xaa can be any naturally occurring amino acid
<220>
   <221> VARIANT
   <222> (7)..(8)
   <223> Xaa can be any naturally occurring amino acid
<220>
   <221> VARIANT
   <222> (10)..(12)
   <223> Xaa can be any naturally occurring amino acid
<220>
   <221> MOD_RES
   <222> (12)..(12)
   <223> N(alpha)METHYLATION
<220>
   <221> MOD_RES
   <222> (12)..(12)
   <223> D-Lys, Dap, Dab, Orn, D-Orn, D-Dab, D-Dap
<220>
   <221> MOD_RES
   <222> (12)..(12)
   <223> ACETYLATION
<400> 21
   Xaa Xaa Xaa Arg Xaa Asp Xaa Xaa Cys Xaa Xaa Xaa
   1               5                   10
<210> 22
   <211> 12
   <212> PRT
   <213> Unknown
<220>
   <223> Synthetic Construct
<220>
   <221> VARIANT
   <222> (1)..(2)
   <223> Xaa can be any naturally occurring amino acid
<220>
   <221> MOD_RES
   <222> (1)..(1)
   <223> ACETYLATION
<220>
   <221> MOD_RES
   <222> (3)..(3)
   <223> Pen
<220>
   <221> DISULFID
   <222> (3)..(9)
   <223> Disulfide bond between Xaa at position 3 and Xaa at position 9
<220>
   <221> MOD_RES
   <222> (4)..(4)
   <223> N(alpha)METHYLATION
<220>
   <221> VARIANT
   <222> (5)..(5)
<220>
   <221> VARIANT
   <222> (7)..(8)
<220>
   <221> MOD_RES
   <222> (9)..(9)
   <223> Pen
<220>
   <221> VARIANT
   <222> (10)..(12)
   <223> Xaa can be any naturally occurring amino acid
<220>
   <221> MOD_RES
   <222> (12)..(12)
   <223> N(alpha)METHYLATION
<220>
   <221> MOD_RES
   <222> (12)..(12)
   <223> D-Lys, Dap, Dab, Orn, D-Orn, D-Dab, D-Dap
<220>
   <221> MOD_RES
   <222> (12)..(12)
   <223> ACETYLATION
<400> 22
   Xaa Xaa Xaa Arg Xaa Asp Xaa Xaa Xaa Xaa Xaa Xaa
   1               5                   10
<210> 23
   <211> 13
   <212> PRT
   <213> Unknown
<220>
   <223> Synthetic Construct
<220>
   <221> VARIANT
   <222> (1)..(3)
   <223> Xaa can be any naturally occurring amino acid
<220>
   <221> MOD_RES
   <222> (1)..(1)
   <223> ACETYLATION
<220>
   <221> DISULFID
   <222> (4)..(10)
   <223> Disulfide bond between Cys at position 4 and Cys at position 10
<220>
   <221> MOD_RES
   <222> (5)..(5)
   <223> N(alpha)METHYLATION
<220>
   <221> VARIANT
   <222> (6)..(6)
   <223> Xaa can be any naturally occurring amino acid
<220>
   <221> VARIANT
   <222> (8)..(9)
   <223> Xaa can be any naturally occurring amino acid
<220>
   <221> VARIANT
   <222> (11)..(13)
   <223> Xaa can be any naturally occurring amino acid
<220>
   <221> MOD_RES
   <222> (13)..(13)
   <223> N(alpha)METHYLATION
<220>
   <221> MOD_RES
   <222> (13)..(13)
   <223> D-Lys, Dap, Dab, Orn, D-Orn, D-Dab, D-Dap
<220>
   <221> MOD_RES
   <222> (13)..(13)
   <223> ACETYLATION
<400> 23
   Xaa Xaa Xaa Cys Arg Xaa Asp Xaa Xaa Cys Xaa Xaa Xaa
   1               5                   10
<210> 24
   <211> 13
   <212> PRT
   <213> Unknown
<220>
   <223> Synthetic Construct
<220>
   <221> VARIANT
   <222> (1)..(3)
   <223> Xaa can be any naturally occurring amino acid
<220>
   <221> MOD_RES
   <222> (1)..(1)
   <223> ACETYLATION
<220>
   <221> DISULFID
   <222> (4)..(10)
   <223> Disulfide bond between Cys at position 4 and Xaa at position 10
<220>
   <221> MOD_RES
   <222> (5)..(5)
   <223> N(alpha)METHYLATION
<220>
   <221> VARIANT
   <222> (6)..(6)
   <223> Xaa can be any naturally occurring amino acid
<220>
   <221> VARIANT
   <222> (8)..(9)
   <223> Xaa can be any naturally occurring amino acid
<220>
   <221> MOD_RES
   <222> (10)..(10)
   <223> Pen
<220>
   <221> VARIANT
   <222> (11)..(13)
   <223> Xaa can be any naturally occurring amino acid
<220>
   <221> MOD_RES
   <222> (13)..(13)
   <223> N(alpha)METHYLATION
<220>
   <221> MOD_RES
   <222> (13)..(13)
   <223> D-Lys, Dap, Dab, Orn, D-Orn, D-Dab, D-Dap
<220>
   <221> MOD_RES
   <222> (13)..(13)
   <223> ACETYLATION
<400> 24
   Xaa Xaa Xaa Cys Arg Xaa Asp Xaa Xaa Xaa Xaa Xaa Xaa
   1               5                   10
<210> 25
   <211> 13
   <212> PRT
   <213> Unknown
<220>
   <223> Synthetic Construct
<220>
   <221> VARIANT
   <222> (1)..(3)
   <223> Xaa can be any naturally occurring amino acid
<220>
   <221> MOD_RES
   <222> (1)..(1)
   <223> ACETYLATION
<220>
   <221> MOD_RES
   <222> (4)..(4)
   <223> Pen
<220>
   <221> DISULFID
   <222> (4)..(10)
   <223> Disulfide bond between Xaa at position 4 and Cys at position 10
<220>
   <221> MOD_RES
   <222> (5)..(5)
   <223> N(alpha)METHYLATION
<220>
   <221> VARIANT
   <222> (6)..(6)
   <223> Xaa can be any naturally occurring amino acid
<220>
   <221> VARIANT
   <222> (8)..(9)
   <223> Xaa can be any naturally occurring amino acid
<220>
   <221> VARIANT
   <222> (11)..(13)
   <223> Xaa can be any naturally occurring amino acid
<220>
   <221> MOD_RES
   <222> (13)..(13)
   <223> ACETYLATION
<220>
   <221> MOD_RES
   <222> (13)..(13)
   <223> N(alpha)METHYLATION
<220>
   <221> MOD_RES
   <222> (13)..(13)
   <223> D-Lys, Dap, Dab, Orn, D-Orn, D-Dab, D-Dap
<400> 25
   Xaa Xaa Xaa Xaa Arg Xaa Asp Xaa Xaa Cys Xaa Xaa Xaa
   1               5                   10
<210> 26
   <211> 13
   <212> PRT
   <213> Unknown
<220>
   <223> Synthetic Construct
<220>
   <221> VARIANT
   <222> (1)..(3)
   <223> Xaa can be any naturally occurring amino acid
<220>
   <221> MOD_RES
   <222> (1)..(1)
   <223> ACETYLATION
<220>
   <221> MOD_RES
   <222> (4)..(4)
   <223> Pen
<220>
   <221> DISULFID
   <222> (4)..(10)
   <223> Disulfide bond between Xaa at position 4 and Xaa at position 10
<220>
   <221> MOD_RES
   <222> (5)..(5)
   <223> N(alpha)METHYLATION
<220>
   <221> VARIANT
   <222> (6)..(6)
   <223> Xaa can be any naturally occurring amino acid
<220>
   <221> VARIANT
   <222> (8)..(9)
   <223> Xaa can be any naturally occurring amino acid
<220>
   <221> MOD_RES
   <222> (10)..(10)
   <223> Pen
<220>
   <221> VARIANT
   <222> (11)..(13)
   <223> Xaa can be any naturally occurring amino acid
<220>
   <221> MOD_RES
   <222> (13)..(13)
   <223> ACETYLATION
<220>
   <221> MOD_RES
   <222> (13)..(13)
   <223> N(alpha)METHYLATION
<220>
   <221> MOD_RES
   <222> (13)..(13)
   <223> D-Lys, Dap, Dab, Orn, D-Orn, D-Dab, D-Dap
<400> 26
   Xaa Xaa Xaa Xaa Arg Xaa Asp Xaa Xaa Xaa Xaa Xaa Xaa
   1               5                   10
<210> 27
   <211> 8
   <212> PRT
   <213> Unknown
<220>
   <223> Synthetic Construct
<220>
   <221> VARIANT
   <222> (1)..(1)
   <223> Xaa can be any naturally occurring amino acid
<220>
   <221> MOD_RES
   <222> (1)..(1)
   <223> ACETYLATION
<220>
   <221> DISULFID
   <222> (2)..(8)
   <223> Disulfide bond between Cys at position 2 and Cys at position 8
<220>
   <221> MOD_RES
   <222> (3)..(3)
   <223> N(alpha)METHYLATION
<220>
   <221> VARIANT
   <222> (4)..(4)
   <223> Xaa can be any naturally occurring amino acid
<220>
   <221> VARIANT
   <222> (6)..(7)
   <223> Xaa can be any naturally occurring amino acid
<220>
   <221> MOD_RES
   <222> (8)..(8)
   <223> ACETYLATION
<220>
   <221> MOD_RES
   <222> (8)..(8)
   <223> N(alpha)METHYLATION
<220>
   <221> MOD_RES
   <222> (8)..(8)
   <223> D-Lys, Dap, Dab, Orn, D-Orn, D-Dab, D-Dap
<400> 27
   Xaa Cys Arg Xaa Asp Xaa Xaa Cys
   1               5
<210> 28
   <211> 9
   <212> PRT
   <213> Unknown
<220>
   <223> Synthetic Construct
<220>
   <221> VARIANT
   <222> (1)..(1)
   <223> Xaa can be any naturally occurring amino acid
<220>
   <221> MOD_RES
   <222> (1)..(1)
   <223> ACETYLATION
<220>
   <221> DISULFID
   <222> (2)..(8)
   <223> Disulfide bond between Cys at position 2 and Xaa at position 8
<220>
   <221> MOD_RES
   <222> (3)..(3)
   <223> N(alpha)METHYLATION
<220>
   <221> VARIANT
   <222> (4)..(4)
   <223> Xaa can be any naturally occurring amino acid
<220>
   <221> VARIANT
   <222> (6)..(7)
   <223> Xaa can be any naturally occurring amino acid
<220>
   <221> MOD_RES
   <222> (8)..(8)
   <223> Pen
<220>
   <221> MOD_RES
   <222> (9)..(9)
   <223> ACETYLATION
<220>
   <221> VARIANT
   <222> (9)..(9)
   <223> Xaa can be any naturally occurring amino acid
<220>
   <221> MOD_RES
   <222> (9)..(9)
   <223> N(alpha)METHYLATION
<220>
   <221> MOD_RES
   <222> (9)..(9)
   <223> D-Lys, Dap, Dab, Orn, D-Orn, D-Dab, D-Dap
<400> 28
   Xaa Cys Arg Xaa Asp Xaa Xaa Xaa Xaa
   1               5
<210> 29
   <211> 9
   <212> PRT
   <213> Unknown
<220>
   <223> Synthetic Construct
<220>
   <221> VARIANT
   <222> (1)..(1)
   <223> Xaa can be any naturally occurring amino acid
<220>
   <221> MOD_RES
   <222> (1)..(1)
   <223> ACETYLATION
<220>
   <221> MOD_RES
   <222> (2)..(2)
   <223> Pen
<220>
   <221> DISULFID
   <222> (2)..(8)
   <223> Disulfide bond between Xaa at position 2 and Cys at position 8
<220>
   <221> MOD_RES
   <222> (3)..(3)
   <223> N(alpha)METHYLATION
<220>
   <221> VARIANT
   <222> (4)..(4)
   <223> Xaa can be any naturally occurring amino acid
<220>
   <221> VARIANT
   <222> (6)..(7)
   <223> Xaa can be any naturally occurring amino acid
<220>
   <221> MOD_RES
   <222> (9)..(9)
   <223> ACETYLATION
<220>
   <221> VARIANT
   <222> (9)..(9)
   <223> Xaa can be any naturally occurring amino acid
<220>
   <221> MOD_RES
   <222> (9)..(9)
   <223> N(alpha)METHYLATION
<220>
   <221> MOD_RES
   <222> (9)..(9)
   <223> D-Lys, Dap, Dab, Orn, D-Orn, D-Dab, D-Dap
<400> 29
   Xaa Xaa Arg Xaa Asp Xaa Xaa Cys Xaa
   1               5
<210> 30
   <211> 9
   <212> PRT
   <213> Unknown
<220>
   <223> Synthetic Construct
<220>
   <221> VARIANT
   <222> (1)..(1)
   <223> Xaa can be any naturally occurring amino acid
<220>
   <221> MOD_RES
   <222> (1)..(1)
   <223> ACETYLATION
<220>
   <221> MOD_RES
   <222> (2)..(2)
   <223> Pen
<220>
   <221> DISULFID
   <222> (2)..(8)
   <223> Disulfide bond between Xaa at position 2 and Xaa at position 8
<220>
   <221> MOD_RES
   <222> (3)..(3)
   <223> N(alpha)METHYLATION
<220>
   <221> VARIANT
   <222> (4)..(4)
   <223> Xaa can be any naturally occurring amino acid
<220>
   <221> VARIANT
   <222> (6)..(7)
   <223> Xaa can be any naturally occurring amino acid
<220>
   <221> MOD_RES
   <222> (8)..(8)
   <223> Pen
<220>
   <221> MOD_RES
   <222> (9)..(9)
   <223> ACETYLATION
<220>
   <221> VARIANT
   <222> (9)..(9)
   <223> Xaa can be any naturally occurring amino acid
<220>
   <221> MOD_RES
   <222> (9)..(9)
   <223> N(alpha)METHYLATION
<220>
   <221> MOD_RES
   <222> (9)..(9)
   <223> D-Lys, Dap, Dab, Orn, D-Orn, D-Dab, D-Dap
<400> 30
   Xaa Xaa Arg Xaa Asp Xaa Xaa Xaa Xaa
   1               5
<210> 31
   <211> 10
   <212> PRT
   <213> Unknown
<220>
   <223> Synthetic Construct
<220>
   <221> VARIANT
   <222> (1)..(2)
   <223> Xaa can be any naturally occurring amino acid
<220>
   <221> MOD_RES
   <222> (1)..(1)
   <223> ACETYLATION
<220>
   <221> DISULFID
   <222> (3)..(9)
   <223> Disulfide bond between Cys at position 3 and Cys at position 9
<220>
   <221> MOD_RES
   <222> (4)..(4)
   <223> N(alpha)METHYLATION
<220>
   <221> VARIANT
   <222> (5)..(5)
   <223> Xaa can be any naturally occurring amino acid
<220>
   <221> VARIANT
   <222> (7)..(8)
   <223> Xaa can be any naturally occurring amino acid
<220>
   <221> VARIANT
   <222> (10)..(10)
   <223> Xaa can be any naturally occurring amino acid
<220>
   <221> MOD_RES
   <222> (10)..(10)
   <223> N(alpha)METHYLATION
<220>
   <221> MOD_RES
   <222> (10)..(10)
   <223> D-Lys, Dap, Dab, Orn, D-Orn, D-Dab, D-Dap
<220>
   <221> MOD_RES
   <222> (10)..(10)
   <223> ACETYLATION
<400> 31
   Xaa Xaa Cys Arg Xaa Asp Xaa Xaa Cys Xaa
   1               5                   10
<210> 32
   <211> 10
   <212> PRT
   <213> Unknown
<220>
   <223> Synthetic Construct
<220>
   <221> VARIANT
   <222> (1)..(2)
   <223> Xaa can be any naturally occurring amino acid
<220>
   <221> MOD_RES
   <222> (1)..(1)
   <223> ACETYLATION
<220>
   <221> DISULFID
   <222> (3)..(9)
   <223> Disulfide bond between Cys at position 3 and Xaa at position 9
<220>
   <221> MOD_RES
   <222> (4)..(4)
   <223> N(alpha)METHYLATION
<220>
   <221> VARIANT
   <222> (5)..(5)
   <223> Xaa can be any naturally occurring amino acid
<220>
   <221> VARIANT
   <222> (7)..(8)
   <223> Xaa can be any naturally occurring amino acid
<220>
   <221> MOD_RES
   <222> (9)..(9)
   <223> Pen
<220>
   <221> VARIANT
   <222> (10)..(10)
   <223> Xaa can be any naturally occurring amino acid
<220>
   <221> MOD_RES
   <222> (10)..(10)
   <223> ACETYLATION
<220>
   <221> MOD_RES
   <222> (10)..(10)
   <223> N(alpha)METHYLATION
<220>
   <221> MOD_RES
   <222> (10)..(10)
   <223> D-Lys, Dap, Dab, Orn, D-Orn, D-Dab, D-Dap
<400> 32
   Xaa Xaa Cys Arg Xaa Asp Xaa Xaa Xaa Xaa
   1               5                   10
<210> 33
   <211> 10
   <212> PRT
   <213> Unknown
<220>
   <223> Synthetic Construct
<220>
   <221> VARIANT
   <222> (1)..(2)
   <223> Xaa can be any naturally occurring amino acid
<220>
   <221> MOD_RES
   <222> (1)..(1)
   <223> ACETYLATION
<220>
   <221> MOD_RES
   <222> (3)..(3)
   <223> Pen
<220>
   <221> DISULFID
   <222> (3)..(9)
   <223> Disulfide bond between Xaa at position 3 and Cys at position 9
<220>
   <221> MOD_RES
   <222> (4)..(4)
   <223> N(alpha)METHYLATION
<220>
   <221> VARIANT
   <222> (5)..(5)
   <223> Xaa can be any naturally occurring amino acid
<220>
   <221> VARIANT
   <222> (7)..(8)
   <223> Xaa can be any naturally occurring amino acid
<220>
   <221> VARIANT
   <222> (10)..(10)
   <223> Xaa can be any naturally occurring amino acid
<220>
   <221> MOD_RES
   <222> (10)..(10)
   <223> ACETYLATION
<220>
   <221> MOD_RES
   <222> (10)..(10)
   <223> N(alpha)METHYLATION
<220>
   <221> MOD_RES
   <222> (10)..(10)
   <223> D-Lys, Dap, Dab, Orn, D-Orn, D-Dab, D-Dap
<400> 33
   Xaa Xaa Xaa Arg Xaa Asp Xaa Xaa Cys Xaa
   1               5                   10
<210> 34
   <211> 10
   <212> PRT
   <213> Unknown
<220>
   <223> Synthetic Construct
<220>
   <221> VARIANT
   <222> (1)..(2)
   <223> Xaa can be any naturally occurring amino acid
<220>
   <221> MOD_RES
   <222> (1)..(1)
   <223> ACETYLATION
<220>
   <221> MOD_RES
   <222> (3)..(3)
   <223> Pen
<220>
   <221> DISULFID
   <222> (3)..(9)
   <223> Disulfide bond between Xaa at position 3 and Xaa at position 9
<220>
   <221> MOD_RES
   <222> (4)..(4)
   <223> N(alpha)METHYLATION
<220>
   <221> VARIANT
   <222> (5)..(5)
   <223> Xaa can be any naturally occurring amino acid
<220>
   <221> VARIANT
   <222> (7)..(8)
   <223> Xaa can be any naturally occurring amino acid
<220>
   <221> MOD_RES
   <222> (9)..(9)
   <223> Pen
<220>
   <221> VARIANT
   <222> (10)..(10)
   <223> Xaa can be any naturally occurring amino acid
<220>
   <221> MOD_RES
   <222> (10)..(10)
   <223> ACETYLATION
<220>
   <221> MOD_RES
   <222> (10)..(10)
   <223> N(alpha)METHYLATION
<220>
   <221> MOD_RES
   <222> (10)..(10)
   <223> D-Lys, Dap, Dab, Orn, D-Orn, D-Dab, D-Dap
<400> 34
   Xaa Xaa Xaa Arg Xaa Asp Xaa Xaa Xaa Xaa
   1               5                   10
<210> 35
   <211> 11
   <212> PRT
   <213> Unknown
<220>
   <223> Synthetic Construct
<220>
   <221> VARIANT
   <222> (1)..(3)
   <223> Xaa can be any naturally occurring amino acid
<220>
   <221> MOD_RES
   <222> (1)..(1)
   <223> ACETYLATION
<220>
   <221> DISULFID
   <222> (4)..(10)
   <223> Disulfide bond between Cys at position 4 and Cys at position 10
<220>
   <221> MOD_RES
   <222> (5)..(5)
   <223> N(alpha)METHYLATION
<220>
   <221> VARIANT
   <222> (6)..(6)
   <223> Xaa can be any naturally occurring amino acid
<220>
   <221> VARIANT
   <222> (8)..(9)
   <223> Xaa can be any naturally occurring amino acid
<220>
   <221> VARIANT
   <222> (11)..(11)
   <223> Xaa can be any naturally occurring amino acid
<220>
   <221> MOD_RES
   <222> (11)..(11)
   <223> N(alpha)METHYLATION
<220>
   <221> MOD_RES
   <222> (11)..(11)
   <223> D-Lys, Dap, Dab, Orn, D-Orn, D-Dab, D-Dap
<220>
   <221> MOD_RES
   <222> (11)..(11)
   <223> ACETYLATION
<400> 35
   Xaa Xaa Xaa Cys Arg Xaa Asp Xaa Xaa Cys Xaa
   1               5                   10
<210> 36
   <211> 11
   <212> PRT
   <213> Unknown
<220>
   <223> Synthetic Construct
<220>
   <221> VARIANT
   <222> (1)..(3)
   <223> Xaa can be any naturally occurring amino acid
<220>
   <221> MOD_RES
   <222> (1)..(1)
   <223> ACETYLATION
<220>
   <221> DISULFID
   <222> (4)..(10)
   <223> Disulfide bond between Cys at position 4 and Xaa at position 10
<220>
   <221> MOD_RES
   <222> (5)..(5)
   <223> N(alpha)METHYLATION
<220>
   <221> VARIANT
   <222> (6)..(6)
   <223> Xaa can be any naturally occurring amino acid
<220>
   <221> VARIANT
   <222> (8)..(9)
   <223> Xaa can be any naturally occurring amino acid
<220>
   <221> MOD_RES
   <222> (10)..(10)
   <223> Pen
<220>
   <221> VARIANT
   <222> (11)..(11)
   <223> Xaa can be any naturally occurring amino acid
<220>
   <221> MOD_RES
   <222> (11)..(11)
   <223> ACETYLATION
<220>
   <221> MOD_RES
   <222> (11)..(11)
   <223> N(alpha)METHYLATION
<220>
   <221> MOD_RES
   <222> (11)..(11)
   <223> D-Lys, Dap, Dab, Orn, D-Orn, D-Dab, D-Dap
<400> 36
   Xaa Xaa Xaa Cys Arg Xaa Asp Xaa Xaa Xaa Xaa
   1               5                   10
<210> 37
   <211> 11
   <212> PRT
   <213> Unknown
<220>
   <223> Synthetic Construct
<220>
   <221> VARIANT
   <222> (1)..(3)
   <223> Xaa can be any naturally occurring amino acid
<220>
   <221> MOD_RES
   <222> (1)..(1)
   <223> ACETYLATION
<220>
   <221> MOD_RES
   <222> (4)..(4)
   <223> Pen
<220>
   <221> DISULFID
   <222> (4)..(10)
   <223> Disulfide bond between Xaa at position 4 and Cys at position 10
<220>
   <221> MOD_RES
   <222> (5)..(5)
   <223> N(alpha)METHYLATION
<220>
   <221> VARIANT
   <222> (6)..(6)
   <223> Xaa can be any naturally occurring amino acid
<220>
   <221> VARIANT
   <222> (8)..(9)
   <223> Xaa can be any naturally occurring amino acid
<220>
   <221> VARIANT
   <222> (11)..(11)
   <223> Xaa can be any naturally occurring amino acid
<220>
   <221> MOD_RES
   <222> (11)..(11)
   <223> ACETYLATION
<220>
   <221> MOD_RES
   <222> (11)..(11)
   <223> N(alpha)METHYLATION
<220>
   <221> MOD_RES
   <222> (11)..(11)
   <223> D-Lys, Dap, Dab, Orn, D-Orn, D-Dab, D-Dap
<400> 37
   Xaa Xaa Xaa Xaa Arg Xaa Asp Xaa Xaa Cys Xaa
   1               5                   10
<210> 38
   <211> 11
   <212> PRT
   <213> Unknown
<220>
   <223> Synthetic Construct
<220>
   <221> VARIANT
   <222> (1)..(3)
   <223> Xaa can be any naturally occurring amino acid
<220>
   <221> MOD_RES
   <222> (1)..(1)
   <223> ACETYLATION
<220>
   <221> MOD_RES
   <222> (4)..(4)
   <223> Pen
<220>
   <221> DISULFID
   <222> (4)..(10)
   <223> Disulfide bond between Xaa at position 4 and Xaa at position 10
<220>
   <221> MOD_RES
   <222> (5)..(5)
   <223> N(alpha)METHYLATION
<220>
   <221> VARIANT
   <222> (6)..(6)
   <223> Xaa can be any naturally occurring amino acid
<220>
   <221> VARIANT
   <222> (8)..(9)
   <223> Xaa can be any naturally occurring amino acid
<220>
   <221> MOD_RES
   <222> (10)..(10)
   <223> Pen
<220>
   <221> MOD_RES
   <222> (11)..(11)
   <223> ACETYLATION
<220>
   <221> VARIANT
   <222> (11)..(11)
   <223> Xaa can be any naturally occurring amino acid
<220>
   <221> MOD_RES
   <222> (11)..(11)
   <223> N(alpha)METHYLATION
<220>
   <221> MOD_RES
   <222> (11)..(11)
   <223> D-Lys, Dap, Dab, Orn, D-Orn, D-Dab, D-Dap
<400> 38
   Xaa Xaa Xaa Xaa Arg Xaa Asp Xaa Xaa Xaa Xaa
   1               5                   10
<210> 39
   <211> 8
   <212> PRT
   <213> Unknown
<220>
   <223> Synthetic Construct
<220>
   <221> MOD_RES
   <222> (1)..(1)
   <223> ACETYLATION
<220>
   <221> DISULFID
   <222> (1)..(7)
   <223> Disulfide bond between Cys at position 1 and Cys at position 7
<220>
   <221> VARIANT
   <222> (2)..(2)
   <223> Arg, Tyr
<400> 39
   Cys Xaa Ser Asp Thr Leu Cys Lys
   1               5
<210> 40
   <211> 10
   <212> PRT
   <213> Unknown
<220>
   <223> Synthetic Construct
<220>
   <221> MOD_RES
   <222> (1)..(1)
   <223> ACETYLATION
<220>
   <221> DISULFID
   <222> (1)..(7)
   <223> Disulfide bond between Cys at position 1 and Cys at position 7
<400> 40
   Cys Arg Ser Asp Thr Leu Cys Gly Glu Lys
   1               5                   10
<210> 41
   <211> 8
   <212> PRT
   <213> Unknown
<220>
   <223> Synthetic Construct
<220>
   <221> MOD_RES
   <222> (1)..(1)
   <223> ACETYLATION
<220>
   <221> DISULFID
   <222> (2)..(8)
   <223> Disulfide bond between Cys at position 2 and Cys at position 8
<400> 41
   Lys Cys Arg Ser Asp Thr Leu Cys
   1               5
<210> 42
   <211> 10
   <212> PRT
   <213> Unknown
<220>
   <223> Synthetic Construct
<220>
   <221> MOD_RES
   <222> (1)..(1)
   <223> ACETYLATION
<220>
   <221> DISULFID
   <222> (2)..(8)
   <223> Disulfide bond between Cys at position 2 and Cys at position 8
<400> 42
   Lys Cys Arg Ser Asp Thr Leu Cys Gly Glu
   1               5                   10
<210> 43
   <211> 9
   <212> PRT
   <213> Unknown
<220>
   <223> Synthetic Construct
<220>
   <221> MOD_RES
   <222> (1)..(1)
   <223> ACETYLATION
<220>
   <221> DISULFID
   <222> (1)..(7)
   <223> Disulfide bond between Cys at position 1 and Cys at position 7
<220>
   <221> MOD_RES
   <222> (2)..(2)
   <223> METHYLATION: Arg-Me-sym; Arg-Me-asym
<400> 43
   Cys Arg Ser Asp Thr Leu Cys Gly Glu
   1               5
<210> 44
   <211> 9
   <212> PRT
   <213> Unknown
<220>
   <223> Synthetic Construct
<220>
   <221> MOD_RES
   <222> (1)..(1)
   <223> ACETYLATION
<220>
   <221> DISULFID
   <222> (1)..(7)
   <223> Disulfide bond between Cys at position 1 and Cys at position 7
<220>
   <221> MOD_RES
   <222> (2)..(2)
   <223> Dab, Cit, Cav, Dap
<400> 44
   Cys Xaa Ser Asp Thr Leu Cys Gly Glu
   1               5
<210> 45
   <211> 9
   <212> PRT
   <213> Unknown
<220>
   <223> Synthetic Construct
<220>
   <221> MOD_RES
   <222> (1)..(1)
   <223> ACETYLATION
<220>
   <221> DISULFID
   <222> (1)..(7)
   <223> Disulfide bond between Cys at position 1 and Cys at position 7
<400> 45
   Cys His Ser Asp Thr Leu Cys Gly Glu
   1               5
<210> 46
   <211> 7
   <212> PRT
   <213> Unknown
<220>
   <223> Synthetic Construct
<220>
   <221> DISULFID
   <222> (1)..(7)
   <223> Disulfide bond between Cys at position 1 and Cys at position 7
<220>
   <221> MOD_RES
   <222> (2)..(2)
   <223> N(alpha)METHYLATION
<400> 46
   Cys Arg Ser Asp Thr Leu Cys
   1               5
<210> 47
   <211> 8
   <212> PRT
   <213> Unknown
<220>
   <223> Synthetic Construct
<220>
   <221> MOD_RES
   <222> (1)..(1)
   <223> ACETYLATION
<220>
   <221> DISULFID
   <222> (1)..(7)
   <223> Disulfide bond between Cys at position 1 and Cys at position 7
<220>
   <221> MOD_RES
   <222> (2)..(2)
   <223> N(alpha)METHYLATION, Arg-Me-sym, Arg-Me-asym
<220>
   <221> MOD_RES
   <222> (8)..(8)
   <223> Dap, Dab, D-Lys
<220>
   <221> MOD_RES
   <222> (8)..(8)
   <223> ACETYLATION
<400> 47
   Cys Arg Ser Asp Thr Leu Cys Xaa
   1 5
<210> 48
   <211> 8
   <212> PRT
   <213> Unknown
<220>
   <223> Synthetic Construct
<220>
   <221> MOD_RES
   <222> (1)..(1)
   <223> ACETYLATION
<220>
   <221> DISULFID
   <222> (1)..(7)
   <223> Disulfide bond between Cys at position 1 and Cys at position 7
<220>
   <221> MOD_RES
   <222> (2)..(2)
   <223> N(alpha)METHYLATION
<220>
   <221> MOD_RES
   <222> (8)..(8)
   <223> N(alpha)METHYLATION
<400> 48
   Cys Arg Ser Asp Thr Leu Cys Lys
   1 5
<210> 49
   <211> 8
   <212> PRT
   <213> Unknown
<220>
   <223> Synthetic Construct
<220>
   <221> DISULFID
   <222> (1)..(7)
   <223> Disulfide bond between Cys at position 1 and Cys at position 7
<220>
   <221> MOD_RES
   <222> (2)..(2)
   <223> N(alpha)METHYLATION
<220>
   <221> MOD_RES
   <222> (2)..(2)
   <223> Guan, 4-Guan, Dap, Dab, Phe(4-NH2)
<220>
   <221> MOD_RES
   <222> (8)..(8)
   <223> Dap, Dab, D-Lys
<220>
   <221> MOD_RES
   <222> (8)..(8)
   <223> ACETYLATION
<400> 49
   Cys Xaa Ser Asp Thr Leu Cys Xaa
   1               5
<210> 50
   <211> 7
   <212> PRT
   <213> Unknown
<220>
   <223> Synthetic Construct
<220>
   <221> DISULFID
   <222> (1)..(7)
   <223> Disulfide bond between Cys at position 1 and Cys at position 7
<220>
   <221> MOD_RES
   <222> (2)..(2)
   <223> N(alpha)METHYLATION
<400> 50
   Cys Arg Ser Asp Thr Leu Cys
   1               5
<210> 51
   <211> 10
   <212> PRT
   <213> Unknown
<220>
   <223> Synthetic Construct
<220>
   <221> MOD_RES
   <222> (1)..(1)
   <223> ACETYLATION
<220>
   <221> DISULFID
   <222> (1)..(7)
   <223> Disulfide bond between Cys at position 1 and Cys at position 7
<220>
   <221> MOD_RES
   <222> (2)..(2)
   <223> N(alpha)METHYLATION
<220>
   <221> MOD_RES
   <222> (2)..(2)
   <223> 4-Guan
<220>
   <221> VARIANT
   <222> (2)..(2)
   <223> Arg
<220>
   <221> MOD_RES
   <222> (10)..(10)
   <223> N(alpha)METHYLATION
<400> 51
   Cys Xaa Ser Asp Thr Leu Cys Gly Glu Lys
   1               5                   10
<210> 52
   <211> 9
   <212> PRT
   <213> Unknown
<220>
   <223> Synthetic Construct
<220>
   <221> MOD_RES
   <222> (1)..(1)
   <223> ACETYLATION
<220>
   <221> DISULFID
   <222> (1)..(7)
   <223> Disulfide bond between Cys at position 1 and Cys at position 7
<220>
   <221> MOD_RES
   <222> (5)..(6)
   <223> N(alpha)METHYLATION
<400> 52
   Cys His Ser Asp Thr Leu Cys Gly Glu
   1               5
<210> 53
   <211> 10
   <212> PRT
   <213> Unknown
<220>
   <223> Synthetic Construct
<220>
   <221> MOD_RES
   <222> (1)..(1)
   <223> ACETYLATION
<220>
   <221> DISULFID
   <222> (1)..(7)
   <223> Disulfide bond between Cys at position 1 and Cys at position 7
<220>
   <221> MOD_RES
   <222> (8)..(8)
   <223> Sar
<400> 53
   Cys Arg Ser Asp Thr Leu Cys Xaa Glu Lys
   1               5                   10
<210> 54
   <211> 10
   <212> PRT
   <213> Unknown
<220>
   <223> Synthetic Construct
<220>
   <221> MOD_RES
   <222> (1)..(1)
   <223> ACETYLATION
<220>
   <221> DISULFID
   <222> (1)..(7)
   <223> Disulfide bond between Cys at position 1 and Cys at position 7
<220>
   <221> MOD_RES
   <222> (2)..(2)
   <223> N(alpha)METHYLATION
<220>
   <221> MOD_RES
   <222> (10)..(10)
   <223> D-Lys, Dab, Dab (2-Me-trifluorobutyl), Dab (trifluorpentyl), Dab (Acetyl), Dab (Octonyl)
<400> 54
   Cys Arg Ser Asp Thr Leu Cys Gly Glu Xaa
   1 5 10
<210> 55
   <211> 8
   <212> PRT
   <213> Unknown
<220>
   <223> Synthetic Construct
<220>
   <221> MOD_RES
   <222> (1)..(1)
   <223> ACETYLATION
<220>
   <221> DISULFID
   <222> (1)..(7)
   <223> Disulfide bond between Cys at position 1 and Xaa at position 7
<220>
   <221> MOD_RES
   <222> (2)..(2)
   <223> N(alpha)METHYLATION
<220>
   <221> MOD_RES
   <222> (7)..(7)
   <223> Pen
<220>
   <221> MOD_RES
   <222> (8)..(8)
   <223> D-Lys
<400> 55
   Cys Arg Ser Asp Thr Leu Xaa Xaa
   1 5
<210> 56
   <211> 8
   <212> PRT
   <213> Unknown
<220>
   <223> Synthetic Construct
<220>
   <221> MOD_RES
   <222> (1)..(1)
   <223> ACETYLATION
<220>
   <221> MOD_RES
   <222> (1)..(1)
   <223> D-Lys
<220>
   <221> DISULFID
   <222> (2)..(8)
   <223> Disulfide bond between Cys at position 2 and Xaa at position 8
<220>
   <221> MOD_RES
   <222> (3)..(3)
   <223> N(alpha)METHYLATION
<220>
   <221> MOD_RES
   <222> (8)..(8)
   <223> Pen
<400> 56
   Xaa Cys Arg Ser Asp Thr Leu Xaa
   1               5
<210> 57
   <211> 9
   <212> PRT
   <213> Unknown
<220>
   <223> Synthetic Construct
<220>
   <221> MOD_RES
   <222> (1)..(1)
   <223> ACETYLATION
<220>
   <221> DISULFID
   <222> (1)..(7)
   <223> Disulfide bond between Cys at position 1 and Xaa at position 7
<220>
   <221> MOD_RES
   <222> (2)..(2)
   <223> N(alpha)METHYLATION
<220>
   <221> MOD_RES
   <222> (7)..(7)
   <223> Pen
<220>
   <221> MOD_RES
   <222> (9)..(9)
   <223> D-Lys
<400> 57
   Cys Arg Ser Asp Thr Leu Xaa Trp Xaa
   1               5
<210> 58
   <211> 9
   <212> PRT
   <213> Unknown
<220>
   <223> Synthetic Construct
<220>
   <221> MOD_RES
   <222> (1)..(1)
   <223> ACETYLATION
<220>
   <221> DISULFID
   <222> (1)..(7)
   <223> Disulfide bond between Cys at position 1 and Xaa at position 7
<220>
   <221> MOD_RES
   <222> (2)..(2)
   <223> N(alpha)METHYLATION
<220>
   <221> MOD_RES
   <222> (7)..(7)
   <223> Pen
<220>
   <221> MOD_RES
   <222> (9)..(9)
   <223> D-Lys
<400> 58
   Cys Arg Ser Asp Thr Leu Xaa Tyr Xaa
   1               5
<210> 59
   <211> 8
   <212> PRT
   <213> Unknown
<220>
   <223> Synthetic Construct
<220>
   <221> DISULFID
   <222> (2)..(8)
   <223> Disulfide bond between Cys at position 2 and Xaa at position 8
<220>
   <221> MOD_RES
   <222> (3)..(3)
   <223> N(alpha)METHYLATION
<220>
   <221> MOD_RES
   <222> (8)..(8)
   <223> Pen
<400> 59
   Arg Cys Arg Ser Asp Thr Leu Xaa
   1               5
<210> 60
   <211> 8
   <212> PRT
   <213> Unknown
<220>
   <223> Synthetic Construct
<220>
   <221> DISULFID
   <222> (2)..(8)
   <223> Disulfide bond between Cys at position 2 and Xaa at position 8
<220>
   <221> MOD_RES
   <222> (3)..(3)
   <223> N(alpha)METHYLATION
<220>
   <221> MOD_RES
   <222> (8)..(8)
   <223> Pen
<400> 60
   Leu Cys Arg Ser Asp Thr Leu Xaa
   1               5
<210> 61
   <211> 9
   <212> PRT
   <213> Unknown
<220>
   <223> Synthetic Construct
<220>
   <221> MOD_RES
   <222> (1)..(1)
   <223> ACETYLATION
<220>
   <221> DISULFID
   <222> (3)..(9)
   <223> Disulfide bond between Cys at position 3 and Xaa at position 9
<220>
   <221> MOD_RES
   <222> (4)..(4)
   <223> N(alpha)METHYLATION
<220>
   <221> MOD_RES
   <222> (9)..(9)
   <223> Pen
<400> 61
   Lys Leu Cys Arg Ser Asp Thr Leu Xaa
   1               5
<210> 62
   <211> 8
   <212> PRT
   <213> Unknown
<220>
   <223> Synthetic Construct
<220>
   <221> DISULFID
   <222> (2)..(8)
   <223> Disulfide bond between Cys at position 2 and Xaa at position 8
<220>
   <221> MOD_RES
   <222> (3)..(3)
   <223> N(alpha)METHYLATION
<220>
   <221> MOD_RES
   <222> (8)..(8)
   <223> Pen
<400> 62
   His Cys Arg Ser Asp Thr Leu Xaa
   1               5
<210> 63
   <211> 9
   <212> PRT
   <213> Unknown
<220>
   <223> Synthetic Construct
<220>
   <221> MOD_RES
   <222> (1)..(1)
   <223> ACETYLATION
<220>
   <221> DISULFID
   <222> (3)..(9)
   <223> Disulfide bond between Cys at position 3 and Xaa at position 9
<220>
   <221> MOD_RES
   <222> (4)..(4)
   <223> N(alpha)METHYLATION
<220>
   <221> MOD_RES
   <222> (9)..(9)
   <223> Pen
<400> 63
   Lys His Cys Arg Ser Asp Thr Leu Xaa
   1               5
<210> 64
   <211> 8
   <212> PRT
   <213> Unknown
<220>
   <223> Synthetic Construct
<220>
   <221> DISULFID
   <222> (2)..(8)
   <223> Disulfide bond between Cys at position 3 and Xaa at position 9
<220>
   <221> MOD_RES
   <222> (3)..(3)
   <223> N(alpha)METHYLATION
<220>
   <221> MOD_RES
   <222> (8)..(8)
   <223> Pen
<400> 64
   Glu Cys Arg Ser Asp Thr Leu Xaa
   1               5
<210> 65
   <211> 8
   <212> PRT
   <213> Unknown
<220>
   <223> Synthetic Construct
<220>
   <221> MOD_RES
   <222> (1)..(1)
   <223> ACETYLATION
<220>
   <221> DISULFID
   <222> (3)..(8)
   <223> Disulfide bond between Cys at position 3 and Xaa at position 8
<220>
   <221> MOD_RES
   <222> (4)..(4)
   <223> N(alpha)METHYLATION
<220>
   <221> MOD_RES
   <222> (8)..(8)
   <223> Pen
<400> 65
   Lys Glu Cys Arg Ser Asp Thr Xaa
   1               5
<210> 66
   <211> 9
   <212> PRT
   <213> Unknown
<220>
   <223> Synthetic Construct
<220>
   <221> MOD_RES
   <222> (1)..(1)
   <223> ACETYLATION
<220>
   <221> DISULFID
   <222> (3)..(9)
   <223> Disulfide bond between Cys at position 3 and Xaa at position 9
<220>
   <221> MOD_RES
   <222> (4)..(4)
   <223> N(alpha)METHYLATION
<220>
   <221> MOD_RES
   <222> (9)..(9)
   <223> Pen
<400> 66
   Lys Trp Cys Arg Ser Asp Thr Leu Xaa
   1 5
<210> 67
   <211> 8
   <212> PRT
   <213> Unknown
<220>
   <223> Synthetic Construct
<220>
   <221> MOD_RES
   <222> (1)..(1)
   <223> ACETYLATION
<220>
   <221> DISULFID
   <222> (2)..(8)
   <223> Disulfide bond between Cys at position 2 and Xaa at position 8
<220>
   <221> MOD_RES
   <222> (3)..(3)
   <223> N(alpha)METHYLATION
<220>
   <221> MOD_RES
   <222> (8)..(8)
   <223> Pen
<400> 67
   Pro Cys Arg Ser Asp Thr Leu Xaa
   1 5
<210> 68
   <211> 9
   <212> PRT
   <213> Unknown
<220>
   <223> Synthetic Construct
<220>
   <221> MOD_RES
   <222> (1)..(1)
   <223> ACETYLATION
<220>
   <221> DISULFID
   <222> (3)..(9)
   <223> Disulfide bond between Cys at position 3 and Xaa at position 9
<220>
   <221> MOD_RES
   <222> (4)..(4)
   <223> N(alpha)METHYLATION
<220>
   <221> MOD_RES
   <222> (9)..(9)
   <223> Pen
<400> 68
   Lys Pro Cys Arg Ser Asp Thr Leu Xaa
   1               5
<210> 69
   <211> 9
   <212> PRT
   <213> Unknown
<220>
   <223> Synthetic Construct
<220>
   <221> MOD_RES
   <222> (1)..(1)
   <223> ACETYLATION
<220>
   <221> DISULFID
   <222> (3)..(9)
   <223> Disulfide bond between Cys at position 3 and Xaa at position 9
<220>
   <221> MOD_RES
   <222> (4)..(4)
   <223> N(alpha)METHYLATION
<220>
   <221> MOD_RES
   <222> (9)..(9)
   <223> Pen
<400> 69
   Lys Ser Cys Arg Ser Asp Thr Leu Xaa
   1               5
<210> 70
   <211> 9
   <212> PRT
   <213> Unknown
<220>
   <223> Synthetic Construct
<220>
   <221> MOD_RES
   <222> (1)..(1)
   <223> ACETYLATION
<220>
   <221> DISULFID
   <222> (3)..(9)
   <223> Disulfide bond between Cys at position 3 and Xaa at position 9
<220>
   <221> MOD_RES
   <222> (4)..(4)
   <223> N(alpha)METHYLATION
<220>
   <221> MOD_RES
   <222> (9)..(9)
   <223> Pen
<400> 70
   Lys Asn Cys Arg Ser Asp Thr Leu Xaa
   1               5
<210> 71
   <211> 9
   <212> PRT
   <213> Unknown
<220>
   <223> Synthetic Construct
<220>
   <221> MOD_RES
   <222> (1)..(1)
   <223> ACETYLATION
<220>
   <221> DISULFID
   <222> (3)..(9)
   <223> Disulfide bond between Cys at position 3 and Xaa at position 9
<220>
   <221> MOD_RES
   <222> (4)..(4)
   <223> N(alpha)METHYLATION
<220>
   <221> MOD_RES
   <222> (9)..(9)
   <223> Pen
<400> 71
   Lys Tyr Cys Arg Ser Asp Thr Leu Xaa
   1               5
<210> 72
   <211> 9
   <212> PRT
   <213> Unknown
<220>
   <223> Synthetic Construct
<220>
   <221> MOD_RES
   <222> (1)..(1)
   <223> ACETYLATION
<220>
   <221> DISULFID
   <222> (1)..(7)
   <223> Disulfide bond between Cys at position 1 and Xaa at position 7
<220>
   <221> MOD_RES
   <222> (2)..(2)
   <223> N(alpha)METHYLATION
<220>
   <221> MOD_RES
   <222> (7)..(7)
   <223> Pen
<220>
   <221> MOD_RES
   <222> (9)..(9)
   <223> D-Lys
<400> 72
   Cys Arg Ser Asp Thr Leu Xaa Leu Xaa
   1               5
<210> 73
   <211> 9
   <212> PRT
   <213> Unknown
<220>
   <223> Synthetic Construct
<220>
   <221> MOD_RES
   <222> (1)..(1)
   <223> ACETYLATION
<220>
   <221> DISULFID
   <222> (1)..(7)
   <223> Disulfide bond between Cys at position 1 and Xaa at position 7
<220>
   <221> MOD_RES
   <222> (2)..(2)
   <223> N(alpha)METHYLATION
<220>
   <221> MOD_RES
   <222> (7)..(7)
   <223> Pen
<220>
   <221> MOD_RES
   <222> (9)..(9)
   <223> D-Lys
<400> 73
   Cys Arg Ser Asp Thr Leu Xaa His Xaa
   1               5
<210> 74
   <211> 9
   <212> PRT
   <213> Unknown
<220>
   <223> Synthetic Construct
<220>
   <221> MOD_RES
   <222> (1)..(1)
   <223> ACETYLATION
<220>
   <221> DISULFID
   <222> (1)..(7)
   <223> Disulfide bond between Cys at position 1 and Xaa at position 7
<220>
   <221> MOD_RES
   <222> (2)..(2)
   <223> N(alpha)METHYLATION
<220>
   <221> MOD_RES
   <222> (7)..(7)
   <223> Pen
<220>
   <221> MOD_RES
   <222> (9)..(9)
   <223> D-Lys
<400> 74
   Cys Arg Ser Asp Thr Leu Xaa Glu Xaa
   1               5
<210> 75
   <211> 9
   <212> PRT
   <213> Unknown
<220>
   <223> Synthetic Construct
<220>
   <221> MOD_RES
   <222> (1)..(1)
   <223> ACETYLATION
<220>
   <221> DISULFID
   <222> (1)..(7)
   <223> Disulfide bond between Cys at position 1 and Xaa at position 7
<220>
   <221> MOD_RES
   <222> (2)..(2)
   <223> N(alpha)METHYLATION
<220>
   <221> MOD_RES
   <222> (7)..(7)
   <223> Pen
<400> 75
   Cys Arg Ser Asp Thr Leu Xaa Tyr Lys
   1               5
<210> 76
   <211> 9
   <212> PRT
   <213> Unknown
<220>
   <223> Synthetic Construct
<220>
   <221> MOD_RES
   <222> (1)..(1)
   <223> ACETYLATION
<220>
   <221> DISULFID
   <222> (1)..(7)
   <223> Disulfide bond between Cys at position 1 and Xaa at position 7
<220>
   <221> MOD_RES
   <222> (2)..(2)
   <223> N(alpha)METHYLATION
<220>
   <221> MOD_RES
   <222> (7)..(7)
   <223> Pen
<400> 76
   Cys Arg Ser Asp Thr Leu Xaa Trp Lys
   1               5
<210> 77
   <211> 9
   <212> PRT
   <213> Unknown
<220>
   <223> Synthetic Construct
<220>
   <221> MOD_RES
   <222> (1)..(1)
   <223> ACETYLATION
<220>
   <221> DISULFID
   <222> (1)..(7)
   <223> Disulfide bond between Cys at position 1 and Xaa at position 7
<220>
   <221> MOD_RES
   <222> (2)..(2)
   <223> N(alpha)METHYLATION
<220>
   <221> MOD_RES
   <222> (7)..(7)
   <223> Pen
<220>
   <221> MOD_RES
   <222> (9)..(9)
   <223> D-Lys
<400> 77
   Cys Arg Ser Asp Thr Leu Xaa Arg Xaa
   1               5
<210> 78
   <211> 9
   <212> PRT
   <213> Unknown
<220>
   <223> Synthetic Construct
<220>
   <221> MOD_RES
   <222> (1)..(1)
   <223> ACETYLATION
<220>
   <221> DISULFID
   <222> (1)..(7)
   <223> Disulfide bond between Cys at position 1 and Xaa at position 7
<220>
   <221> MOD_RES
   <222> (2)..(2)
   <223> N(alpha)METHYLATION
<220>
   <221> MOD_RES
   <222> (7)..(7)
   <223> Pen
<220>
   <221> MOD_RES
   <222> (9)..(9)
   <223> D-Lys
<400> 78
   Cys Arg Ser Asp Thr Leu Xaa Pro Xaa
   1               5
<210> 79
   <211> 9
   <212> PRT
   <213> Unknown
<220>
   <223> Synthetic Construct
<220>
   <221> MOD_RES
   <222> (1)..(1)
   <223> ACETYLATION
<220>
   <221> DISULFID
   <222> (1)..(7)
   <223> Disulfide bond between Cys at position 1 and Xaa at position 7
<220>
   <221> MOD_RES
   <222> (2)..(2)
   <223> N(alpha)METHYLATION
<220>
   <221> MOD_RES
   <222> (7)..(7)
   <223> Pen
<220>
   <221> MOD_RES
   <222> (9)..(9)
   <223> D-Lys
<400> 79
   Cys Arg Ser Asp Thr Leu Xaa Ser Xaa
   1               5
<210> 80
   <211> 9
   <212> PRT
   <213> Unknown
<220>
   <223> Synthetic Construct
<220>
   <221> MOD_RES
   <222> (1)..(1)
   <223> ACETYLATION
<220>
   <221> DISULFID
   <222> (1)..(7)
   <223> Disulfide bond between Cys at position 1 and Xaa at position 7
<220>
   <221> MOD_RES
   <222> (2)..(2)
   <223> N(alpha)METHYLATION
<220>
   <221> MOD_RES
   <222> (7)..(7)
   <223> Pen
<220>
   <221> MOD_RES
   <222> (9)..(9)
   <223> D-Lys
<400> 80
   Cys Arg Ser Asp Thr Leu Xaa Asn Xaa
   1               5
<210> 81
   <211> 10
   <212> PRT
   <213> Unknown
<220>
   <223> Synthetic Construct
<220>
   <221> MOD_RES
   <222> (1)..(1)
   <223> ACETYLATION
<220>
   <221> DISULFID
   <222> (2)..(8)
   <223> Disulfide bond between Cys at position 2 and Xaa at position 8
<220>
   <221> MOD_RES
   <222> (3)..(3)
   <223> N(alpha)METHYLATION
<220>
   <221> MOD_RES
   <222> (8)..(8)
   <223> Pen
<220>
   <221> MOD_RES
   <222> (9)..(9)
   <223> 1-Nal
<400> 81
   Arg Cys Arg Ser Asp Thr Leu Xaa Xaa Lys
   1               5                   10
<210> 82
   <211> 10
   <212> PRT
   <213> Unknown
<220>
   <223> Synthetic Construct
<220>
   <221> MOD_RES
   <222> (1)..(1)
   <223> ACETYLATION
<220>
   <221> DISULFID
   <222> (2)..(8)
   <223> Disulfide bond between Cys at position 2 and Xaa at position 8
<220>
   <221> MOD_RES
   <222> (3)..(3)
   <223> N(alpha)METHYLATION
<220>
   <221> MOD_RES
   <222> (8)..(8)
   <223> Pen
<220>
   <221> MOD_RES
   <222> (9)..(9)
   <223> 1-Nal
<400> 82
   Glu Cys Arg Ser Asp Thr Leu Xaa Xaa Lys
   1               5                   10
<210> 83
   <211> 10
   <212> PRT
   <213> Unknown
<220>
   <223> Synthetic Construct
<220>
   <221> MOD_RES
   <222> (1)..(1)
   <223> ACETYLATION
<220>
   <221> DISULFID
   <222> (2)..(8)
   <223> Disulfide bond between Cys at position 2 and Xaa at position 8
<220>
   <221> MOD_RES
   <222> (3)..(3)
   <223> N(alpha)METHYLATION
<220>
   <221> MOD_RES
   <222> (8)..(8)
   <223> Pen
<400> 83
   Arg Cys Arg Ser Asp Thr Leu Xaa Trp Lys
   1               5                   10
<210> 84
   <211> 10
   <212> PRT
   <213> Unknown
<220>
   <223> Synthetic Construct
<220>
   <221> MOD_RES
   <222> (1)..(1)
   <223> ACETYLATION
<220>
   <221> DISULFID
   <222> (2)..(8)
   <223> Disulfide bond between Cys at position 2 and Xaa at position 8
<220>
   <221> MOD_RES
   <222> (3)..(3)
   <223> N(alpha)METHYLATION
<220>
   <221> MOD_RES
   <222> (8)..(8)
   <223> Pen
<400> 84
   Glu Cys Arg Ser Asp Thr Leu Xaa Trp Lys
   1               5                   10
<210> 85
   <211> 10
   <212> PRT
   <213> Unknown
<220>
   <223> Synthetic Construct
<220>
   <221> MOD_RES
   <222> (1)..(1)
   <223> ACETYLATION
<220>
   <221> DISULFID
   <222> (2)..(8)
   <223> Disulfide bond between Cys at position 2 and Xaa at position 8
<220>
   <221> MOD_RES
   <222> (3)..(3)
   <223> N(alpha)METHYLATION
<220>
   <221> MOD_RES
   <222> (8)..(8)
   <223> Pen
<400> 85
   Arg Cys Arg Ser Asp Thr Leu Xaa Tyr Lys
   1               5                   10
<210> 86
   <211> 10
   <212> PRT
   <213> Unknown
<220>
   <223> Synthetic Construct
<220>
   <221> MOD_RES
   <222> (1)..(1)
   <223> ACETYLATION
<220>
   <221> DISULFID
   <222> (2)..(8)
   <223> Disulfide bond between Cys at position 2 and Xaa at position 8
<220>
   <221> MOD_RES
   <222> (3)..(3)
   <223> N(alpha)METHYLATION
<220>
   <221> MOD_RES
   <222> (8)..(8)
   <223> Pen
<400> 86
   Glu Cys Arg Ser Asp Thr Leu Xaa Tyr Lys
   1               5                   10
<210> 87
   <211> 8
   <212> PRT
   <213> Unknown
<220>
   <223> Synthetic Construct
<220>
   <221> DISULFID
   <222> (2)..(8)
   <223> Disulfide bond between Cys at position 2 and Xaa at position 8
<220>
   <221> MOD_RES
   <222> (3)..(3)
   <223> N(alpha)METHYLATION
<220>
   <221> MOD_RES
   <222> (8)..(8)
   <223> Pen
<400> 87
   Tyr Cys Arg Ser Asp Thr Leu Xaa
   1 5
<210> 88
   <211> 9
   <212> PRT
   <213> Unknown
<220>
   <223> Synthetic Construct
<220>
   <221> MOD_RES
   <222> (1)..(1)
   <223> ACETYLATION
<220>
   <221> DISULFID
   <222> (1)..(7)
   <223> Disulfide bond between Cys at position 1 and Xaa at position 7
<220>
   <221> MOD_RES
   <222> (2)..(2)
   <223> N(alpha)METHYLATION
<220>
   <221> MOD_RES
   <222> (7)..(7)
   <223> Pen
<220>
   <221> MOD_RES
   <222> (8)..(8)
   <223> 1-Nal
<220>
   <221> MOD_RES
   <222> (9)..(9)
   <223> D-Lys
<400> 88
   Cys Arg Ser Asp Thr Leu Xaa Xaa Xaa
   1 5
<210> 89
   <211> 9
   <212> PRT
   <213> Unknown
<220>
   <223> Synthetic Construct
<220>
   <221> MOD_RES
   <222> (1)..(1)
   <223> ACETYLATION
<220>
   <221> DISULFID
   <222> (1)..(7)
   <223> Disulfide bond between Cys at position 1 and Xaa at position 7
<220>
   <221> MOD_RES
   <222> (2)..(2)
   <223> N(alpha)METHYLATION
<220>
   <221> MOD_RES
   <222> (7)..(7)
   <223> Pen
<400> 89
   Cys Arg Ser Asp Thr Leu Xaa His Lys
   1               5
<210> 90
   <211> 9
   <212> PRT
   <213> Unknown
<220>
   <223> Synthetic Construct
<220>
   <221> MOD_RES
   <222> (1)..(1)
   <223> ACETYLATION
<220>
   <221> MOD_RES
   <222> (1)..(1)
   <223> Pen
<220>
   <221> DISULFID
   <222> (1)..(7)
   <223> Disulfide bond between Xaa at position 1 and Xaa at position 7
<220>
   <221> MOD_RES
   <222> (2)..(2)
   <223> N(alpha)METHYLATION
<220>
   <221> MOD_RES
   <222> (7)..(7)
   <223> Pen
<220>
   <221> MOD_RES
   <222> (9)..(9)
   <223> D-Lys
<400> 90
   Xaa Arg Ser Asp Thr Leu Xaa His Xaa
   1               5
<210> 91
   <211> 9
   <212> PRT
   <213> Unknown
<220>
   <223> Synthetic Construct
<220>
   <221> MOD_RES
   <222> (1)..(1)
   <223> Pen
<220>
   <221> MOD_RES
   <222> (1)..(1)
   <223> ACETYLATION
<220>
   <221> DISULFID
   <222> (1)..(7)
   <223> Disulfide bond between Xaa at position 1 and Xaa at position 7
<220>
   <221> MOD_RES
   <222> (2)..(2)
   <223> N(alpha)METHYLATION
<220>
   <221> MOD_RES
   <222> (7)..(7)
   <223> Pen
<220>
   <221> MOD_RES
   <222> (9)..(9)
   <223> D-Lys
<400> 91
   Xaa Arg Ser Asp Thr Leu Xaa Tyr Xaa
   1               5
<210> 92
   <211> 9
   <212> PRT
   <213> Unknown
<220>
   <223> Synthetic Construct
<220>
   <221> MOD_RES
   <222> (1)..(1)
   <223> Pen
<220>
   <221> MOD_RES
   <222> (1)..(1)
   <223> ACETYLATION
<220>
   <221> DISULFID
   <222> (1)..(7)
   <223> Disulfide bond between Xaa at position 1 and Xaa at position 7
<220>
   <221> MOD_RES
   <222> (2)..(2)
   <223> N(alpha)METHYLATION
<220>
   <221> MOD_RES
   <222> (7)..(7)
   <223> Pen
<220>
   <221> MOD_RES
   <222> (9)..(9)
   <223> D-Lys
<400> 92
   Xaa Arg Ser Asp Thr Leu Xaa Trp Xaa
   1               5
<210> 93
   <211> 8
   <212> PRT
   <213> Unknown
<220>
   <223> Synthetic Construct
<220>
   <221> MOD_RES
   <222> (1)..(1)
   <223> Pen
<220>
   <221> MOD_RES
   <222> (1)..(1)
   <223> ACETYLATION
<220>
   <221> DISULFID
   <222> (1)..(7)
   <223> Disulfide bond between Xaa at position 1 and Xaa at position 7
<220>
   <221> MOD_RES
   <222> (2)..(2)
   <223> N(alpha)METHYLATION
<220>
   <221> MOD_RES
   <222> (7)..(7)
   <223> Pen
<220>
   <221> MOD_RES
   <222> (8)..(8)
   <223> D-Lys, D-Phe
<400> 93
   Xaa Arg Ser Asp Thr Leu Xaa Xaa
   1               5
<210> 94
   <211> 8
   <212> PRT
   <213> Unknown
<220>
   <223> Synthetic Construct
<220>
   <221> MOD_RES
   <222> (1)..(1)
   <223> ACETYLATION
<220>
   <221> DISULFID
   <222> (1)..(7)
   <223> Disulfide bond between Cys at position 1 and Xaa at position 7
<220>
   <221> MOD_RES
   <222> (2)..(2)
   <223> N(alpha)METHYLATION
<220>
   <221> MOD_RES
   <222> (7)..(7)
   <223> Pen
<220>
   <221> MOD_RES
   <222> (8)..(8)
   <223> D-Lys
<400> 94
   Cys Arg Ser Asp Thr Leu Xaa Xaa
   1 5
<210> 95
   <211> 8
   <212> PRT
   <213> Unknown
<220>
   <223> Synthetic Construct
<220>
   <221> MOD_RES
   <222> (1)..(1)
   <223> Pen
<220>
   <221> MOD_RES
   <222> (1)..(1)
   <223> ACETYLATION
<220>
   <221> DISULFID
   <222> (1)..(7)
   <223> Disulfide bond between Xaa at position 1 and Cys at position 7
<220>
   <221> MOD_RES
   <222> (2)..(2)
   <223> N(alpha)METHYLATION
<220>
   <221> MOD_RES
   <222> (8)..(8)
   <223> D-Lys
<400> 95
   Xaa Arg Ser Asp Thr Leu Cys Xaa
   1 5
<210> 96
   <211> 9
   <212> PRT
   <213> Unknown
<220>
   <223> Synthetic Construct
<220>
   <221> MOD_RES
   <222> (1)..(1)
   <223> ACETYLATION
<220>
   <221> DISULFID
   <222> (1)..(7)
   <223> Disulfide bond between Cys at position 1 and Xaa at position 7
<220>
   <221> MOD_RES
   <222> (2)..(2)
   <223> N(alpha)METHYLATION
<220>
   <221> MOD_RES
   <222> (7)..(7)
   <223> Pen
<220>
   <221> MOD_RES
   <222> (9)..(9)
   <223> D-Lys
<400> 96
   Cys Arg Ser Asp Thr Leu Xaa Phe Xaa
   1               5
<210> 97
   <211> 9
   <212> PRT
   <213> Unknown
<220>
   <223> Synthetic Construct
<220>
   <221> MOD_RES
   <222> (1)..(1)
   <223> ACETYLATION
<220>
   <221> DISULFID
   <222> (1)..(7)
   <223> Disulfide bond between Cys at position 1 and Xaa at position 7
<220>
   <221> MOD_RES
   <222> (2)..(2)
   <223> N(alpha)METHYLATION
<220>
   <221> MOD_RES
   <222> (7)..(7)
   <223> Pen
<220>
   <221> MOD_RES
   <222> (8)..(8)
   <223> D-Phe, 2-Nal, HPhe, Bip, Phe(4-F), Tyr(OMe), Ala(3,3 biphenyl), Trp(di-hydro), dTrp (di-hydro), Phe(4-Me)
<220>
   <221> MOD_RES
   <222> (9)..(9)
   <223> D-Lys
<400> 97
   Cys Arg Ser Asp Thr Leu Xaa Xaa Xaa
   1               5
<210> 98
   <211> 8
   <212> PRT
   <213> Unknown
<220>
   <223> Synthetic Construct
<220>
   <221> DISULFID
   <222> (2)..(8)
   <223> Disulfide bond between Cys at position 2 and Xaa at position 8
<220>
   <221> MOD_RES
   <222> (3)..(3)
   <223> N(alpha)METHYLATION
<220>
   <221> MOD_RES
   <222> (8)..(8)
   <223> Pen
<400> 98
   Ser Cys Arg Ser Asp Thr Leu Xaa
           1               5
<210> 99
   <211> 8
   <212> PRT
   <213> Unknown
<220>
   <223> Synthetic Construct
<220>
   <221> DISULFID
   <222> (2)..(8)
   <223> Disulfide bond between Cys at position 2 and Xaa at position 8
<220>
   <221> MOD_RES
   <222> (3)..(3)
   <223> N(alpha)METHYLATION
<220>
   <221> MOD_RES
   <222> (8)..(8)
   <223> Pen
<400> 99
   Asn Cys Arg Ser Asp Thr Leu Xaa
   1               5
<210> 100
   <211> 8
   <212> PRT
   <213> Unknown
<220>
   <223> Synthetic Construct
<220>
   <221> DISULFID
   <222> (1)..(7)
   <223> Disulfide bond between Cys at position 1 and Xaa at position 7
<220>
   <221> MOD_RES
   <222> (2)..(2)
   <223> N(alpha)METHYLATION
<220>
   <221> MOD_RES
   <222> (7)..(7)
   <223> Pen
<400> 100
   Cys Arg Ser Asp Thr Leu Xaa Trp
   1               5
<210> 101
   <211> 8
   <212> PRT
   <213> Unknown
<220>
   <223> Synthetic Construct
<220>
   <221> DISULFID
   <222> (1)..(7)
   <223> Disulfide bond between Cys at position 1 and Xaa at position 7
<220>
   <221> MOD_RES
   <222> (2)..(2)
   <223> N(alpha)METHYLATION
<220>
   <221> MOD_RES
   <222> (7)..(7)
   <223> Pen
<220>
   <221> MOD_RES
   <222> (8)..(8)
   <223> D-Trp, D-Phe
<400> 101
   Cys Arg Ser Asp Thr Leu Xaa Xaa
   1               5
<210> 102
   <211> 9
   <212> PRT
   <213> Unknown
<220>
   <223> Synthetic Construct
<220>
   <221> MOD_RES
   <222> (1)..(1)
   <223> ACETYLATION
<220>
   <221> MOD_RES
   <222> (1)..(1)
   <223> N(alpha)METHYLATION
<220>
   <221> MOD_RES
   <222> (2)..(2)
   <223> Pen
<220>
   <221> DISULFID
   <222> (2)..(8)
   <223> Disulfide bond between Xaa at position 2 and Xaa at position 8
<220>
   <221> MOD_RES
   <222> (2)..(2)
   <223> N(alpha)METHYLATION
<220>
   <221> MOD_RES
   <222> (3)..(3)
   <223> N(alpha)METHYLATION
<220>
   <221> MOD_RES
   <222> (8)..(8)
   <223> Pen
<400> 102
   Lys Xaa Arg Ser Asp Thr Leu Xaa Trp
   1               5
<210> 103
   <211> 10
   <212> PRT
   <213> Unknown
<220>
   <223> Synthetic Construct
<220>
   <221> MOD_RES
   <222> (1)..(1)
   <223> ACETYLATION
<220>
   <221> MOD_RES
   <222> (1)..(1)
   <223> N(alpha)METHYLATION
<220>
   <221> DISULFID
   <222> (2)..(8)
   <223> Disulfide bond between Xaa at position 2 and Xaa at position 8
<220>
   <221> MOD_RES
   <222> (2)..(2)
   <223> Pen
<220>
   <221> MOD_RES
   <222> (3)..(3)
   <223> N(alpha)METHYLATION
<220>
   <221> MOD_RES
   <222> (8)..(8)
   <223> Pen
<220>
   <221> MOD_RES
   <222> (10)..(10)
   <223> N(alpha)METHYLATION
<400> 103
   Lys Xaa Arg Ser Asp Thr Leu Xaa Trp Lys
   1               5                   10
<210> 104
   <211> 10
   <212> PRT
   <213> Unknown
<220>
   <223> Synthetic Construct
<220>
   <221> MOD_RES
   <222> (1)..(1)
   <223> ACETYLATION
<220>
   <221> MOD_RES
   <222> (1)..(1)
   <223> N(alpha)METHYLATION
<220>
   <221> MOD_RES
   <222> (1)..(1)
   <223> D-Lys
<220>
   <221> MOD_RES
   <222> (2)..(2)
   <223> Pen
<220>
   <221> DISULFID
   <222> (2)..(8)
   <223> Disulfide bond between Xaa at position 2 and Xaa at position 8
<220>
   <221> MOD_RES
   <222> (3)..(3)
   <223> N(alpha)METHYLATION
<220>
   <221> MOD_RES
   <222> (8)..(8)
   <223> Pen
<220>
   <221> MOD_RES
   <222> (9)..(9)
   <223> N(alpha)METHYLATION
<220>
   <221> MOD_RES
   <222> (10)..(10)
   <223> D-Lys
<400> 104
   Xaa Xaa Arg Ser Asp Thr Leu Xaa Trp Xaa
   1 5 10
<210> 105
   <211> 10
   <212> PRT
   <213> Unknown
<220>
   <223> Synthetic Construct
<220>
   <221> MOD_RES
   <222> (1)..(1)
   <223> ACETYLATION
<220>
   <221> MOD_RES
   <222> (1)..(1)
   <223> Pen
<220>
   <221> DISULFID
   <222> (1)..(7)
   <223> Disulfide bond between Xaa at position 1 and Xaa at position 7
<220>
   <221> MOD_RES
   <222> (2)..(2)
   <223> N(alpha)METHYLATION
<220>
   <221> MOD_RES
   <222> (7)..(7)
   <223> Pen
<220>
   <221> MOD_RES
   <222> (10)..(10)
   <223> D-Lys
<400> 105
   Xaa Arg Ser Asp Thr Leu Xaa Trp Phe Xaa
   1 5 10
<210> 106
   <211> 10
   <212> PRT
   <213> Unknown
<220>
   <223> Synthetic Construct
<220>
   <221> MOD_RES
   <222> (1)..(1)
   <223> ACETYLATION
<220>
   <221> MOD_RES
   <222> (1)..(1)
   <223> Pen
<220>
   <221> DISULFID
   <222> (1)..(7)
   <223> Disulfide bond between Xaa at position 1 and Xaa at position 7
<220>
   <221> MOD_RES
   <222> (2)..(2)
   <223> N(alpha)METHYLATION
<220>
   <221> MOD_RES
   <222> (7)..(7)
   <223> Pen
<220>
   <221> MOD_RES
   <222> (9)..(9)
   <223> N(alpha)METHYLATION
<220>
   <221> MOD_RES
   <222> (10)..(10)
   <223> D-Lys
<220>
   <221> MOD_RES
   <222> (10)..(10)
   <223> N(alpha)METHYLATION
<400> 106
   Xaa Arg Ser Asp Thr Leu Xaa Trp Glu Xaa
   1               5                   10
<210> 107
   <211> 10
   <212> PRT
   <213> Unknown
<220>
   <223> Synthetic Construct
<220>
   <221> MOD_RES
   <222> (1)..(1)
   <223> ACETYLATION
<220>
   <221> MOD_RES
   <222> (1)..(1)
   <223> Pen
<220>
   <221> DISULFID
   <222> (1)..(7)
   <223> Disulfide bond between Xaa at postion 1 and Xaa at position 7
<220>
   <221> MOD_RES
   <222> (2)..(2)
   <223> N(alpha)METHYLATION
<220>
   <221> MOD_RES
   <222> (7)..(7)
   <223> Pen
<220>
   <221> MOD_RES
   <222> (10)..(10)
   <223> N(alpha)METHYLATION
<400> 107
   Xaa Arg Ser Asp Thr Leu Xaa Trp Glu Lys
   1               5                   10
<210> 108
   <211> 10
   <212> PRT
   <213> Unknown
<220>
   <223> Synthetic Construct
<220>
   <221> MOD_RES
   <222> (1)..(1)
   <223> ACETYLATION
<220>
   <221> MOD_RES
   <222> (1)..(1)
   <223> Pen
<220>
   <221> DISULFID
   <222> (1)..(7)
   <223> Disulfide bond between Xaa at position 1 and Xaa at position 7
<220>
   <221> MOD_RES
   <222> (2)..(2)
   <223> N(alpha)METHYLATION
<220>
   <221> MOD_RES
   <222> (7)..(7)
   <223> Pen
<220>
   <221> MOD_RES
   <222> (10)..(10)
   <223> D-Lys
<400> 108
   Xaa Arg Ser Asp Thr Leu Xaa Trp Ser Xaa
   1               5                   10
<210> 109
   <211> 10
   <212> PRT
   <213> Unknown
<220>
   <223> Synthetic Construct
<220>
   <221> MOD_RES
   <222> (1)..(1)
   <223> ACETYLATION
<220>
   <221> MOD_RES
   <222> (1)..(1)
   <223> Pen
<220>
   <221> DISULFID
   <222> (1)..(7)
   <223> Disulfide bond between Xaa at position 1 and Xaa at position 7
<220>
   <221> MOD_RES
   <222> (2)..(2)
   <223> N(alpha)METHYLATION
<220>
   <221> MOD_RES
   <222> (7)..(7)
   <223> Pen
<220>
   <221> MOD_RES
   <222> (10)..(10)
   <223> D-Lys
<400> 109
   Xaa Arg Ser Asp Thr Leu Xaa Trp Tyr Xaa
   1 5 10
<210> 110
   <211> 9
   <212> PRT
   <213> Unknown
<220>
   <223> Synthetic Construct
<220>
   <221> MOD_RES
   <222> (1)..(1)
   <223> ACETYLATION
<220>
   <221> MOD_RES
   <222> (1)..(1)
   <223> Pen
<220>
   <221> DISULFID
   <222> (1)..(7)
   <223> Disulfide bond between Xaa at position 1 and Xaa at position 7
<220>
   <221> MOD_RES
   <222> (2)..(2)
   <223> N(alpha)METHYLATION
<220>
   <221> MOD_RES
   <222> (7)..(7)
   <223> Pen
<220>
   <221> MOD_RES
   <222> (8)..(8)
   <223> D-Trp, D-Phe
<220>
   <221> MOD_RES
   <222> (9)..(9)
   <223> D-Lys
<400> 110
   Xaa Arg Ser Asp Thr Leu Xaa Xaa Xaa
   1 5
<210> 111
   <211> 9
   <212> PRT
   <213> Unknown
<220>
   <223> Synthetic Construct
<220>
   <221> MOD_RES
   <222> (1)..(1)
   <223> ACETYLATION
<220>
   <221> MOD_RES
   <222> (1)..(1)
   <223> Pen
<220>
   <221> DISULFID
   <222> (1)..(7)
   <223> Disulfide bond between Xaa at position 1 and Xaa at position 7
<220>
   <221> MOD_RES
   <222> (2)..(2)
   <223> N(alpha)METHYLATION
<220>
   <221> MOD_RES
   <222> (7)..(7)
   <223> Pen
<220>
   <221> MOD_RES
   <222> (9)..(9)
   <223> N(alpha)METHYLATION
<400> 111
   Xaa Arg Ser Asp Thr Leu Xaa Trp Lys
   1               5
<210> 112
   <211> 9
   <212> PRT
   <213> Unknown
<220>
   <223> Synthetic Construct
<220>
   <221> MOD_RES
   <222> (1)..(1)
   <223> ACETYLATION
<220>
   <221> MOD_RES
   <222> (1)..(1)
   <223> Pen
<220>
   <221> DISULFID
   <222> (1)..(7)
   <223> Disulfide bond between Xaa at position 1 and Xaa at position 7
<220>
   <221> MOD_RES
   <222> (2)..(2)
   <223> N(alpha)METHYLATION
<220>
   <221> MOD_RES
   <222> (4)..(4)
   <223> D-Asp
<220>
   <221> MOD_RES
   <222> (9)..(9)
   <223> D-Lys
<400> 112
   Xaa Arg Ser Xaa Thr Leu Xaa Trp Xaa
   1               5
<210> 113
   <211> 9
   <212> PRT
   <213> Unknown
<220>
   <223> Synthetic Construct
<220>
   <221> MOD_RES
   <222> (2)..(2)
   <223> Pen
<220>
   <221> DISULFID
   <222> (2)..(8)
   <223> Disulfide bond between Xaa at position 2 and Xaa at position 8
<220>
   <221> MOD_RES
   <222> (3)..(3)
   <223> N(alpha)METHYLATION
<220>
   <221> MOD_RES
   <222> (8)..(8)
   <223> Pen
<400> 113
   Pro Xaa Arg Ser Asp Thr Leu Xaa Trp
   1               5
<210> 114
   <211> 9
   <212> PRT
   <213> Unknown
<220>
   <223> Synthetic Construct
<220>
   <221> MOD_RES
   <222> (2)..(2)
   <223> Pen
<220>
   <221> DISULFID
   <222> (2)..(8)
   <223> Disulfide bond between Xaa at position 2 and Xaa at position 8
<220>
   <221> MOD_RES
   <222> (3)..(3)
   <223> N(alpha)METHYLATION
<220>
   <221> MOD_RES
   <222> (8)..(8)
   <223> Pen
<400> 114
   Leu Xaa Arg Ser Asp Thr Leu Xaa Trp
   1               5
<210> 115
   <211> 9
   <212> PRT
   <213> Unknown
<220>
   <223> Synthetic Construct
<220>
   <221> MOD_RES
   <222> (2)..(2)
   <223> Pen
<220>
   <221> DISULFID
   <222> (2)..(8)
   <223> Disulfide bond between Xaa at position 2 and Xaa at position 8
<220>
   <221> MOD_RES
   <222> (3)..(3)
   <223> N(alpha)METHYLATION
<220>
   <221> MOD_RES
   <222> (8)..(8)
   <223> Pen
<400> 115
   His Xaa Arg Ser Asp Thr Leu Xaa Trp
   1 5
<210> 116
   <211> 9
   <212> PRT
   <213> Unknown
<220>
   <223> Synthetic Construct
<220>
   <221> MOD_RES
   <222> (2)..(2)
   <223> Pen
<220>
   <221> DISULFID
   <222> (2)..(8)
   <223> Disulfide bond between Xaa at position 2 and Xaa at position 8
<220>
   <221> MOD_RES
   <222> (3)..(3)
   <223> N(alpha)METHYLATION
<220>
   <221> MOD_RES
   <222> (8)..(8)
   <223> Pen
<400> 116
   Glu Xaa Arg Ser Asp Thr Leu Xaa Trp
   1 5
<210> 117
   <211> 9
   <212> PRT
   <213> Unknown
<220>
   <223> Synthetic Construct
<220>
   <221> MOD_RES
   <222> (2)..(2)
   <223> Pen
<220>
   <221> DISULFID
   <222> (2)..(8)
   <223> Disulfide bond between Xaa at position 2 and Xaa at position 8
<220>
   <221> MOD_RES
   <222> (3)..(3)
   <223> N(alpha)METHYLATION
<220>
   <221> MOD_RES
   <222> (8)..(8)
   <223> Pen
<400> 117
   Arg Xaa Arg Ser Asp Thr Leu Xaa Trp
   1               5
<210> 118
   <211> 9
   <212> PRT
   <213> Unknown
<220>
   <223> Synthetic Construct
<220>
   <221> MOD_RES
   <222> (2)..(2)
   <223> Pen
<220>
   <221> DISULFID
   <222> (2)..(8)
   <223> Disulfide bond between Xaa at position 2 and Xaa at position 8
<220>
   <221> MOD_RES
   <222> (3)..(3)
   <223> N(alpha)METHYLATION
<220>
   <221> MOD_RES
   <222> (8)..(8)
   <223> Pen
<400> 118
   Ser Xaa Arg Ser Asp Thr Leu Xaa Trp
   1               5
<210> 119
   <211> 9
   <212> PRT
   <213> Unknown
<220>
   <223> Synthetic Construct
<220>
   <221> MOD_RES
   <222> (2)..(2)
   <223> Pen
<220>
   <221> DISULFID
   <222> (2)..(8)
   <223> Disulfide bond between Xaa at position 2 and Xaa at position 8
<220>
   <221> MOD_RES
   <222> (3)..(3)
   <223> N(alpha)METHYLATION
<220>
   <221> MOD_RES
   <222> (8)..(8)
   <223> Pen
<400> 119
   Phe Xaa Arg Ser Asp Thr Leu Xaa Trp
   1               5
<210> 120
   <211> 10
   <212> PRT
   <213> Unknown
<220>
   <223> Synthetic Construct
<220>
   <221> MOD_RES
   <222> (1)..(1)
   <223> ACETYLATION
<220>
   <221> MOD_RES
   <222> (1)..(1)
   <223> Pen
<220>
   <221> DISULFID
   <222> (1)..(7)
   <223> Disulfide bond between Xaa at position 1 and Xaa at position 7
<220>
   <221> MOD_RES
   <222> (2)..(2)
   <223> N(alpha)METHYLATION
<220>
   <221> MOD_RES
   <222> (7)..(7)
   <223> Pen
<220>
   <221> MOD_RES
   <222> (8)..(8)
   <223> D-Phe, D-Trp, 2-Nal, 3-3-diPhe, Beta-HTrp, Phe(4-CF3), 1-Nal, 2-Nal, Phe(2,4-C12), Phe(3,4-C12), D-1-Nal, D-2-Nal, HPhe, D-HPhe, 2,2-Indane, 1,1-Indane
<220>
   <221> MOD_RES
   <222> (10)..(10)
   <223> D-Lys
<220>
   <221> MOD_RES
   <222> (10)..(10)
   <223> N(alpha)METHYLATION
<400> 120
   Xaa Arg Ser Asp Thr Leu Xaa Xaa Glu Xaa
   1               5                   10
<210> 121
   <211> 10
   <212> PRT
   <213> Unknown
<220>
   <223> Synthetic Construct
<220>
   <221> MOD_RES
   <222> (1)..(1)
   <223> ACETYLATION
<220>
   <221> MOD_RES
   <222> (1)..(1)
   <223> Pen
<220>
   <221> DISULFID
   <222> (1)..(7)
   <223> Disulfide bond between Xaa at position 1 and Xaa at position 7
<220>
   <221> MOD_RES
   <222> (2)..(2)
   <223> N(alpha)METHYLATION
<220>
   <221> MOD_RES
   <222> (7)..(7)
   <223> Pen
<220>
   <221> MOD_RES
   <222> (10)..(10)
   <223> D-Lys
<400> 121
   Xaa Arg Ser Asp Thr Leu Xaa Trp Leu Xaa
   1               5                   10
<210> 122
   <211> 10
   <212> PRT
   <213> Unknown
<220>
   <223> Synthetic Construct
<220>
   <221> MOD_RES
   <222> (1)..(1)
   <223> ACETYLATION
<220>
   <221> MOD_RES
   <222> (1)..(1)
   <223> Pen
<220>
   <221> DISULFID
   <222> (1)..(7)
   <223> Disulfide bond between Xaa at position 1 and Xaa at position 7
<220>
   <221> MOD_RES
   <222> (2)..(2)
   <223> N(alpha)METHYLATION
<220>
   <221> MOD_RES
   <222> (7)..(7)
   <223> Pen
<220>
   <221> MOD_RES
   <222> (10)..(10)
   <223> D-Lys
<400> 122
   Xaa Arg Ser Asp Thr Leu Xaa Trp His Xaa
   1               5                   10
<210> 123
   <211> 10
   <212> PRT
   <213> Unknown
<220>
   <223> Synthetic Construct
<220>
   <221> MOD_RES
   <222> (1)..(1)
   <223> ACETYLATION
<220>
   <221> MOD_RES
   <222> (1)..(1)
   <223> Pen
<220>
   <221> DISULFID
   <222> (1)..(7)
   <223> Disulfide bond between Xaa at position 1 and Xaa at position 7
<220>
   <221> MOD_RES
   <222> (2)..(2)
   <223> N(alpha)METHYLATION
<220>
   <221> MOD_RES
   <222> (7)..(7)
   <223> Pen
<220>
   <221> MOD_RES
   <222> (10)..(10)
   <223> D-Lys
<400> 123
   Xaa Arg Ser Asp Thr Leu Xaa Trp Arg Xaa
   1               5                   10
<210> 124
   <211> 10
   <212> PRT
   <213> Unknown
<220>
   <223> Synthetic Construct
<220>
   <221> MOD_RES
   <222> (1)..(1)
   <223> ACETYLATION
<220>
   <221> MOD_RES
   <222> (1)..(1)
   <223> Pen
<220>
   <221> DISULFID
   <222> (1)..(7)
   <223> Disulfide bond between Xaa at position 1 and Xaa at position 7
<220>
   <221> MOD_RES
   <222> (2)..(2)
   <223> N(alpha)METHYLATION
<220>
   <221> MOD_RES
   <222> (7)..(7)
   <223> Pen
<220>
   <221> MOD_RES
   <222> (10)..(10)
   <223> D-Lys
<400> 124
   Xaa Arg Ser Asp Thr Leu Xaa Trp Trp Xaa
   1               5                   10
<210> 125
   <211> 10
   <212> PRT
   <213> Unknown
<220>
   <223> Synthetic Construct
<220>
   <221> MOD_RES
   <222> (1)..(1)
   <223> ACETYLATION
<220>
   <221> MOD_RES
   <222> (1)..(1)
   <223> Pen
<220>
   <221> DISULFID
   <222> (1)..(7)
   <223> Disulfide bond between Xaa at position 1 and Xaa at position 7
<220>
   <221> MOD_RES
   <222> (2)..(2)
   <223> N(alpha)METHYLATION
<220>
   <221> MOD_RES
   <222> (7)..(7)
   <223> Pen
<220>
   <221> MOD_RES
   <222> (10)..(10)
   <223> D-Lys
<400> 125
   Xaa Arg Ser Asp Thr Leu Xaa Trp Pro Xaa
   1               5                   10
<210> 126
   <211> 10
   <212> PRT
   <213> Unknown
<220>
   <223> Synthetic Construct
<220>
   <221> MOD_RES
   <222> (1)..(1)
   <223> ACETYLATION
<220>
   <221> MOD_RES
   <222> (1)..(1)
   <223> Pen
<220>
   <221> DISULFID
   <222> (1)..(7)
   <223> Disulfide bond between Xaa at position 1 and Xaa at position 7
<220>
   <221> MOD_RES
   <222> (2)..(2)
   <223> N(alpha)METHYLATION
<220>
   <221> MOD_RES
   <222> (7)..(7)
   <223> Pen
<220>
   <221> MOD_RES
   <222> (10)..(10)
   <223> D-Lys
<400> 126
   Xaa Arg Ser Asp Thr Leu Xaa Trp Asn Xaa
   1               5                   10
<210> 127
   <211> 9
   <212> PRT
   <213> Unknown
<220>
   <223> Synthetic Construct
<220>
   <221> MOD_RES
   <222> (1)..(1)
   <223> ACETYLATION
<220>
   <221> MOD_RES
   <222> (1)..(1)
   <223> Pen
<220>
   <221> DISULFID
   <222> (1)..(7)
   <223> Disulfide bond between Xaa at position 1 and Xaa at position 7
<220>
   <221> MOD_RES
   <222> (2)..(2)
   <223> N(alpha)METHYLATION
<220>
   <221> MOD_RES
   <222> (7)..(7)
   <223> Pen
<220>
   <221> MOD_RES
   <222> (8)..(8)
   <223> D-Phe
<220>
   <221> MOD_RES
   <222> (9)..(9)
   <223> D-Lys
<400> 127
   Xaa Arg Thr Asp Thr Leu Xaa Xaa Xaa
   1               5
<210> 128
   <211> 9
   <212> PRT
   <213> Unknown
<220>
   <223> Synthetic Construct
<220>
   <221> MOD_RES
   <222> (1)..(1)
   <223> ACETYLATION
<220>
   <221> MOD_RES
   <222> (1)..(1)
   <223> Pen
<220>
   <221> DISULFID
   <222> (1)..(7)
   <223> Disulfide bond between Xaa at position 1 and Xaa at position 7
<220>
   <221> MOD_RES
   <222> (2)..(2)
   <223> N(alpha)METHYLATION
<220>
   <221> MOD_RES
   <222> (7)..(7)
   <223> Pen
<220>
   <221> MOD_RES
   <222> (8)..(8)
   <223> D-Phe
<220>
   <221> MOD_RES
   <222> (9)..(9)
   <223> D-Lys
<400> 128
   Xaa Arg Ile Asp Thr Leu Xaa Xaa Xaa
   1               5
<210> 129
   <211> 9
   <212> PRT
   <213> Unknown
<220>
   <223> Synthetic Construct
<220>
   <221> MOD_RES
   <222> (1)..(1)
   <223> ACETYLATION
<220>
   <221> DISULFID
   <222> (1)..(7)
   <223> Disulfide bond between Cys at position 1 and Cys at position 7
<220>
   <221> MOD_RES
   <222> (2)..(2)
   <223> N(alpha)METHYLATION
<220>
   <221> MOD_RES
   <222> (8)..(8)
   <223> D-Phe
<220>
   <221> MOD_RES
   <222> (9)..(9)
   <223> D-Lys
<400> 129
   Cys Arg Ser Asp Ile Leu Cys Xaa Xaa
   1               5
<210> 130
   <211> 9
   <212> PRT
   <213> Unknown
<220>
   <223> Synthetic Construct
<220>
   <221> MOD_RES
   <222> (1)..(1)
   <223> ACETYLATION
<220>
   <221> DISULFID
   <222> (1)..(7)
   <223> Disulfide bond between Cys at position 1 and Cys at position 7
<220>
   <221> MOD_RES
   <222> (2)..(2)
   <223> N(alpha)METHYLATION
<220>
   <221> MOD_RES
   <222> (8)..(8)
   <223> D-Phe
<220>
   <221> MOD_RES
   <222> (9)..(9)
   <223> D-Lys
<400> 130
   Cys Arg Ser Asp Val Leu Cys Xaa Xaa
   1               5
<210> 131
   <211> 9
   <212> PRT
   <213> Unknown
<220>
   <223> Synthetic Construct
<220>
   <221> MOD_RES
   <222> (1)..(1)
   <223> ACETYLATION
<220>
   <221> DISULFID
   <222> (1)..(7)
   <223> Disulfide bond between Cys at position 1 and Cys at position 7
<220>
   <221> MOD_RES
   <222> (2)..(2)
   <223> N(alpha)METHYLATION
<220>
   <221> MOD_RES
   <222> (6)..(6)
   <223> HLeu, cyclobutyl-Ala, HCha, Nle
<220>
   <221> MOD_RES
   <222> (9)..(9)
   <223> D-Lys
<400> 131
   Cys Arg Ser Asp Thr Xaa Cys Trp Xaa
   1               5
<210> 132
   <211> 9
   <212> PRT
   <213> Unknown
<220>
   <223> Synthetic Construct
<220>
   <221> MOD_RES
   <222> (1)..(1)
   <223> ACETYLATION
<220>
   <221> DISULFID
   <222> (1)..(7)
   <223> Disulfide bond between Cys at position 1 and Cys at position 7
<220>
   <221> MOD_RES
   <222> (2)..(2)
   <223> N(alpha)METHYLATION
<220>
   <221> MOD_RES
   <222> (9)..(9)
   <223> D-Lys
<400> 132
   Cys Arg Ser Asp Thr Leu Cys Trp Xaa
   1               5
<210> 133
   <211> 10
   <212> PRT
   <213> Unknown
<220>
   <223> Synthetic Construct
<220>
   <221> MOD_RES
   <222> (1)..(1)
   <223> ACETYLATION
<220>
   <221> MOD_RES
   <222> (1)..(1)
   <223> Pen
<220>
   <221> DISULFID
   <222> (1)..(7)
   <223> Disulfide bond between Xaa at position 1 and Xaa at position 7
<220>
   <221> MOD_RES
   <222> (2)..(2)
   <223> N(alpha)METHYLATION
<220>
   <221> MOD_RES
   <222> (7)..(7)
   <223> Pen
<220>
   <221> MOD_RES
   <222> (9)..(9)
   <223> D-Asp, D-Glu, Beta-HGlu, 1-Nal, 2-Nal, Bip, Beta-HPhe, Beta-Glu
<220>
   <221> MOD_RES
   <222> (10)..(10)
   <223> D-Lys
<400> 133
   Xaa Arg Ser Asp Thr Leu Xaa Trp Xaa Xaa
   1               5                   10
<210> 134
   <211> 10
   <212> PRT
   <213> Unknown
<220>
   <223> Synthetic Construct
<220>
   <221> MOD_RES
   <222> (1)..(1)
   <223> ACETYLATION
<220>
   <221> MOD_RES
   <222> (1)..(1)
   <223> Pen
<220>
   <221> DISULFID
   <222> (1)..(7)
   <223> Disulfide bond between Xaa at position 1 and Xaa at position 7
<220>
   <221> MOD_RES
   <222> (2)..(2)
   <223> N(alpha)METHYLATION
<220>
   <221> MOD_RES
   <222> (7)..(7)
   <223> Pen
<220>
   <221> MOD_RES
   <222> (8)..(8)
   <223> D-Phe, D-Trp, 1-Nal, 2-Nal, Phe(4-CF3), HPhe
<220>
   <221> MOD_RES
   <222> (8)..(8)
   <223> D-Phe, D-Trp, 1-Nal, 2-Nal, Phe(4-CF3), HPhe
<220>
   <221> MOD_RES
   <222> (9)..(9)
   <223> D-Glu, D-Tyr
<220>
   <221> MOD_RES
   <222> (10)..(10)
   <223> D-Lys
<400> 134
   Xaa Arg Ser Asp Thr Leu Xaa Xaa Xaa Xaa
   1               5                   10
<210> 135
   <211> 10
   <212> PRT
   <213> Unknown
<220>
   <223> Synthetic Construct
<220>
   <221> MOD_RES
   <222> (1)..(1)
   <223> ACETYLATION
<220>
   <221> MOD_RES
   <222> (1)..(1)
   <223> Pen
<220>
   <221> DISULFID
   <222> (1)..(7)
   <223> Disulfide bond between Xaa at position 1 and Xaa at position 7
<220>
   <221> MOD_RES
   <222> (2)..(2)
   <223> N(alpha)METHYLATION
<220>
   <221> MOD_RES
   <222> (7)..(7)
   <223> Pen
<220>
   <221> MOD_RES
   <222> (9)..(9)
   <223> D-Glu
<220>
   <221> MOD_RES
   <222> (10)..(10)
   <223> D-Lys
<400> 135
   Xaa Arg Ser Asp Thr Leu Xaa Phe Xaa Xaa
   1               5                   10
<210> 136
   <211> 9
   <212> PRT
   <213> Unknown
<220>
   <223> Synthetic Construct
<220>
   <221> MOD_RES
   <222> (1)..(1)
   <223> ACETYLATION
<220>
   <221> MOD_RES
   <222> (1)..(1)
   <223> Pen
<220>
   <221> DISULFID
   <222> (1)..(7)
   <223> Disulfide bond between Xaa at position 1 and Xaa at position 7
<220>
   <221> MOD_RES
   <222> (2)..(2)
   <223> N(alpha)METHYLATION
<220>
   <221> MOD_RES
   <222> (7)..(7)
   <223> Pen
<220>
   <221> MOD_RES
   <222> (9)..(9)
   <223> D-Lys
<400> 136
   Xaa Lys Ser Asp Thr Leu Xaa Trp Xaa
   1               5
<210> 137
   <211> 10
   <212> PRT
   <213> Unknown
<220>
   <223> Synthetic Construct
<220>
   <221> MOD_RES
   <222> (1)..(1)
   <223> ACETYLATION
<220>
   <221> MOD_RES
   <222> (1)..(1)
   <223> Pen
<220>
   <221> DISULFID
   <222> (1)..(7)
   <223> Disulfide bond between Xaa at position 1 and Xaa at position 7
<220>
   <221> MOD_RES
   <222> (2)..(2)
   <223> N(alpha)METHYLATION
<220>
   <221> MOD_RES
   <222> (7)..(7)
   <223> Pen
<220>
   <221> MOD_RES
   <222> (10)..(10)
   <223> N(alpha)METHYLATION
<400> 137
   Xaa Arg Ser Asp Thr Leu Xaa Trp Tyr Lys
   1               5                   10
<210> 138
   <211> 9
   <212> PRT
   <213> Unknown
<220>
   <223> Synthetic Construct
<220>
   <221> MOD_RES
   <222> (1)..(1)
   <223> ACETYLATION
<220>
   <221> MOD_RES
   <222> (1)..(1)
   <223> Pen
<220>
   <221> DISULFID
   <222> (1)..(7)
   <223> Disulfide bond between Xaa at position 1 and Xaa at position 7
<220>
   <221> MOD_RES
   <222> (2)..(2)
   <223> N(alpha)METHYLATION
<220>
   <221> MOD_RES
   <222> (6)..(6)
   <223> Nle
<220>
   <221> MOD_RES
   <222> (7)..(7)
   <223> Pen
<220>
   <221> MOD_RES
   <222> (9)..(9)
   <223> D-Lys
<400> 138
   Xaa Arg Ser Asp Thr Xaa Xaa Trp Xaa
   1               5
<210> 139
   <211> 10
   <212> PRT
   <213> Unknown
<220>
   <223> Synthetic Construct
<220>
   <221> MOD_RES
   <222> (1)..(1)
   <223> ACETYLATION
<220>
   <221> MOD_RES
   <222> (1)..(1)
   <223> Pen
<220>
   <221> DISULFID
   <222> (1)..(7)
   <223> Disulfide bond between Xaa at position 1 and Xaa at position 7
<220>
   <221> MOD_RES
   <222> (2)..(2)
   <223> N(alpha)METHYLATION
<220>
   <221> MOD_RES
   <222> (6)..(6)
   <223> Nle
<220>
   <221> MOD_RES
   <222> (7)..(7)
   <223> Pen
<220>
   <221> MOD_RES
   <222> (10)..(10)
   <223> D-Lys
<400> 139
   Xaa Arg Ser Asp Thr Xaa Xaa Trp Glu Xaa
   1               5                   10
<210> 140
   <211> 8
   <212> PRT
   <213> Unknown
<220>
   <223> Synthetic Construct
<220>
   <221> MOD_RES
   <222> (1)..(1)
   <223> Pen
<220>
   <221> DISULFID
   <222> (1)..(7)
   <223> Disulfide bond between Xaa at position 1 and Xaa at position 7
<220>
   <221> MOD_RES
   <222> (2)..(2)
   <223> N(alpha)METHYLATION
<220>
   <221> MOD_RES
   <222> (7)..(7)
   <223> Pen
<220>
   <221> MOD_RES
   <222> (8)..(8)
   <223> D-Phe
<400> 140
   Xaa Arg Ser Asp Thr Leu Xaa Xaa
   1               5
<210> 141
   <211> 8
   <212> PRT
   <213> Unknown
<220>
   <223> Synthetic Construct
<220>
   <221> MOD_RES
   <222> (1)..(1)
   <223> Pen
<220>
   <221> DISULFID
   <222> (1)..(7)
   <223> Disulfide bond between Xaa at position 1 and Xaa at position 7
<220>
   <221> MOD_RES
   <222> (2)..(2)
   <223> N(alpha)METHYLATION
<220>
   <221> MOD_RES
   <222> (7)..(7)
   <223> Pen
<400> 141
   Xaa Arg Ser Asp Thr Leu Xaa Trp
   1               5
<210> 142
   <211> 10
   <212> PRT
   <213> Unknown
<220>
   <223> Synthetic Construct
<220>
   <221> MOD_RES
   <222> (1)..(1)
   <223> ACETYLATION
<220>
   <221> MOD_RES
   <222> (1)..(1)
   <223> Pen
<220>
   <221> DISULFID
   <222> (1)..(7)
   <223> Disulfide bond between Xaa at position 1 and Xaa at position 7
<220>
   <221> MOD_RES
   <222> (2)..(2)
   <223> N(alpha)METHYLATION
<220>
   <221> MOD_RES
   <222> (7)..(7)
   <223> Pen
<220>
   <221> MOD_RES
   <222> (10)..(10)
   <223> D-Lys
<400> 142
   Xaa Arg Ser Asp Thr Leu Xaa Trp Asp Xaa
   1               5                   10
<210> 143
   <211> 10
   <212> PRT
   <213> Unknown
<220>
   <223> Synthetic Construct
<220>
   <221> MOD_RES
   <222> (1)..(1)
   <223> ACETYLATION
<220>
   <221> MOD_RES
   <222> (1)..(1)
   <223> Pen
<220>
   <221> DISULFID
   <222> (1)..(7)
   <223> Disulfide bond between Xaa at position 1 and Xaa at position 7
<220>
   <221> MOD_RES
   <222> (2)..(2)
   <223> N(alpha)METHYLATION
<220>
   <221> MOD_RES
   <222> (7)..(7)
   <223> Pen
<220>
   <221> MOD_RES
   <222> (10)..(10)
   <223> D-Lys
<400> 143
   Xaa Arg Ser Asp Thr Leu Xaa His Glu Xaa
   1               5                   10
<210> 144
   <211> 10
   <212> PRT
   <213> Unknown
<220>
   <223> Synthetic Construct
<220>
   <221> MOD_RES
   <222> (1)..(1)
   <223> ACETYLATION
<220>
   <221> MOD_RES
   <222> (1)..(1)
   <223> Pen
<220>
   <221> DISULFID
   <222> (1)..(7)
   <223> Disulfide bond between Xaa at position 1 and Xaa at position 7
<220>
   <221> MOD_RES
   <222> (2)..(2)
   <223> N(alpha)METHYLATION
<220>
   <221> MOD_RES
   <222> (7)..(7)
   <223> Pen
<220>
   <221> MOD_RES
   <222> (10)..(10)
   <223> D-Lys
<400> 144
   Xaa Arg Ser Asp Thr Leu Xaa His Tyr Xaa
   1               5                   10
<210> 145
   <211> 10
   <212> PRT
   <213> Unknown
<220>
   <223> Synthetic Construct
<220>
   <221> MOD_RES
   <222> (1)..(1)
   <223> ACETYLATION
<220>
   <221> MOD_RES
   <222> (1)..(1)
   <223> Pen
<220>
   <221> DISULFID
   <222> (1)..(7)
   <223> Disulfide bond between Xaa at position 1 and Xaa at position 7
<220>
   <221> MOD_RES
   <222> (2)..(2)
   <223> N(alpha)METHYLATION
<220>
   <221> MOD_RES
   <222> (7)..(7)
   <223> Pen
<220>
   <221> MOD_RES
   <222> (10)..(10)
   <223> D-Lys
<400> 145
   Xaa Arg Ser Asp Thr Leu Xaa Tyr Glu Xaa
   1               5                   10
<210> 146
   <211> 10
   <212> PRT
   <213> Unknown
<220>
   <223> Synthetic Construct
<220>
   <221> MOD_RES
   <222> (1)..(1)
   <223> ACETYLATION
<220>
   <221> MOD_RES
   <222> (1)..(1)
   <223> Pen
<220>
   <221> DISULFID
   <222> (1)..(7)
   <223> Disulfide bond between Xaa at position 1 and Xaa at position 7
<220>
   <221> MOD_RES
   <222> (2)..(2)
   <223> N(alpha)METHYLATION
<220>
   <221> MOD_RES
   <222> (7)..(7)
   <223> Pen
<220>
   <221> MOD_RES
   <222> (8)..(8)
   <223> 2-Nal
<220>
   <221> MOD_RES
   <222> (10)..(10)
   <223> D-Lys
<400> 146
   Xaa Arg Ser Asp Thr Leu Xaa Xaa Tyr Xaa
   1               5                   10